# EUROPEAN PATENT APPLICATION

(11) **EP 4 286 004 A1**
(43) Date of publication of application: **06.12.2023**
(21) Application number: 22382516.7
(22) Date of filing: 30.05.2022
(51) Int. Cl.: A61P 35/00, C07H 15/14, C07H 15/26, C07H 21/02, C07H 21/04, C07H 99/00, A61K 47/54

(54) **DISULFIDE OLIGOSACCHARIDE COMPOUNDS AND COMPLEXES**

(71) Applicant: BioNTech SE, 55131 Mainz (DE); Consejo Superior de Investigaciones Científicas (CSIC), 41013 Sevilla (ES)
(72) Inventor: Moreno Herrero, Jorge, 55131 Mainz (DE); Haas, Heinrich, 55131 Mainz (DE); Erbar, Stephanie, 55131 Mainz (DE); Garcia Fernandez, Jose Manuel, 41092 Sevilla (ES); Benito Hernandez, Juan Manuel, 41092 Sevilla (ES); Lopez Fernandez, Jose, 41092 Sevilla (ES); Sánchez Mellado, Práxedes, 41092 Sevilla (ES)
(74) Representative: Carlos Hernando, Borja

(57) **Abstract**

The present disclosure provides compositions comprising an oligosaccharide composition of formula I, wherein said compositions are useful for delivery of certain agents, including, for example, nucleic acids.

## Description

### Background

Targeted delivery and expression of therapeutic and/or prophylactic agents such as nucleic acids present certain challenges due to the instability of nucleic acids and their inability to permeate the cell membrane. Certain approaches, including use of lipid or polymer-based systems, exhibit promise but suffer from drawbacks related to manufacturing difficulties, poor structural definition, and high polydispersity. Still further, many such compositions lack satisfactory safety and efficacy for use in delivery of therapeutic agents.

### Summary

The present disclosure provides, among other things, cationic and/or ionizable oligosaccharide complexes that overcome certain deficiencies associated with previous compositions, and are useful for targeted delivery of biological agents, including small molecules and nucleic acids.

In some embodiments, the presently disclosure provides a composition comprising an oligosaccharide of formula I: 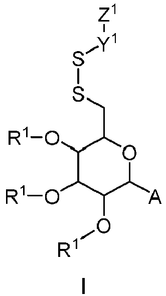 or a pharmaceutically acceptable salt thereof, wherein:
A is A¹, A², or A³: 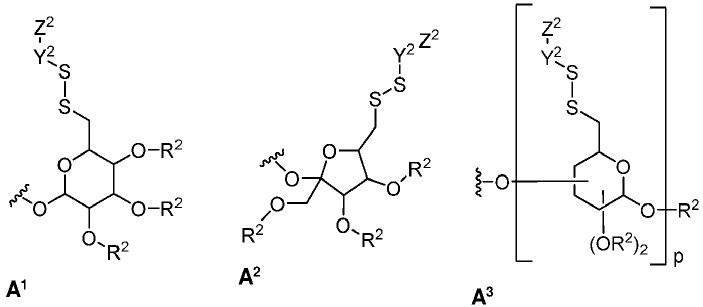
R¹ and R² are each independently selected from H, R^{a}, and -C(O)-R^{a}, and wherein at least one instance of R¹ or R² is not H;
each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b};
each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, and -NHC(O)NHR^{c};
each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S;
Y¹ and Y² are each independently an optionally substituted C₁₋₃₀ aliphatic group wherein one or more carbons are optionally and independently replaced by -Cy-, -NH-, -NHC(O)-, -C(O)NH-, -NHC(O)O-, -OC(O)NH-, -NHC(O)NH-, -NHC(S)NH-, -C(S)NH- - C(O)NHSO₂-, -SO₂NHC(O)-,-OC(O)O-, -O-, -C(O)-, -OC(O)-, -C(O)O-, -SO-, or -SO₂-; each Cy is independently an optionally substituted C₃-C₁₄ cycloaliphatic, 5- to 14-membered heterocyclyl ring having 1-3 heteroatoms selected from N, O, S, 5- to 14-membered heteroaryl ring having 1-3 heteroatoms selected from N, O, S;
Z¹ and Z² are each independently a cationic or ionizable group selected from optionally substituted 5- to 14-membered heterocyclyl ring having 1-3 heteroatoms selected from N, O, and S, 5- to 14-membered heteroaryl ring having 1-3 heteroatoms selected from N, O, and S, -N+(M)₃, 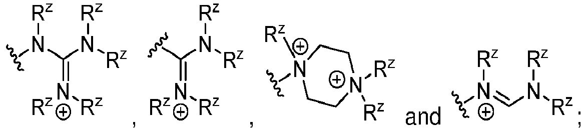
each M is independently -C₀-C₆ aliphatic-R^{z} or -C₀-C₆ aliphatic-N⁺(R^{z})₃;
each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S; and
p is an integer selected from 1, 2, 3, 4, or 5.

In some embodiments, the present disclosure provides a complex comprising an oligosaccharide composition described herein and a nucleic acid.

In some embodiments, the present disclosure provides a method of delivering a composition to a target in a subject, the method comprising administering to the subject a complex as described herein.

In some embodiments, the present disclosure provides a method of treating and or providing prophylaxis for a disease, disorder, or condition in a subject comprising administering to the subject a complex as described herein.

In some embodiments, the present disclosure provides a method of preparing an oligosaccharide of formula **la:** 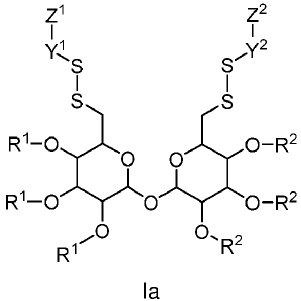 or a pharmaceutically acceptable salt thereof, comprising contacting a compound of formula II 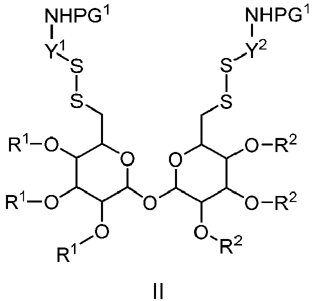 with a first acid to provide the compound of formula la, wherein
Y¹ and Y² are each -CH₂-CH₂-NHC(S)NH-CH₂-CH₂-;
Z¹ and Z² are each -NH₃Cl;
R¹ and R² are each -C(O)-R^{a};
R^{a} is optionally substituted C₁-C₂₀ aliphatic; and
PG¹ is a nitrogen protecting group.

### Brief Description of the Drawings

**FIG. 1A** is a plot indicating percentage of free RNA quantified by gel electrophoresis of formulated saRNA at different N/P ratios between 0-3 for either JRL13 or ssJRL13.
**FIG. 1B** is a plot indicating hydrodynamic diameter for formulated saRNA at different N/P ratios with either JRL13 or ssJRL13.
**FIGs. 1C-1D** are plots indicating expression of luciferase-encoding saRNA after 24 of transfection of C2C12 cells (FIG. 1C) or HepG2 cells (FIG. 1D), N/P12 formulated saRNA with either JRL13 or ssJRL13, at 12.5ng to 400ng of RNA/well. Data here represents 3 independent experiments (n=3) with technical duplicates.
**FIGs. 1E-1F** are plots indicating expression viability of cells after 24 of transfection of C2C12 cells (FIG. 1E) or HepG2 cells (FIG. 1F), N/P12 formulated saRNA with either JRL13 or ssJRL13, at 12.5ng to 400ng of RNA/well.
**FIG. 2A** is a bar graph indicating hydrodynamic diameter for formulated saRNA with either JRL13 or ssJRL13.
**FIG. 2B** is a plot indicating serological levels of specific IgG against HA over the course of the experiment at a serum dilution of 1:300.
**FIG. 3A** is a bar graph indicating the hydrodynamic diameter for formulated saRNA with either JRL13 or ssJRL13.
**FIG. 3B** is a plot illustrating serological levels of specific IgG against HA over the course of the experiment at a serum dilution of 1:300.
**FIG. 3C** is a plot indicating end-point titration of the serological levels of specific IgG against HA.
**FIG. 3D** is a bar graph indicating CD8/CD4 response against HA-peptide pools.
**FIG. 4A** is a bar graph the hydrodynamic diameter for formulated saRNA with ssJRL13, ssJRL13 or ssJLF94.
**FIG. 4B** is a plot illustrating serological levels of specific IgG against HA over the course of the experiment at a serum dilution of 1:300.
**FIG. 4C** is a plot illustrating end-point titration of the serological levels of specific IgG against HA.
**FIG. 4D** is a bar graph illustrating CD8/CD4 response against HA-peptide pools.

### Detailed Description of Certain Embodiments

Compounds of this disclosure include those described generally above and are further illustrated by the classes, subclasses, and species disclosed herein. As used herein, the following definitions shall apply unless otherwise indicated. For purposes of this disclosure, the chemical elements are identified in accordance with the Periodic Table of Elements, CAS version, Handbook of Chemistry and Physics, 75^{th} Ed. Additionally, general principles of organic chemistry are described in "Organic Chemistry", Thomas Sorrell, University Science Books, Sausalito: 1999, and "March's Advanced Organic Chemistry", 5th Ed., Ed.: Smith, M.B. and March, J., John Wiley & Sons, New York: 2001, the entire contents of which are hereby incorporated by reference.

Unless otherwise stated, structures depicted herein are meant to include all stereoisomeric (e.g., enantiomeric or diastereomeric) forms of the structure, as well as all geometric or conformational isomeric forms of the structure. For example, the R and S configurations of each stereocenter are contemplated as part of the disclosure. Therefore, single stereochemical isomers, as well as enantiomeric, diastereomeric, and geometric (or conformational) mixtures of provided compounds are within the scope of the disclosure. For example, in some cases, Table 1 shows one or more stereoisomers of a compound, and unless otherwise indicated, represents each stereoisomer alone and/or as a mixture. Unless otherwise stated, all tautomeric forms of provided compounds are within the scope of the disclosure.

Unless otherwise indicated, structures depicted herein are meant to include compounds that differ only in the presence of one or more isotopically enriched atoms. For example, compounds having the present structures including replacement of hydrogen by deuterium or tritium, or replacement of a carbon by ¹³C- or ¹⁴C-enriched carbon are within the scope of this disclosure.

### Definitions

***About or approximately:*** As used herein, the term "approximately" or "about," as applied to one or more values of interest, refers to a value that is similar to a stated reference value. In general, those skilled in the art, familiar within the context, will appreciate the relevant degree of variance encompassed by "about" or "approximately" in that context. For example, in some embodiments, the term "approximately" or "about" may encompass a range of values that are within (i.e., ±) 25%, 20%, 19%, 18%, 17%, 16%, 15%, 14%, 13%, 12%, 11%, 10%, 9%, 8%, 7%, 6%, 5%, 4%, 3%, 2%, 1%, or less of the referred value.

***Administering:*** As used herein, the term "administering" or "administration" typically refers to the administration of a composition to a subject to achieve delivery of an agent that is, or is included in, a composition to a target site or a site to be treated. Those of ordinary skill in the art will be aware of a variety of routes that may, in appropriate circumstances, be utilized for administration to a subject, for example a human. For example, in some embodiments, administration may be ocular, oral, parenteral, topical, *etc.* In some particular embodiments, administration may be bronchial *(e.g.,* by bronchial instillation), buccal, dermal (which may be or comprise, for example, one or more of topical to the dermis, intradermal, interdermal, transdermal, *etc.),* enteral, intra-arterial, intradermal, intragastric, intramedullary, intramuscular, intranasal, intraperitoneal, intrathecal, intravenous, intraventricular, within a specific organ (e.g., intrahepatic), mucosal, nasal, oral, rectal, subcutaneous, sublingual, topical, tracheal (e.g., by intratracheal instillation), vaginal, vitreal, *etc.* In some embodiments, administration may be parenteral. In some embodiments, administration may be oral. In some particular embodiments, administration may be intravenous. In some particular embodiments, administration may be subcutaneous. In some embodiments, administration may involve only a single dose. In some embodiments, administration may involve application of a fixed number of doses. In some embodiments, administration may involve dosing that is intermittent *(e.g.,* a plurality of doses separated in time) and/or periodic *(e.g.,* individual doses separated by a common period of time) dosing. In some embodiments, administration may involve continuous dosing (e.g., perfusion) for at least a selected period of time. In some embodiments, administration may comprise a prime-and-boost protocol. A prime-and-boost protocol can include administration of a first dose of a pharmaceutical composition (e.g., an immunogenic composition, e.g., a vaccine) followed by, after an interval of time, administration of a second or subsequent dose of a pharmaceutical composition (e.g., an immunogenic composition, e.g., a vaccine). In the case of an immunogenic composition, a prime-and-boost protocol can result in an increased immune response in a patient.

***Aliphatic:*** The term "aliphatic" refers to a straight-chain (i.e., unbranched) or branched, substituted or unsubstituted hydrocarbon chain that is completely saturated or that contains one or more units of unsaturation, or a monocyclic hydrocarbon or bicyclic hydrocarbon that is completely saturated or that contains one or more units of unsaturation, but which is not aromatic (also referred to herein as "cycloaliphatic"), that has a single point or more than one points of attachment to the rest of the molecule. Unless otherwise specified, aliphatic groups contain 1-12 aliphatic carbon atoms. As used herein, it is understood that an aliphatic group can also be bivalent (e.g., encompass a bivalent hydrocarbon chain that is saturated or contains one or more units of unsaturation, such as, for example, -CH₂-, -CH₂-CH₂-, -CH₂-CH₂-CH₂-, and so on). In some embodiments, aliphatic groups contain 1-6 aliphatic carbon atoms (e.g., C₁₋₆). In some embodiments, aliphatic groups contain 1-5 aliphatic carbon atoms (e.g., C₁₋₅). In other embodiments, aliphatic groups contain 1-4 aliphatic carbon atoms (e.g., C₁₋₄). In still other embodiments, aliphatic groups contain 1-3 aliphatic carbon atoms (e.g., C₁₋₃), and in yet other embodiments, aliphatic groups contain 1-2 aliphatic carbon atoms (e.g., C₁₋₂). In some embodiments, "cycloaliphatic" refers to a monocyclic C₃₋₈ hydrocarbon or a bicyclic C₇₋₁₀ hydrocarbon that is completely saturated or that contains one or more units of unsaturation, but which is not aromatic, that has a single point or more than one points of attachment to the rest of the molecule. Suitable aliphatic groups include, but are not limited to, linear or branched, substituted or unsubstituted alkyl, alkenyl, or alkynyl groups and hybrids thereof. A preferred aliphatic group is C₁₋₆ alkyl.

***Alkyl:*** The term "alkyl", used alone or as part of a larger moiety, refers to a saturated, optionally substituted straight or branched chain hydrocarbon group having (unless otherwise specified) 1-12, 1-10, 1-8, 1-6, 1-4, 1-3, or 1-2 carbon atoms (e.g., C₁₋₁₂, C₁₋₁₀, C₁₋₈, C₁₋₆, C₁₋₄, C₁₋₃, or C₁₋₂). Exemplary alkyl groups include methyl, ethyl, propyl, butyl, pentyl, hexyl, and heptyl.

***Alkenyl:*** The term "alkenyl", used alone or as part of a larger moiety, refers to an optionally substituted straight or branched chain or cyclic hydrocarbon group having at least one double bond and having (unless otherwise specified) 2-12, 2-10, 2-8, 2-6, 2-4, or 2-3 carbon atoms(e.g., C₂₋₁₂, C₂₋₁₀, C₂₋₈, C₂₋₆, C₂₋₄, or C₂₋₃). Exemplary alkenyl groups include ethenyl, propenyl, butenyl, pentenyl, hexenyl, and heptenyl. The term "cycloalkenyl" refers to an optionally substituted non-aromatic monocyclic or multicyclic ring system containing at least one carbon-carbon double bond and having about 3 to about 10 carbon atoms. Exemplary monocyclic cycloalkenyl rings include cyclopentenyl, cyclohexenyl, and cycloheptenyl.

***Alkynyl:*** The term "alkynyl", used alone or as part of a larger moiety, refers to an optionally substituted straight or branched chain hydrocarbon group having at least one triple bond and having (unless otherwise specified) 2-12, 2-10, 2-8, 2-6, 2-4, or 2-3 carbon atoms (e.g., C₂₋₁₂, C₂₋₁₀, C₂₋₈, C₂₋₆, C₂₋₄, or C₂₋₃). Exemplary alkynyl groups include ethynyl, propynyl, butynyl, pentynyl, hexynyl, and heptynyl.

***Analog:*** As used herein, the term "analog" refers to a substance that shares one or more particular structural features, elements, components, or moieties with a reference substance. Typically, an "analog" shows significant structural similarity with the reference substance, for example sharing a core or consensus structure, but also differs in certain discrete ways. In some embodiments, an analog is a substance that can be generated from the reference substance, e.g., by chemical manipulation of the reference substance. In some embodiments, an analog is a substance that can be generated through performance of a synthetic process substantially similar to (e.g., sharing a plurality of steps with) one that generates the reference substance. In some embodiments, an analog is or can be generated through performance of a synthetic process different from that used to generate the reference substance.

***Aryl:*** The term "aryl" refers to monocyclic and bicyclic ring systems having a total of five to fourteen ring members (e.g., C₅-C₁₄), wherein at least one ring in the system is aromatic and wherein each ring in the system contains three to seven ring members. In some embodiments, an "aryl" group contains between six and twelve total ring members (e.g., C₆-C₁₂). The term "aryl" may be used interchangeably with the term "aryl ring". In certain embodiments of the present invention, "aryl" refers to an aromatic ring system which includes, but not limited to, phenyl, biphenyl, naphthyl, anthracyl and the like, which may bear one or more substituents. Unless otherwise specified, "aryl" groups are hydrocarbons. In some embodiments, an "aryl" ring system is an aromatic ring (e.g., phenyl) that is fused to a non-aromatic ring (e.g., cycloalkyl). Examples of aryl rings include that are fused include

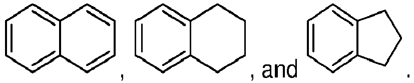

***Associated:*** Two events or entities are "associated" with one another, as that term is used herein, if the presence, level and/or form of one is correlated with that of the other. For example, a particular entity (e.g., polypeptide, genetic signature, metabolite, microbe, etc) is considered to be associated with a particular disease, disorder, or condition, if its presence, level and/or form correlates with incidence of and/or susceptibility to the disease, disorder, or condition (e.g., across a relevant population). In some embodiments, two or more entities are physically "associated" with one another if they interact, directly or indirectly, so that they are and/or remain in physical proximity with one another. In some embodiments, two or more entities that are physically associated with one another are covalently linked to one another; in some embodiments, two or more entities that are physically associated with one another are not covalently linked to one another but are non-covalently associated, for example by means of hydrogen bonds, van der Waals interaction, hydrophobic interactions, magnetism, and combinations thereof.

***Biological sample:*** As used herein, the term "biological sample" typically refers to a sample obtained or derived from a biological source (e.g., a tissue or organism or cell culture) of interest, as described herein. In some embodiments, a source of interest comprises an organism, such as an animal or human. In some embodiments, a biological sample is or comprises biological tissue or fluid. In some embodiments, a biological sample may be or comprise bone marrow; blood; blood cells; ascites; tissue or fine needle biopsy samples; cell-containing body fluids; free floating nucleic acids; sputum; saliva; urine; cerebrospinal fluid, peritoneal fluid; pleural fluid; feces; lymph; gynecological fluids; skin swabs; vaginal swabs; oral swabs; nasal swabs; washings or lavages such as a ductal lavages or broncheoalveolar lavages; aspirates; scrapings; bone marrow specimens; tissue biopsy specimens; surgical specimens; feces, other body fluids (e.g., sperm, sweat, tears), secretions, and/or excretions; and/or cells therefrom, etc. In some embodiments, a biological sample is or comprises cells obtained from an individual. In some embodiments, obtained cells are or include cells from an individual from whom the sample is obtained. In some embodiments, a sample is a "primary sample" obtained directly from a source of interest by any appropriate means. For example, in some embodiments, a primary biological sample is obtained by methods selected from the group consisting of biopsy (e.g., fine needle aspiration or tissue biopsy), surgery, collection of body fluid (e.g., blood, lymph, feces etc.), etc. In some embodiments, as will be clear from context, the term "sample" refers to a preparation that is obtained by processing (e.g., by removing one or more components of and/or by adding one or more agents to) a primary sample. For example, filtering using a semipermeable membrane. Such a "processed sample" may comprise, for example, nucleic acids or proteins extracted from a sample or obtained by subjecting a primary sample to techniques such as amplification or reverse transcription of mRNA, isolation and/or purification of certain components, etc.

***Carrier:*** As used herein, the term "carrier" refers to a diluent, adjuvant, excipient, or vehicle with which a composition is administered. In some exemplary embodiments, carriers can include sterile liquids, such as, for example, water and oils, including oils of petroleum, animal, vegetable or synthetic origin, such as, for example, peanut oil, soybean oil, mineral oil, sesame oil and the like. In some embodiments, carriers are or include one or more solid components.

***Combination therapy:*** As used herein, the term "combination therapy" refers to those situations in which a subject is simultaneously exposed to two or more therapeutic regimens (e.g., two or more therapeutic agents or modality(ies)). In some embodiments, the two or more regimens may be administered simultaneously; in some embodiments, such regimens may be administered sequentially (e.g., all "doses" of a first regimen are administered prior to administration of any doses of a second regimen); in some embodiments, such agents are administered in overlapping dosing regimens. In some embodiments, "administration" of combination therapy may involve administration of one or more agent(s) or modality(ies) to a subject receiving the other agent(s) or modality(ies) in the combination. For clarity, combination therapy does not require that individual agents be administered together in a single composition (or even necessarily at the same time), although in some embodiments, two or more agents, or active moieties thereof, may be administered together in a combination composition, or even in a combination compound (e.g., as part of a single chemical complex or covalent entity).

***Comparable:*** As used herein, the term "comparable" refers to two or more agents, entities, situations, sets of conditions, etc., that may not be identical to one another but that are sufficiently similar to permit comparison therebetween so that one skilled in the art will appreciate that conclusions may reasonably be drawn based on differences or similarities observed. In some embodiments, comparable sets of conditions, circumstances, individuals, or populations are characterized by a plurality of substantially identical features and one or a small number of varied features. Those of ordinary skill in the art will understand, in context, what degree of identity is required in any given circumstance for two or more such agents, entities, situations, sets of conditions, etc. to be considered comparable. For example, those of ordinary skill in the art will appreciate that sets of circumstances, individuals, or populations are comparable to one another when characterized by a sufficient number and type of substantially identical features to warrant a reasonable conclusion that differences in results obtained or phenomena observed under or with different sets of circumstances, individuals, or populations are caused by or indicative of the variation in those features that are varied.

***Composition:*** Those skilled in the art will appreciate that the term "composition" may be used to refer to a discrete physical entity that comprises one or more specified components. In general, unless otherwise specified, a composition may be of any form - e.g., gas, gel, liquid, solid, etc.

***Cycloaliphatic:*** As used herein, the term "cycloaliphatic" refers to a monocyclic C₃₋₈ hydrocarbon or a bicyclic C₇₋₁₀ hydrocarbon that is completely saturated or that contains one or more units of unsaturation, but which is not aromatic, that has a single point or more than one points of attachment to the rest of the molecule.

***Cycloalkyl:*** As used herein, the term "cycloalkyl" refers to an optionally substituted saturated ring monocyclic or polycyclic system of about 3 to about 10 ring carbon atoms. Exemplary monocyclic cycloalkyl rings include cyclopropyl, cyclobutyl, cyclopentyl, cyclohexyl, and cycloheptyl.

***Dosage form*** or ***unit dosage form:*** Those skilled in the art will appreciate that the term "dosage form" may be used to refer to a physically discrete unit of an active agent (e.g., a therapeutic or diagnostic agent) for administration to a subject. Typically, each such unit contains a predetermined quantity of active agent. In some embodiments, such quantity is a unit dosage amount (or a whole fraction thereof) appropriate for administration in accordance with a dosing regimen that has been determined to correlate with a desired or beneficial outcome when administered to a relevant population (*i.e.,* with a therapeutic dosing regimen). Those of ordinary skill in the art appreciate that the total amount of a therapeutic composition or agent administered to a particular subject is determined by one or more attending physicians and may involve administration of multiple dosage forms.

***Dosing regimen* or *therapeutic regimen:*** Those skilled in the art will appreciate that the terms "dosing regimen" and "therapeutic regimen" may be used to refer to a set of unit doses (typically more than one) that are administered individually to a subject, typically separated by periods of time. In some embodiments, a given therapeutic agent has a recommended dosing regimen, which may involve one or more doses. In some embodiments, a dosing regimen comprises a plurality of doses each of which is separated in time from other doses. In some embodiments, individual doses are separated from one another by a time period of the same length; in some embodiments, a dosing regimen comprises a plurality of doses and at least two different time periods separating individual doses. In some embodiments, all doses within a dosing regimen are of the same unit dose amount. In some embodiments, different doses within a dosing regimen are of different amounts. In some embodiments, a dosing regimen comprises a first dose in a first dose amount, followed by one or more additional doses in a second dose amount different from the first dose amount. In some embodiments, a dosing regimen comprises a first dose in a first dose amount, followed by one or more additional doses in a second dose amount same as the first dose amount. In some embodiments, a dosing regimen is correlated with a desired or beneficial outcome when administered across a relevant population (*i.e.,* is a therapeutic dosing regimen).

***Excipient:*** As used herein, the term "excipient" refers to a non-therapeutic agent that may be included in a pharmaceutical composition, for example, to provide or contribute to a desired consistency or stabilizing effect. Suitable pharmaceutical excipients include, for example, starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

***Heteroaliphatic*:** The term "heteroaliphatic" or "heteroaliphatic group", as used herein, denotes an optionally substituted hydrocarbon moiety having, in addition to carbon atoms, from one to five heteroatoms, that may be straight-chain (*i.e.,* unbranched), branched, or cyclic ("heterocyclic") and may be completely saturated or may contain one or more units of unsaturation, but which is not aromatic. The term "heteroatom" refers to nitrogen, oxygen, or sulfur, and includes any oxidized form of nitrogen or sulfur, and any quaternized form of a basic nitrogen. The term "nitrogen" also includes a substituted nitrogen. Unless otherwise specified, heteroaliphatic groups contain 1-10 carbon atoms wherein 1-3 carbon atoms are optionally and independently replaced with heteroatoms selected from oxygen, nitrogen, and sulfur. In some embodiments, heteroaliphatic groups contain 1-4 carbon atoms, wherein 1-2 carbon atoms are optionally and independently replaced with heteroatoms selected from oxygen, nitrogen, and sulfur. In yet other embodiments, heteroaliphatic groups contain 1-3 carbon atoms, wherein 1 carbon atom is optionally and independently replaced with a heteroatom selected from oxygen, nitrogen, and sulfur. Suitable heteroaliphatic groups include, but are not limited to, linear or branched, heteroalkyl, heteroalkenyl, and heteroalkynyl groups. For example, a 1- to 10 atom heteroaliphatic group includes the following exemplary groups: -O-CH₃, -CH₂-O-CH₃, -O-CH₂-CH₂-O-CH₂-CH₂-O-CH₃, and the like.

***Heteroaryl:*** The terms "heteroaryl" and "heteroar-", used alone or as part of a larger moiety, e.g., "heteroaralkyl", or "heteroaralkoxy", refer to monocyclic or bicyclic ring groups having 5 to 12 ring atoms (e.g., 5- to 6- membered monocyclic heteroaryl or 9- to 12-membered bicyclic heteroaryl); having 6, 10, or 14 π-electrons shared in a cyclic array; and having, in addition to carbon atoms, from one to five heteroatoms. The term "heteroatom" refers to nitrogen, oxygen, or sulfur, and includes any oxidized form of nitrogen or sulfur, and any quaternized form of a basic nitrogen. Heteroaryl groups include, without limitation, thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, indolizinyl, purinyl, naphthyridinyl, pteridinyl, imidazo[1,2-a]pyrimidinyl, imidazo[1,2-a]pyridyl, imidazo[4,5-b]pyridyl, imidazo[4,5-c]pyridyl, pyrrolopyridyl, pyrrolopyrazinyl, thienopyrimidinyl, triazolopyridyl, and benzoisoxazolyl. The terms "heteroaryl" and "heteroar-", as used herein, also include groups in which a heteroaromatic ring is fused to one or more aryl, cycloaliphatic, or heterocyclyl rings, where the radical or point of attachment is on the heteroaromatic ring (i.e., a bicyclic heteroaryl ring having 1 to 3 heteroatoms). Nonlimiting examples include indolyl, isoindolyl, benzothienyl, benzofuranyl, dibenzofuranyl, indazolyl, benzimidazolyl, benzotriazolyl, benzothiazolyl, benzothiadiazolyl, benzoxazolyl, quinolyl, isoquinolyl, cinnolinyl, phthalazinyl, quinazolinyl, quinoxalinyl, 4*H*-quinolizinyl, carbazolyl, acridinyl, phenazinyl, phenothiazinyl, phenoxazinyl, tetrahydroquinolinyl, tetrahydroisoquinolinyl, pyrido[2,3-b]-1,4-oxazin-3(4H)-one, 4H-thieno[3,2-b]pyrrole, and benzoisoxazolyl. A heteroaryl group may be mono- or bicyclic. The term "heteroaryl" may be used interchangeably with the terms "heteroaryl ring", "heteroaryl group", or "heteroaromatic", any of which terms include rings that are optionally substituted. The term "heteroaralkyl" refers to an alkyl group substituted by a heteroaryl, wherein the alkyl and heteroaryl portions independently are optionally substituted.

***Heteroatom:*** The term "heteroatom" as used herein refers to nitrogen, oxygen, or sulfur, and includes any oxidized form of nitrogen or sulfur, and any quaternized form of a basic nitrogen.

***Heterocycle:*** As used herein, the terms "heterocycle", "heterocyclyl", "heterocyclic radical", and "heterocyclic ring" are used interchangeably and refer to a stable 3- to 8-membered monocyclic, a 7- to 12-membered bicyclic, or a 10- to 16-membered polycyclic heterocyclic moiety that is either saturated or partially unsaturated, and having, in addition to carbon atoms, one or more, such as one to four, heteroatoms, as defined above. When used in reference to a ring atom of a heterocycle, the term "nitrogen" includes a substituted nitrogen. As an example, in a saturated or partially unsaturated ring having 0-3 heteroatoms selected from oxygen, sulfur or nitrogen, the nitrogen may be N (as in 3,4-dihydro-2H-pyrrolyl), NH (as in pyrrolidinyl), or NR+ (as in N-substituted pyrrolidinyl). A heterocyclic ring can be attached to its pendant group at any heteroatom or carbon atom that results in a stable structure and any of the ring atoms can be optionally substituted. Examples of such saturated or partially unsaturated heterocyclic radicals include, without limitation, azetidinyl, oxetanyl, tetrahydrofuranyl, tetrahydrothienyl, pyrrolidinyl, piperidinyl, decahydroquinolinyl, oxazolidinyl, piperazinyl, dioxanyl, dioxolanyl, diazepinyl, oxazepinyl, thiazepinyl, morpholinyl, and thiamorpholinyl. A heterocyclyl group may be mono-, bi-, tri-, or polycyclic, preferably mono-, bi-, or tricyclic, more preferably mono- or bicyclic. The term "heterocyclylalkyl" refers to an alkyl group substituted by a heterocyclyl, wherein the alkyl and heterocyclyl portions independently are optionally substituted. A bicyclic heterocyclic ring also includes groups in which the heterocyclic ring is fused to one or more aryl rings. Exemplary bicyclic heterocyclic groups include indolinyl, isoindolinyl, benzodioxolyl, 1,3-dihydroisobenzofuranyl, 2,3-dihydrobenzofuranyl, tetrahydroquinolinyl, 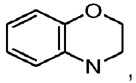 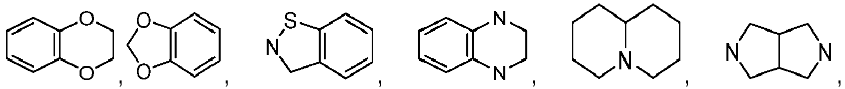 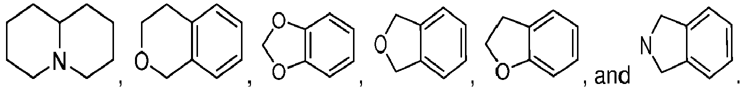 A bicyclic heterocyclic ring can also be a spirocyclic ring system (e.g., 7- to 11-membered spirocyclic fused heterocyclic ring having, in addition to carbon atoms, one or more heteroatoms as defined above (e.g., one, two, three or four heteroatoms)). A bicyclic heterocyclic ring can also be a bridged ring system (e.g., 7- to 11-membered bridged heterocyclic ring having one, two, or three bridging atoms.

***Nucleic acid*/ *Polynucleotide*:** As used herein, the term "nucleic acid" refers to a polymer of at least 10 nucleotides or more. In some embodiments, a nucleic acid is or comprises DNA. In some embodiments, a nucleic acid is or comprises RNA. In some embodiments, a nucleic acid is or comprises peptide nucleic acid (PNA). In some embodiments, a nucleic acid is or comprises a single stranded nucleic acid. In some embodiments, a nucleic acid is or comprises a double-stranded nucleic acid. In some embodiments, a nucleic acid comprises both single and double-stranded portions. In some embodiments, a nucleic acid comprises a backbone that comprises one or more phosphodiester linkages. In some embodiments, a nucleic acid comprises a backbone that comprises both phosphodiester and non-phosphodiester linkages. For example, in some embodiments, a nucleic acid may comprise a backbone that comprises one or more phosphorothioate or 5'-N-phosphoramidite linkages and/or one or more peptide bonds, e.g., as in a "peptide nucleic acid". In some embodiments, a nucleic acid comprises one or more, or all, natural residues (e.g., adenine, cytosine, deoxyadenosine, deoxycytidine, deoxyguanosine, deoxythymidine, guanine, thymine, uracil). In some embodiments, a nucleic acid comprises on or more, or all, non-natural residues. In some embodiments, a non-natural residue comprises a nucleoside analog (*e*.*g*., 2-aminoadenosine, 2-thiothymidine, inosine, pyrrolo-pyrimidine, 3 -methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5 -propynyl-cytidine, C5-methylcytidine, 2-aminoadenosine, 7-deazaadenosine, 7-deazaguanosine, 8-oxoadenosine, 8-oxoguanosine, 6-O-methylguanine, 2-thiocytidine, methylated bases, intercalated bases, and combinations thereof). In some embodiments, a non-natural residue comprises one or more modified sugars (e.g., 2'-fluororibose, ribose, 2'-deoxyribose, arabinose, and hexose) as compared to those in natural residues. In some embodiments, a nucleic acid has a nucleotide sequence that encodes a functional gene product such as an RNA or polypeptide. In some embodiments, a nucleic acid has a nucleotide sequence that comprises one or more introns. In some embodiments, a nucleic acid may be prepared by isolation from a natural source, enzymatic synthesis (*e.g.,* by polymerization based on a complementary template, *e.g., in vivo* or *in vitro,* reproduction in a recombinant cell or system, or chemical synthesis. In some embodiments, a nucleic acid is at least 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, 150, 160, 170, 180, 190, 20, 225, 250, 275, 300, 325, 350, 375, 400, 425, 450, 475, 500, 600, 700, 800, 900, 1000, 1500, 2000, 2500, 3000, 3500, 4000, 4500, 5000, 5500, 6000, 6500, 7000, 7500, 8000, 8500, 9000, 9500, 10,000, 10,500, 11,000, 11,500, 12,000, 12,500, 13,000, 13,500, 14,000, 14,500, 15,000, 15,500, 16,000, 16,500, 17,000, 17,500, 18,000, 18,500, 19,000, 19,500, or 20,000 or more residues or nucleotides long.

***Nucleic acid particle:*** A "nucleic acid particle" can be used to deliver nucleic acid to a target site of interest (e.g., cell, tissue, organ, and the like). A nucleic acid particle may be formed from at least one cationic or cationically ionizable lipid or lipid-like material, at least one cationic polymer such as protamine, or a mixture thereof and nucleic acid. Nucleic acid particles include lipid nanoparticle (LNP)-based and lipoplex (LPX)-based formulations.

***Nucleotide:*** As used herein, the term "nucleotide" refers to its art-recognized meaning. When a number of nucleotides is used as an indication of size, e.g., of a polynucleotide, a certain number of nucleotides refers to the number of nucleotides on a single strand, *e.g.,* of a polynucleotide.

*Oral:* The phrases "oral administration" and "administered orally" as used herein have their art-understood meaning referring to administration by mouth of a compound or composition.

***Parenteral:*** The phrases "parenteral administration" and "administered parenterally" as used herein have their art-understood meaning referring to modes of administration other than enteral and topical administration, usually by injection, and include, without limitation, intravenous, intramuscular, intra-arterial, intrathecal, intracapsular, intraorbital, intracardiac, intradermal, intraperitoneal, transtracheal, subcutaneous, subcuticular, intraarticulare, subcapsular, subarachnoid, intraspinal, and intrasternal injection and infusion.

***Partially unsaturated:*** As used herein, the term "partially unsaturated" refers to a ring moiety that includes at least one double or triple bond between ring atoms. The term "partially unsaturated" is intended to encompass rings having multiple sites of unsaturation, but is not intended to include aromatic (e.g., aryl or heteroaryl) moieties, as herein defined.

***Patient*** or ***subject:*** As used herein, the term "patient" or "subject" refers to any organism to which a provided composition is or may be administered, e.g., for experimental, diagnostic, prophylactic, cosmetic, and/or therapeutic purposes. Typical patients or subjects include animals (e.g., mammals such as mice, rats, rabbits, non-human primates, and/or humans). In some embodiments, a patient is a human. In some embodiments, a patient or a subject is suffering from or susceptible to one or more disorders or conditions. In some embodiments, a patient or subject displays one or more symptoms of a disorder or condition. In some embodiments, a patient or subject has been diagnosed with one or more disorders or conditions. In some embodiments, a patient or a subject is receiving or has received certain therapy to diagnose and/or to treat a disease, disorder, or condition.

***Pharmaceutical composition:*** As used herein, the term "pharmaceutical composition" refers to an active agent, formulated together with one or more pharmaceutically acceptable carriers. In some embodiments, the active agent is present in unit dose amount appropriate for administration in a therapeutic or dosing regimen that shows a statistically significant probability of achieving a predetermined therapeutic effect when administered to a relevant population. In some embodiments, pharmaceutical compositions may be specially formulated for administration in solid or liquid form, including those adapted for the following: oral administration, for example, drenches (aqueous or non-aqueous solutions or suspensions), tablets, e.g., those targeted for buccal, sublingual, and systemic absorption, boluses, powders, granules, pastes for application to the tongue; parenteral administration, for example, by subcutaneous, intramuscular, intravenous or epidural injection as, for example, a sterile solution or suspension, or sustained-release formulation; topical application, for example, as a cream, ointment, or a controlled-release patch or spray applied to the skin, lungs, or oral cavity; intravaginally or intrarectally, for example, as a pessary, cream, or foam; sublingually; ocularly; transdermally; or nasally, pulmonary, and to other mucosal surfaces.

***Pharmaceutically acceptable:*** As used herein, the phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

***Pharmaceutically acceptable carrier:*** As used herein, the term "pharmaceutically acceptable carrier" means a pharmaceutically-acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations.

***Pharmaceutically acceptable salt:*** The term "pharmaceutically acceptable salt", as used herein, refers to salts of such compounds that are appropriate for use in pharmaceutical contexts, *i.e.,* salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, et al. describes pharmaceutically acceptable salts in detail in J. Pharmaceutical Sciences, 66: 1-19 (1977). In some embodiments, pharmaceutically acceptable salts include, but are not limited to, nontoxic acid addition salts, which are salts of an amino group formed with inorganic acids such as hydrochloric acid, hydrobromic acid, phosphoric acid, sulfuric acid and perchloric acid or with organic acids such as acetic acid, maleic acid, tartaric acid, citric acid, succinic acid or malonic acid or by using other methods used in the art such as ion exchange. In some embodiments, pharmaceutically acceptable salts include, but are not limited to, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxyethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, *p-*toluenesulfonate, undecanoate, valerate salts, and the like. Representative alkali or alkaline earth metal salts include sodium, lithium, potassium, calcium, magnesium, and the like. In some embodiments, pharmaceutically acceptable salts include, when appropriate, nontoxic ammonium, quaternary ammonium, and amine cations formed using counterions such as halide, hydroxide, carboxylate, sulfate, phosphate, nitrate, alkyl having from 1 to 6 carbon atoms, sulfonate and aryl sulfonate.

***Polycyclic*:** As used herein, the term "polycyclic" refers to a saturated or unsaturated ring system having two or more rings (for example, heterocyclyl rings, heteroaryl rings, cycloalkyl rings, or aryl rings), having between 7 and 20 atoms, in which one or more carbon atoms are common to two adjacent rings. For example, in some embodiments, a polycyclic ring system refers to a saturated or unsaturated ring system having three or more rings (for example, heterocyclyl rings, heteroaryl rings, cycloalkyl rings, or aryl rings), having between 14 and 20 atoms, in which one or more carbon atoms are common to two adjacent rings. The rings in a polycyclic ring system may be fused (i.e., bicyclic or tricyclic), spirocyclic, or a combination thereof. An example polycyclic ring is a steroid.

***Polypeptide*:** The term "polypeptide", as used herein, typically has its art-recognized meaning of a polymer of at least three amino acids or more. Those of ordinary skill in the art will appreciate that the term "polypeptide" is intended to be sufficiently general as to encompass not only polypeptides having a complete sequence recited herein, but also to encompass polypeptides that represent functional, biologically active, or characteristic fragments, portions or domains (e.g., fragments, portions, or domains retaining at least one activity) of such complete polypeptides. In some embodiments, polypeptides may contain L-amino acids, D-amino acids, or both and/or may contain any of a variety of amino acid modifications or analogs known in the art. Useful modifications include, e.g., terminal acetylation, amidation, methylation, *etc.* In some embodiments, polypeptides may comprise natural amino acids, non-natural amino acids, synthetic amino acids, and combinations thereof (e.g., may be or comprise peptidomimetics).

***Prevent*** or ***prevention:*** As used herein, the terms "prevent" or "prevention", when used in connection with the occurrence of a disease, disorder, and/or condition, refer to reducing the risk of developing the disease, disorder and/or condition and/or to delaying onset of one or more characteristics or symptoms of the disease, disorder or condition. Prevention may be considered complete when onset of a disease, disorder or condition has been delayed for a predefined period of time.

***Reference:*** As used herein describes a standard or control relative to which a comparison is performed. For example, in some embodiments, an agent, animal, individual, population, sample, sequence or value of interest is compared with a reference or control agent, animal, individual, population, sample, sequence or value. In some embodiments, a reference or control is tested and/or determined substantially simultaneously with the testing or determination of interest. In some embodiments, a reference or control is a historical reference or control, optionally embodied in a tangible medium. Typically, as would be understood by those skilled in the art, a reference or control is determined or characterized under comparable conditions or circumstances to those under assessment. Those skilled in the art will appreciate when sufficient similarities are present to justify reliance on and/or comparison to a particular possible reference or control.

***Ribonucleotide:*** As used herein, the term "ribonucleotide" encompasses unmodified ribonucleotides and modified ribonucleotides. For example, unmodified ribonucleotides include the purine bases adenine (A) and guanine (G), and the pyrimidine bases cytosine (C) and uracil (U). Modified ribonucleotides may include one or more modifications including, but not limited to, for example, (a) end modifications, e.g., 5' end modifications *(e.g.,* phosphorylation, dephosphorylation, conjugation, inverted linkages, *etc.),* 3' end modifications *(e.g.,* conjugation, inverted linkages, *etc.),* (b) base modifications, *e.g.* , replacement with modified bases, stabilizing bases, destabilizing bases, or bases that base pair with an expanded repertoire of partners, or conjugated bases, (c) sugar modifications (e.g., at the 2' position or 4' position) or replacement of the sugar, and (d) internucleoside linkage modifications, including modification or replacement of the phosphodiester linkages. The term "ribonucleotide" also encompasses ribonucleotide triphosphates including modified and non-modified ribonucleotide triphosphates.

***Ribonucleic acid (RNA):*** As used herein, the term "RNA" refers to a polymer of ribonucleotides. In some embodiments, an RNA is single stranded. In some embodiments, an RNA is double stranded. In some embodiments, an RNA comprises both single and double stranded portions. In some embodiments, an RNA can comprise a backbone structure as described in the definition of *"Nucleic acid* / *Polynucleotide"* above. An RNA can be a regulatory RNA *(e.g.,* siRNA, microRNA, *etc.),* or a messenger RNA (mRNA). In some embodiments where an RNA is a mRNA. In some embodiments where an RNA is a mRNA, a RNA typically comprises at its 3' end a poly(A) region. In some embodiments where an RNA is a mRNA, an RNA typically comprises at its 5' end an art-recognized cap structure, e.g., for recognizing and attachment of a mRNA to a ribosome to initiate translation. In some embodiments, a RNA is a synthetic RNA. Synthetic RNAs include RNAs that are synthesized *in vitro* (*e.g.,* by enzymatic synthesis methods and/or by chemical synthesis methods).

***Sample:*** As used herein, the term "sample" typically refers to an aliquot of material obtained or derived from a source of interest. In some embodiments, a source of interest is a biological or environmental source. In some embodiments, a source of interest may be or comprise a cell, tissue, or organism, such as a microbe, a plant, or an animal (e.g., a human). In some embodiments, a source of interest is or comprises biological tissue or fluid. In some embodiments, a source of interest may be or comprise a preparation generated in a production run. In some embodiments, a sample is a "primary sample" obtained directly from a source of interest by any appropriate means. In some embodiments, as will be clear from context, the term "sample" refers to a preparation that is obtained by processing (e.g., by removing one or more components of and/or by adding one or more agents to) a primary sample.

***Substituted*** or ***optionally substituted:*** As described herein, compounds of the invention may contain "optionally substituted" moieties. In general, the term "substituted," whether preceded by the term "optionally" or not, means that one or more hydrogens of the designated moiety are replaced with a suitable substituent. "Substituted" applies to one or more hydrogens that are either explicit or implicit from the structure (e.g., 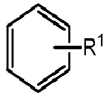 refers to at least 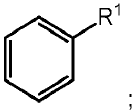 and 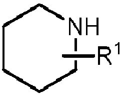 refers to at least 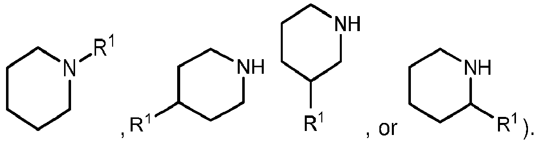 Unless otherwise indicated, an "optionally substituted" group may have a suitable substituent at each substitutable position of the group, and when more than one position in any given structure may be substituted with more than one substituent selected from a specified group, the substituent may be either the same or different at every position. Combinations of substituents envisioned by this invention are preferably those that result in the formation of stable or chemically feasible compounds. The term "stable," as used herein, refers to compounds that are not substantially altered when subjected to conditions to allow for their production, detection, and, in certain embodiments, their recovery, purification, and use for one or more of the purposes provided herein. Groups described as being "substituted" preferably have between 1 and 4 substituents, more preferably 1 or 2 substituents. Groups described as being "optionally substituted" may be unsubstituted or be "substituted" as described above.

Suitable monovalent substituents on a substitutable carbon atom of an "optionally substituted" group are independently halogen; -(CH₂)₀₋₄R°; -(CH₂)₀₋₄OR°; -O(CH₂)₀₋₄R°, -O-(CH₂)₀₋₄C(O)OR°; -(CH₂)₀₋₄CH(OR°)₂; -(CH₂)₀₋₄SR°; -(CH₂)₀₋₄Ph, which may be substituted with R°; -(CH₂)₀₋₄O(CH₂)₀₋₁Ph which may be substituted with R°; - CH=CHPh, which may be substituted with R°; -(CH₂)₀₋₄O(CH₂)₀₋₁-pyridyl which may be substituted with R°; -NO₂; -CN; -N₃; -(CH₂)₀₋₄N(R°)₂; -(CH₂)₀₋₄N(R°)C(O)R°; - N(R°)C(S)R°; -(CH₂)₀₋₄N(R°)C(O)NR°₂; -N(R°)C(S)NR°₂; -(CH₂)₀₋₄N(R°)C(O)OR°; - N(R°)N(R°)C(O)R°; -N(R°)N(R°)C(O)NR°₂; -N(R°)N(R°)C(O)OR°; -(CH₂)₀₋₄C(O)R°; C(S)R°; -(CH₂)₀₋₄C(O)OR°; -(CH₂)₀₋₄C(O)SR°; -(CH₂)₀₋₄C(O)OSiR°₃; -(CH₂)₀₋₄OC(O)R°; -OC(O)(CH₂)₀₋₄SR°; -(CH₂)₀₋₄SC(O)R°; -(CH₂)₀₋₄C(O)NR°₂; -C(S)NR°₂; - C(S)SR°; -SC(S)SR°, -(CH₂)₀₋₄OC(O)NR°₂; -C(O)N(OR°)R°; -C(O)C(O)R°; - C(O)CH₂C(O)R°; -C(NOR°)R°; -(CH₂)₀₋₄SSR°; -(CH₂)₀₋₄S(O)₂R°; -(CH₂)₀₋₄S(O)₂OR°; - (CH₂)₀₋₄OS(O)₂R°; -S(O)₂NR°₂; -(CH₂)₀₋₄S(O)R°; -N(R°)S(O)₂NR°₂; -N(R°)S(O)₂R°; - N(OR°)R°; -C(NH)NR°₂; -P(O)₂R°; -P(O)R°₂; -OP(O)R°₂; -OP(O)(OR°)₂; SiR°₃; -(C₁₋₄ straight or branched alkylene)O-N(R°)₂; or -(C₁₋₄ straight or branched alkylene)C(O)ON(R°)₂, wherein each R° may be substituted as defined below and is independently hydrogen, C₁₋₆ aliphatic, -CH₂Ph, -O(CH₂)₀₋₁Ph, -CH₂-(5- to 6-membered heteroaryl ring), or a 3- to 6-membered saturated, partially unsaturated, or aryl ring having 0-4 heteroatoms independently selected from nitrogen, oxygen, or sulfur, or, notwithstanding the definition above, two independent occurrences of R°, taken together with their intervening atom(s), form a 3- to 12-membered saturated, partially unsaturated, or aryl mono- or bicyclic ring having 0-4 heteroatoms independently selected from nitrogen, oxygen, or sulfur, which may be substituted as defined below.

Suitable monovalent substituents on R° (or the ring formed by taking two independent occurrences of R° together with their intervening atoms), are independently halogen, - (CH₂)₀₋₂R^{•}, -(haloR^{•}), -(CH₂)₀₋₂OH, -(CH₂)₀₋₂OR^{•}, -(CH₂)₀₋₂CH(OR^{•})₂, -O(haloR^{•}), - CN, -N₃, -(CH₂)₀₋₂C(O)R^{•}, -(CH₂)₀₋₂C(O)OH, -(CH₂)₀₋₂C(O)OR^{•}, -(CH₂)₀₋₂SR^{•}, - (CH₂)₀₋₂SH, -(CH₂)₀₋₂NH₂, -(CH₂)₀₋₂NHR^{•}, -(CH₂)₀₋₂NR^{•}₂, -NO₂, -SiR^{•}₃, - OSiR^{•}₃, -C(O)SR^{•}, -(C₁₋₄ straight or branched alkylene)C(O)OR^{•}, or -SSR^{•} wherein each R^{•} is unsubstituted or where preceded by "halo" is substituted only with one or more halogens, and is independently selected from C₁₋₄ aliphatic, -CH₂Ph, -O(CH₂)₀₋₁Ph, or a 3- to 6-membered saturated, partially unsaturated, or aryl ring having 0-4 heteroatoms independently selected from nitrogen, oxygen, or sulfur. Suitable divalent substituents on a saturated carbon atom of R° include =O and =S.

Suitable divalent substituents on a saturated carbon atom of an "optionally substituted" group include the following: =O ("oxo"), =S, =NNR*₂, =NNHC(O)R*, =NNHC(O)OR*, =NNHS(O)₂R*, =NR*, =NOR*, -O(C(R*₂))₂₋₃O-, or -S(C(R*₂))₂₋₃S-, wherein each independent occurrence of R* is selected from hydrogen, C₁₋₆ aliphatic which may be substituted as defined below, or an unsubstituted 5- to 6-membered saturated, partially unsaturated, or aryl ring having 0-4 heteroatoms independently selected from nitrogen, oxygen, or sulfur. Suitable divalent substituents that are bound to vicinal substitutable carbons of an "optionally substituted" group include: -O(CR*₂)₂₋₃O-, wherein each independent occurrence of R* is selected from hydrogen, C₁₋₆ aliphatic which may be substituted as defined below, or an unsubstituted 5-6-membered saturated, partially unsaturated, or aryl ring having 0-4 heteroatoms independently selected from nitrogen, oxygen, or sulfur.

Suitable substituents on the aliphatic group of R* include halogen, -R^{•}, -(haloR^{•}), -OH, -OR^{•}, -O(haloR^{•}), -CN, -C(O)OH, -C(O)OR^{•}, -NH₂, -NHR^{•}, -NR^{•}₂, or -NO₂, wherein each R^{•} is unsubstituted or where preceded by "halo" is substituted only with one or more halogens, and is independently C₁₋₄ aliphatic, -CH₂Ph, -O(CH₂)₀₋₁ Ph, or a 5- to 6-membered saturated, partially unsaturated, or aryl ring having 0-4 heteroatoms independently selected from nitrogen, oxygen, or sulfur.

Suitable substituents on a substitutable nitrogen of an "optionally substituted" group include -R^{†}, -NR^{†}₂, -C(O)R^{†}, -C(O)OR^{†}, -C(O)C(O)R^{†}, - C(O)CH₂C(O)R^{†}, -S(O)₂R^{†}, -S(O)₂NR^{†}₂, -C(S)NR^{†}₂, -C(NH)NR^{†}₂, or -N(R^{†})S(O)₂R^{†}; wherein each R^{†} is independently hydrogen, C₁₋₆ aliphatic which may be substituted as defined below, unsubstituted -OPh, or an unsubstituted 3- to 6-membered saturated, partially unsaturated, or aryl ring having 0-4 heteroatoms independently selected from nitrogen, oxygen, or sulfur, or, notwithstanding the definition above, two independent occurrences of R^{†}, taken together with their intervening atom(s) form an unsubstituted 3-to 12-membered saturated, partially unsaturated, or aryl mono- or bicyclic ring having 0-4 heteroatoms independently selected from nitrogen, oxygen, or sulfur.

Suitable substituents on the aliphatic group of R^{†} are independently halogen, - R^{•}, -(haloR^{•}), -OH, -OR^{•}, -O(haloR^{•}), -CN, -C(O)OH, -C(O)OR^{•}, -NH₂, -NHR^{•}, - NR^{•}₂, or -NO₂, wherein each R^{•} is unsubstituted or where preceded by "halo" is substituted only with one or more halogens, and is independently C₁₋₄ aliphatic, -CH₂Ph, -O(CH₂)₀₋₁ Ph, or a 3- to 6-membered saturated, partially unsaturated, or aryl ring having 0-4 heteroatoms independently selected from nitrogen, oxygen, or sulfur.

***Small molecule:*** As used herein, the term "small molecule" means a low molecular weight organic and/or inorganic compound. In general, a "small molecule" is a molecule that is less than about 5 kilodaltons (kD) in size. In some embodiments, a small molecule is less than about 4 kD, 3 kD, about 2 kD, or about 1 kD. In some embodiments, the small molecule is less than about 800 daltons (D), about 600 D, about 500 D, about 400 D, about 300 D, about 200 D, or about 100 D. In some embodiments, a small molecule is less than about 2000 g/mol, less than about 1500 g/mol, less than about 1000 g/mol, less than about 800 g/mol, or less than about 500 g/mol. In some embodiments, a small molecule is not a polymer.

In some embodiments, a small molecule does not include a polymeric moiety. In some embodiments, a small molecule is not and/or does not comprise a protein or polypeptide (e.g., is not an oligopeptide or peptide). In some embodiments, a small molecule is not and/or does not comprise a polynucleotide (e.g., is not an oligonucleotide). In some embodiments, a small molecule is not and/or does not comprise a polysaccharide; for example, in some embodiments, a small molecule is not a glycoprotein, proteoglycan, glycolipid, *etc.*)*.* In some embodiments, a small molecule is not a lipid.

In some embodiments, a small molecule is a modulating agent (e.g., is an inhibiting agent or an activating agent). In some embodiments, a small molecule is biologically active. In some embodiments, a small molecule is detectable (e.g., comprises at least one detectable moiety). In some embodiments, a small molecule is a therapeutic agent. Those of ordinary skill in the art, reading the present disclosure, will appreciate that certain small molecule compounds described herein may be provided and/or utilized in any of a variety of forms such as, for example, crystal forms (e.g., polymorphs, solvates, etc), salt forms, protected forms, pro-drug forms, ester forms, isomeric forms (e.g., optical and/or structural isomers), isotopic forms, etc.

Those of ordinary skill in the art will appreciate that certain small molecule compounds (e.g., oligosaccharide compounds described herein) have structures that can exist in one or more steroisomeric forms. In some embodiments, such a small molecule may be utilized in accordance with the present disclosure in the form of an individual enantiomer, diastereomer or geometric isomer, or may be in the form of a mixture of stereoisomers; in some embodiments, such a small molecule may be utilized in accordance with the present disclosure in a racemic mixture form.

Those of skill in the art will appreciate that certain small molecule compounds (e.g., oligosaccharide compounds described herein) have structures that can exist in one or more tautomeric forms. In some embodiments, such a small molecule may be utilized in accordance with the present disclosure in the form of an individual tautomer, or in a form that interconverts between tautomeric forms.

Those of skill in the art will appreciate that certain small molecule compounds (e.g., oligosaccharide compounds described herein) have structures that permit isotopic substitution (e.g., ²H or ³H for H; ¹¹C, ¹³C or ¹⁴C for ¹²C; ¹³N or ¹⁵N for ¹⁴N; ¹⁷O or ¹⁸O for ¹⁶O; ³⁶Cl for ³⁵Cl or ³⁷Cl; ¹⁸F for ¹⁹F; ¹³¹I for ¹²⁷I; etc.). In some embodiments, such a small molecule may be utilized in accordance with the present disclosure in one or more isotopically modified forms, or mixtures thereof.

In some embodiments, reference to a particular small molecule compound (e.g., oligosaccharide compounds described herein) may relate to a specific form of that compound. In some embodiments, a particular small molecule compound may be provided and/or utilized in a salt form (e.g., in an acid-addition or base-addition salt form, depending on the compound); in some such embodiments, the salt form may be a pharmaceutically acceptable salt form.

In some embodiments, where a small molecule compound is one that exists or is found in nature, that compound may be provided and/or utilized in accordance in the present disclosure in a form different from that in which it exists or is found in nature. Those of ordinary skill in the art will appreciate that, in some embodiments, a preparation of a particular small molecule compound that contains an absolute or relative amount of the compound, or of a particular form thereof, that is different from the absolute or relative (with respect to another component of the preparation including, for example, another form of the compound) amount of the compound or form that is present in a reference preparation of interest (e.g., in a primary sample from a source of interest such as a biological or environmental source) is distinct from the compound as it exists in the reference preparation or source. Thus, in some embodiments, for example, a preparation of a single stereoisomer of a small molecule compound may be considered to be a different form of the compound than a racemic mixture of the compound; a particular salt of a small molecule compound may be considered to be a different form from another salt form of the compound; a preparation that contains only a form of the compound that contains one conformational isomer ((Z) or (E)) of a double bond may be considered to be a different form of the compound from one that contains the other conformational isomer ((E) or (Z)) of the double bond; a preparation in which one or more atoms is a different isotope than is present in a reference preparation may be considered to be a different form; etc.

Those skilled in the art will further appreciate that, in small molecule structures, the symbol 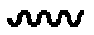 , as used herein, refers to a point of attachment between two atoms.

***Therapeutic agent:*** As used herein, the phrase "therapeutic agent" in general refers to any agent that elicits a desired pharmacological effect when administered to an organism. In some embodiments, an agent is considered to be a therapeutic agent if it demonstrates a statistically significant effect across an appropriate population. In some embodiments, the appropriate population may be a population of model organisms. In some embodiments, an appropriate population may be defined by various criteria, such as a certain age group, gender, genetic background, preexisting clinical conditions, etc. In some embodiments, a therapeutic agent is a substance that can be used to alleviate, ameliorate, relieve, inhibit, prevent, delay onset of, reduce severity of, and/or reduce incidence of one or more symptoms or features of a disease, disorder, and/or condition. In some embodiments, a "therapeutic agent" is an agent that has been or is required to be approved by a government agency before it can be marketed for administration to humans. In some embodiments, a "therapeutic agent" is an agent for which a medical prescription is required for administration to humans.

***Treat:*** As used herein, the terms "treat," "treatment," or "treating" refer to any method used to partially or completely alleviate, ameliorate, relieve, inhibit, prevent, delay onset of, reduce severity of, and/or reduce incidence of one or more symptoms or features of a disease, disorder, and/or condition. Treatment may be administered to a subject who does not exhibit signs of a disease, disorder, and/or condition. In some embodiments, treatment may be administered to a subject who exhibits only early signs of the disease, disorder, and/or condition, for example, for the purpose of decreasing the risk of developing pathology associated with the disease, disorder, and/or condition.

### Polvcationic Oligosaccharide Compositions

The present disclosure, provides, among other things, compositions comprising an oligosaccharide of formula **I:** 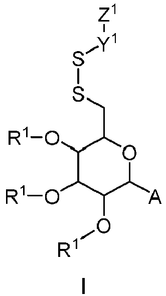 or a pharmaceutically acceptable salt thereof, wherein:
A is A¹, A², or A³: 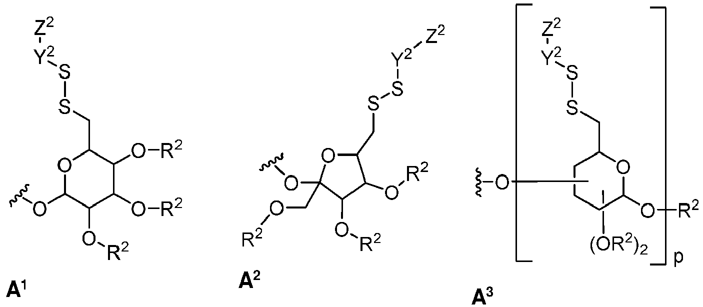
R¹ and R² are each independently selected from H, R^{a}, and -C(O)-R^{a}, and wherein at least one instance of R¹ or R² is not H;
each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b};
each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, and -NHC(O)NHR^{c};
each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S;
Y¹ and Y² are each independently an optionally substituted C₁₋₃₀ aliphatic group wherein one or more carbons are optionally and independently replaced by -Cy-, -NH-, -NHC(O)-, -C(O)NH-, -NHC(O)O-, -OC(O)NH-, -NHC(O)NH-, -NHC(S)NH-, -C(S)NH- - C(O)NHSO₂-, -SO₂NHC(O)-,-OC(O)O-, -O-, -C(O)-, -OC(O)-, -C(O)O-, -SO-, or -SO₂-; each Cy is independently an optionally substituted C₃-C₁₄ cycloaliphatic, 5- to 14-membered heterocyclyl ring having 1-3 heteroatoms selected from N, O, S, 5- to 14-membered heteroaryl ring having 1-3 heteroatoms selected from N, O, S;
Z¹ and Z² are each independently a cationic or ionizable group selected from optionally substituted 5- to 14-membered heterocyclyl ring having 1-3 heteroatoms selected from N, O, and S, 5- to 14-membered heteroaryl ring having 1-3 heteroatoms selected from N, O, and S, -N+(M)₃, 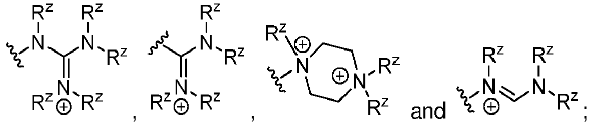
each M is independently -C₀-C₆ aliphatic-R^{z} or -C₀-C₆ aliphatic-N⁺(R^{z})₃;
each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S; and
p is an integer selected from 1, 2, 3, 4, or 5.

As described herein, A is A¹, A², or A³:

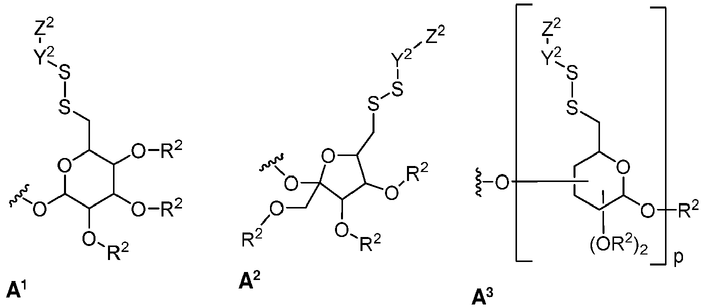

In some embodiments, A is A¹ or A². In some embodiments, A is A¹. In some embodiments, A is A². In some embodiments, A is A³. In some embodiments, A is A¹, and the oligosaccharide is of formula la: 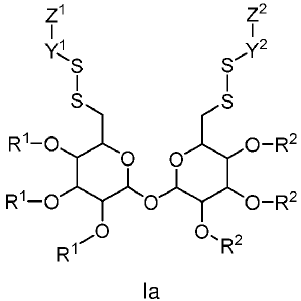 or a pharmaceutically acceptable salt thereof, wherein R¹, R², Y¹, Y², Z¹, and Z² are as described in classes and subclasses herein, both singly and in combination.

In some embodiments, A is A², and an oligosaccharide is of formula Ib: 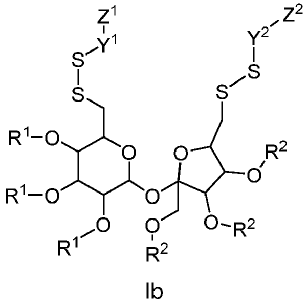 or a pharmaceutically acceptable salt thereof, wherein R¹, R², Y¹, Y², Z¹, and Z² are as described in classes and subclasses herein, both singly and in combination.

In some embodiments, A is A³, and an oligosaccharide is of formula Ic: 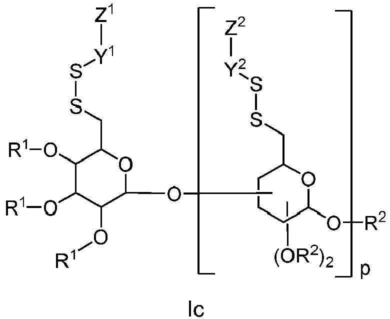 or a pharmaceutically acceptable salt thereof, wherein p, R¹, R², Y¹, Y², Z¹, and Z² are as described in classes and subclasses herein, both singly and in combination.

In some embodiments, p is 1, and an oligosaccharide is of formula Ic-i: 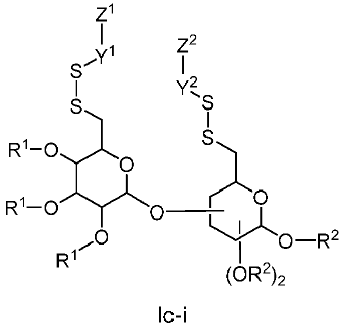 or a pharmaceutically acceptable salt thereof, wherein R¹, R², Y¹, Y², Z¹, and Z² are as described in classes and subclasses herein, both singly and in combination.

In some embodiments, p is 2, and an oligosaccharide is of formula Ic-ii: 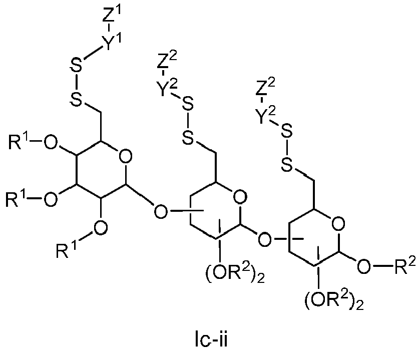 or a pharmaceutically acceptable salt thereof, wherein R¹, R², Y¹, Y², Z¹, and Z² are as described in classes and subclasses herein, both singly and in combination.

In some embodiments, p is 3, and an oligosaccharide is of formula Ic-iii: 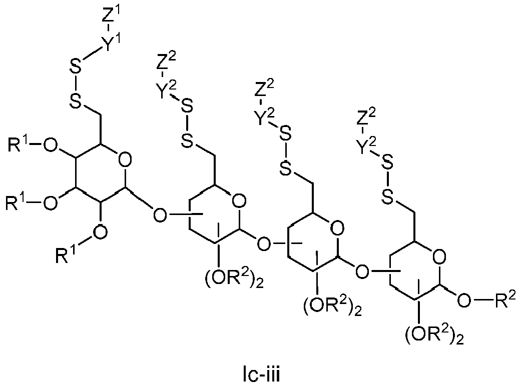 or a pharmaceutically acceptable salt thereof, wherein R¹, R², Y¹, Y², Z¹, and Z² are as described in classes and subclasses herein, both singly and in combination.

In some embodiments, p is 4, and an oligosaccharide is of formula Ic-iv: 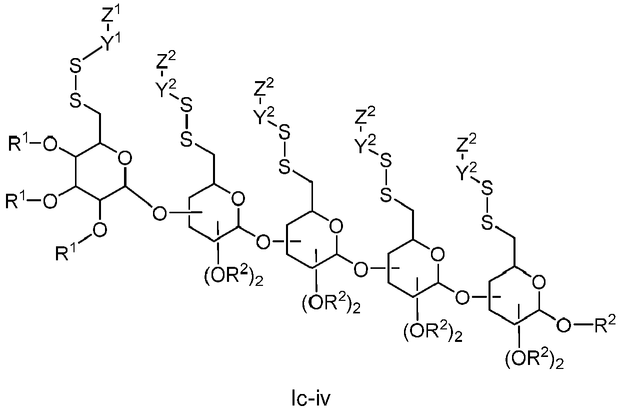 or a pharmaceutically acceptable salt thereof, wherein R¹, R², Y¹, Y², Z¹, and Z² are as described in classes and subclasses herein, both singly and in combination.

In some embodiments, p is 5, and an oligosaccharide is of formula Ic-v: 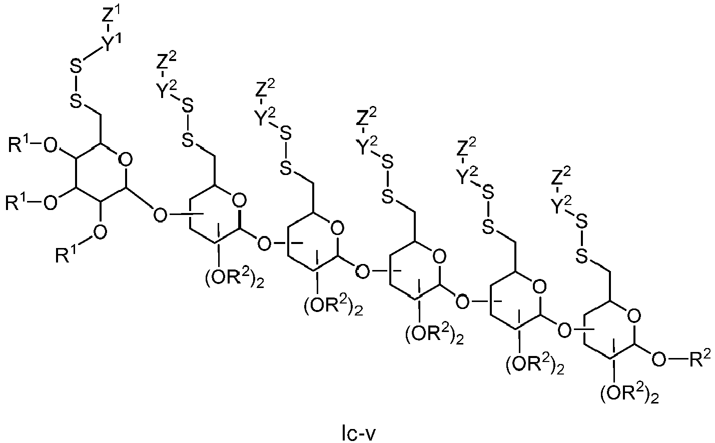 or a pharmaceutically acceptable salt thereof, wherein R¹, R², Y¹, Y², Z¹, and Z² are as described in classes and subclasses herein, both singly and in combination.

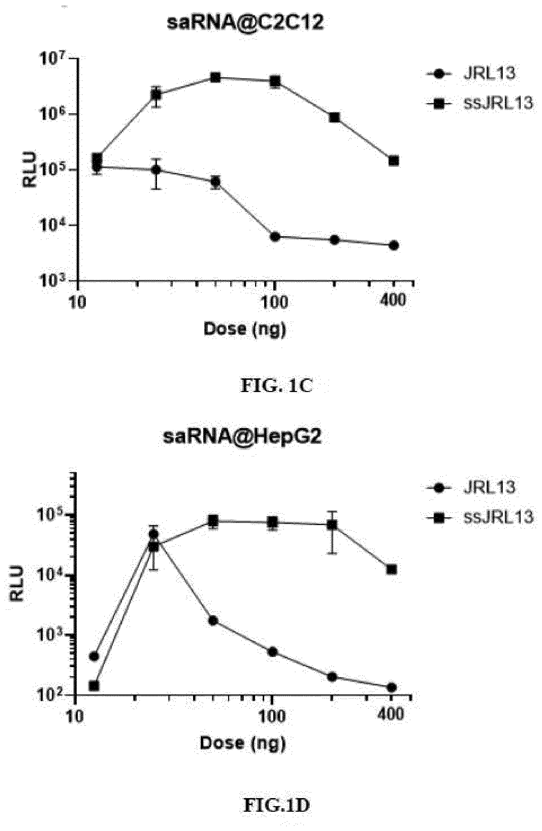

The presently provided oligosaccharide compositions comprise a disulfide moiety, which, surprisingly and unexpectedly, improves expression of RNA in a target relative to use of an analogous oligosaccharide that lacks such a disulfide moiety. See, for example, FIGs. 1C and 1D in Example 1. The present oligosaccharide compositions, without being bound by theory, are believed to reduce inherent cytotoxicity of other cationic compositions used for nucleic acid delivery.

As described generally herein R¹ and R² are each independently selected from H, R^{a}, and -C(O)-R^{a}, and wherein at least one instance of R¹ or R² is not H. In some embodiments, R¹ and R² are each independently selected from R^{a} and -C(O)-R^{a}. In some embodiments, each of R¹ and R² is R^{a}. In some embodiments, each of R¹ and R² is R^{a}, and each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b}.

In some embodiments, each of R¹ and R² is R^{a}, and R^{a} is C₁-C₂₀ aliphatic optionally substituted with one or more R^{b}. In some embodiments, each of R¹ and R² is R^{a}, and each R^{a} is C₁-C₁₄ aliphatic optionally substituted with one or more R^{b}. In some embodiments, each of R¹ and R² is R^{a}, and each R^{a} is C₁-C₁₀ aliphatic optionally substituted with one or more R^{b}. In some embodiments, each of R¹ and R² is R^{a}, and R^{a} is C₅-C₁₀ aliphatic optionally substituted with one or more R^{b}. In some embodiments, each of R¹ and R² is R^{a}, and R^{a} is C₅-C₁₀ alkyl optionally substituted with one or more R^{b}. In some embodiments, each of R¹ and R² is R^{a}, and R^{a} is C₅-C₁₀ linear alkyl. In some embodiments, each of R¹ and R² is R^{a}, and R^{a} is methyl, ethyl, n-propyl, n-butyl, n-pentyl, n-hexyl, n-heptyl, n-octyl, or n-nonyl. In some embodiments, each of R¹ and R² is R^{a}, and each R^{a} is independently selected from

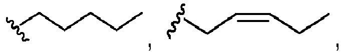

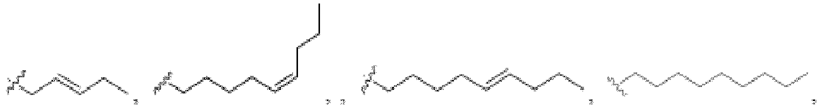

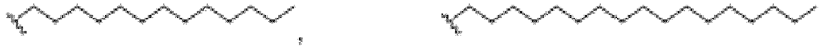

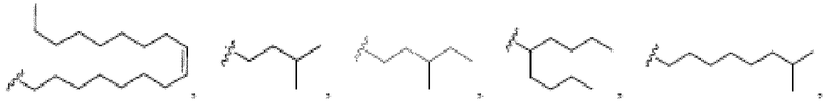

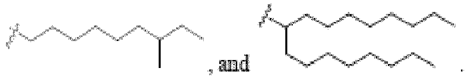

In some embodiments, each of R¹ and R² is R^{a}, and each R^{a} is C₃-C₂₀ cycloaliphatic, optionally substituted with one or more R^{b}. In some embodiments, each of R¹ and R² is R^{a}, and each R^{a} is C₃-C₆ cycloaliphatic optionally substituted with one or more R^{b}. In some embodiments, each of R¹ and R² is R^{a}, and R^{a} is cyclopropyl, cyclobutyl, cyclopentyl, or cyclohexyl.

In some embodiments, each of R¹ and R² is R^{a}, and R^{a} is C₅-C₆ aryl optionally substituted with one or more R^{b}. In some embodiments, each of R¹ and R² is R^{a}, and R^{a} is phenyl. In some embodiments, each of R¹ and R² is R^{a}, and R^{a} is 3- to 12-membered heterocyclyl comprising 1 to 3 heteroatoms selected from N, O, and S optionally substituted with one or more R^{b}. In some embodiments, each of R¹ and R² is R^{a}, and R^{a} is 3- to 6-membered heterocyclyl comprising 1 to 3 heteroatoms selected from N, O, and S optionally substituted with one or more R^{b}.

In some embodiments, each of R¹ and R² is -C(O)-R^{a}, and each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b}.

In some embodiments, each of R¹ and R² is -C(O)-R^{a}. In some embodiments, each R¹ and R² is -C(O)-R^{a}, and each R^{a} is independently a C₁-C₂₀ aliphatic optionally substituted with one or more R^{b}. In some embodiments, each of R¹ and R² is -C(O)-R^{a}, and each R^{a} is independently C₁-C₁₄ aliphatic optionally substituted with one or more R^{b}. In some embodiments, each of R¹ and R² is -C(O)-R^{a}, and each R^{a} is independently C₁-C₁₀ aliphatic optionally substituted with one or more R^{b}. In some embodiments, each of R¹ and R² is -C(O)-R^{a}, and each R^{a} is independently C₅-C₁₀ aliphatic optionally substituted with one or more R^{b}. In some embodiments, each of R¹ and R² is -C(O)-R^{a}, and each R^{a} is independently C₅-C₁₀ alkyl optionally substituted with one or more R^{b}. In some embodiments, each of R¹ and R² is -C(O)-R^{a}, and each R^{a} is independently C₅-C₁₀ linear alkyl. In some embodiments, each of R¹ and R² is -C(O)-R^{a}, and each R^{a} is independently selected from methyl, ethyl, n-propyl, n-butyl, n-pentyl, n-hexyl, n-heptyl, n-octyl, and n-nonyl. In some embodiments, each of R¹ and R² is -C(O)-R^{a}, and each R^{a} is independently selected from 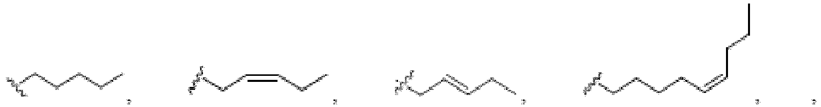 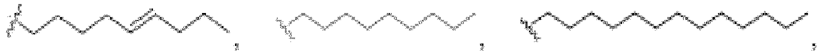 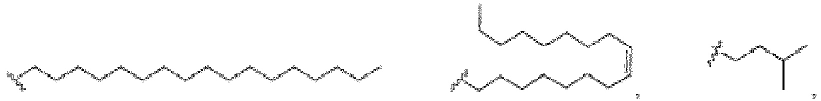 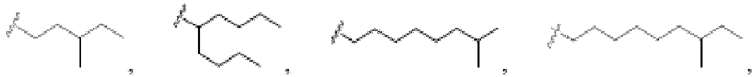 and

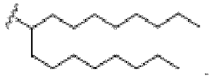

In some embodiments, each of R¹ and R² is -C(O)-R^{a}, and each R^{a} is independently a C₃-C₂₀ cycloaliphatic, optionally substituted with one or more R^{b}. In some embodiments, each of R¹ and R² is -C(O)-R^{a}, and each R^{a} is independently aC₃-C₆ cycloaliphatic optionally substituted with one or more R^{b}. In some embodiments, each of R¹ and R² is -C(O)-R^{a}, and each R^{a} is independently selected from cyclopropyl, cyclobutyl, cyclopentyl, or cyclohexyl. In some embodiments, each of R¹ and R² is -C(O)-R^{a}, and each R^{a} is independently a polycyclic C₁₀-C₂₀ cycloaliphatic, optionally substituted with one or more R^{b}. In some embodiments, one or more of R¹ or R² is:

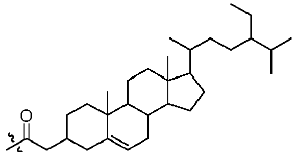

In some embodiments, each R¹ and R² is -C(O)-R^{a}, and each R^{a} is independently a C₅-C₆ aryl optionally substituted with one or more R^{b}. In some embodiments, each of R¹ and R² is -C(O)-R^{a}, and R^{a} is phenyl.

In some embodiments, each of R¹ and R² is -C(O)-R^{a}, and each R^{a} is independently a 3-to 12-membered heterocyclyl comprising 1 to 3 heteroatoms selected from N, O, and S optionally substituted with one or more R^{b}. In some embodiments, each of R¹ and R² is -C(O)-R^{a}, and each R^{a} is independently a 3- to 6-membered heterocyclyl comprising 1 to 3 heteroatoms selected from N, O, and S optionally substituted with one or more R^{b}. In some embodiments, each of R¹ and R² is independently selected from: 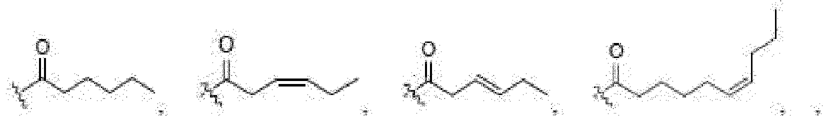 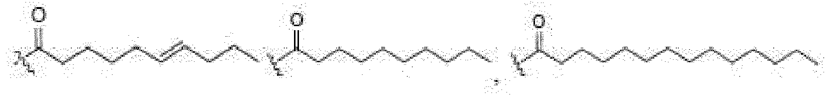 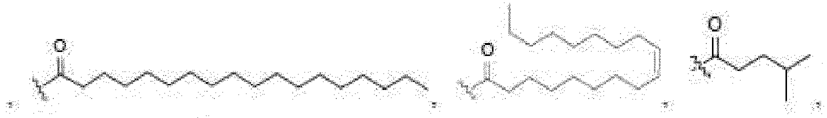 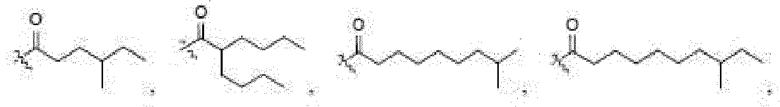 and

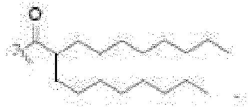

As described herein, each R^{b} is independently selected from halogen, -N₃, -R^{c}, -OR^{c}, - SR^{c}, -NHR^{c}, -C(O)-R^{c}, -OC(O)R^{c}, -NHC(O)R^{c}, -C(O)NHR^{c}, and -NHC(O)NHR^{c}. In some embodiments, R^{b} is halogen. In some embodiments, R^{b} is -N₃. In some embodiments, R^{b} is R^{c}. In some embodiments, R^{b} is -OR^{c}. In some embodiments, R^{b} is -SR^{c}. In some embodiments, R^{b} is -NHR^{c}. In some embodiments, R^{b} is -C(O)-R^{c}. In some embodiments, R^{b} is -OC(O)R^{c}. In some embodiments, R^{b} is -NHC(O)R^{c}. In some embodiments, R^{b} is -C(O)NHR^{c}. In some embodiments, R^{b} is -NHC(O)NHR^{c}.

As described herein, each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S. In some embodiments, R^{c} is optionally substituted C₁-C₂₀ aliphatic. In some embodiments, R^{c} is optionally substituted C₃-C₂₀ cycloaliphatic. In some embodiments, R^{c} is optionally substituted C₅-C₆ aryl. In some embodiments, R^{c} is optionally substituted 3- to 12-membered heterocyclyl comprising 1 to 3 heteroatoms selected from N, O, and S. In some embodiments, R^{c} is optionally substituted 4- to 12-membered heteroaryl comprising 1 to 3 heteroatoms selected from N, O, and S.

As described herein, Y¹ and Y² are each independently an optionally substituted C₁₋₃₀ aliphatic group wherein one or more carbons of a Y¹ and/or Y² group are optionally and independently replaced by -Cy-, -NH-, -NHC(O)-, -C(O)NH-, -NHC(O)O-, -OC(O)NH-, - NHC(O)NH-, -NHC(S)NH-, -C(S)NH- -C(O)NHSO₂-, -SO₂NHC(O)-,-OC(O)O-, -O-, - C(O)-, -OC(O)-, -C(O)O-, -SO-, or -SO₂-.

In some embodiments, Y¹ and Y² are each independently selected from C₁-C₁₀ aliphatic, C₀-C₄-aliphatic-NHC(O)NH-C₀-C₄ aliphatic, C₀-C₄-aliphatic-NHC(S)NH-C₀-C₄ aliphatic, C₀-C₄-aliphatic-C(O)NH-C₀-C₄ aliphatic, C₀-C₄-aliphatic-C(S)NH-C₀-C₄ aliphatic, C₀-C₄-aliphatic-NHC(O)-C₀-C₄ aliphatic, C₀-C₄-aliphatic-NHSO₂-C₀-C₄ aliphatic, and C₀-C₄-aliphatic-C(O)-C₀-C₄ aliphatic.

In some embodiments, Y¹ and Y² are each C₁-C₁₀ aliphatic. In some embodiments, Y¹ and Y² are each methylene, ethylene, propylene, or butylene. In some embodiments, Y¹ and Y² are each -CH₂-CH₂-.

In some embodiments, Y¹ and Y² are each C₀-C₄-aliphatic-NHC(O)NH-C₀-C₄ aliphatic. In some embodiments, Y¹ and Y² are each C₁-C₄-aliphatic-NHC(O)NH-C₁-C₄ aliphatic. In some embodiments, Y¹ and Y² are each -CH₂-CH₂-NHC(O)NH-CH₂-CH₂-.

In some embodiments, Y¹ and Y² are each C₀-C₄-aliphatic-NHC(S)NH-C₀-C₄ aliphatic. In some embodiments, Y¹ and Y² are each C₁-C₄-aliphatic-NHC(S)NH-C₁-C₄ aliphatic. In some embodiments, Y¹ and Y² are each -CH₂-CH₂-NHC(S)NH-CH₂-CH₂-.

In some embodiments, Y¹ and Y² are each C₀-C₄-aliphatic-C(O)NH-C₀-C₄ aliphatic. In some embodiments, Y¹ and Y² are each C₁-C₄-aliphatic-C(O)NH-C₁-C₄ aliphatic. In some embodiments, Y¹ and Y² are each -CH₂-CH₂-C(O)NH-CH₂-CH₂-.

In some embodiments, Y¹ and Y² are each C₀-C₄-aliphatic-NHC(O)-C₀-C₄ aliphatic. In some embodiments, Y¹ and Y² are each C₁-C₄-aliphatic-NHC(O)-C₁-C₄ aliphatic. In some embodiments, Y¹ and Y² are each -CH₂-CH₂-NHC(O)-CH₂-CH₂-.

In some embodiments, Y¹ and Y² are each C₀-C₄-aliphatic-NHSO₂-C₀-C₄ aliphatic. In some embodiments, Y¹ and Y² are each C₁-C₄-aliphatic-NHSO₂-C₁-C₄ aliphatic. In some embodiments, Y¹ and Y² are each -CH₂-CH₂-NHSO₂-CH₂-CH₂-.

In some embodiments, Y¹ is selected from C₁-C₁₀ aliphatic, C₀-C₄-aliphatic-NHC(O)NH-C₀-C₄ aliphatic, C₀-C₄-aliphatic-NHC(S)NH-C₀-C₄ aliphatic, C₀-C₄-aliphatic-C(O)NH-C₀-C₄ aliphatic, C₀-C₄-aliphatic-C(S)NH-C₀-C₄ aliphatic, C₀-C₄-aliphatic-C(O)NH-C₀-C₄ aliphatic, C₀-C₄-aliphatic-NHSO₂-C₀-C₄ aliphatic, and C₀-C₄-aliphatic-C(O)-C₀-C₄ aliphatic.

In some embodiments, embodiments, Y¹ is C₁-C₁₀ aliphatic. In some embodiments, Y¹ is methylene, ethylene, propylene, or butylene. In some embodiments, Y¹ is -CH₂-CH₂-

In some embodiments, Y¹ is C₀-C₄-aliphatic-NHC(O)NH-C₀-C₄ aliphatic. In some embodiments, Y¹ is C₁-C₄-aliphatic-NHC(O)NH-C₁-C₄ aliphatic. In some embodiments, Y1 is -CH₂-CH₂-NHC(O)NH-CH₂-CH₂-.

In some embodiments, Y¹ is C₀-C₄-aliphatic-NHC(S)NH-C₀-C₄ aliphatic. In some embodiments, Y¹ is C₁-C₄-aliphatic-NHC(S)NH-C₁-C₄ aliphatic. In some embodiments, Y¹ is -CH₂-CH₂-NHC(S)NH-CH₂-CH₂-.

In some embodiments, Y¹ is C₀-C₄-aliphatic-C(O)NH-C₀-C₄ aliphatic. In some embodiments, Y¹ is C₁-C₄-aliphatic-C(O)NH-C₁-C₄ aliphatic. In some embodiments, Y¹ is -CH₂-CH₂-C(O)NH-CH₂-CH₂-.

In some embodiments, Y¹ is C₀-C₄-aliphatic-NHC(O)-C₀-C₄ aliphatic. In some embodiments, Y¹ is C₁-C₄-aliphatic-NHC(O)-C₁-C₄ aliphatic. In some embodiments, Y¹ is -CH₂-CH₂-NHC(O)-CH₂-CH₂-.

In some embodiments, Y¹ is C₀-C₄-aliphatic-NHSO₂-C₀-C₄ aliphatic. In some embodiments, Y¹ is C₁-C₄-aliphatic-NHSO₂-C₁-C₄ aliphatic. In some embodiments, Y¹ is -CH₂-CH₂-NHSO₂-CH₂-CH₂-.

In some embodiments, Y² is selected from C₁-C₁₀ aliphatic, C₀-C₄-aliphatic-NHC(O)NH-C₀-C₄ aliphatic, C₀-C₄-aliphatic-NHC(S)NH-C₀-C₄ aliphatic, C₀-C₄-aliphatic-C(O)NH-C₀-C₄ aliphatic, C₀-C₄-aliphatic-C(S)NH-C₀-C₄ aliphatic, C₀-C₄-aliphatic-C(O)NH-C₀-C₄ aliphatic, C₀-C₄-aliphatic-NHSO₂-C₀-C₄ aliphatic, and C₀-C₄-aliphatic-C(O)-C₀-C₄ aliphatic.

In some embodiments, embodiments, Y² is C₁-C₁₀ aliphatic. In some embodiments, Y² is methylene, ethylene, propylene, or butylene. In some embodiments, Y² is -CH₂-CH₂-

In some embodiments, Y² is C₀-C₄-aliphatic-NHC(O)NH-C₀-C₄ aliphatic. In some embodiments, Y² is C₁-C₄-aliphatic-NHC(O)NH-C₁-C₄ aliphatic. In some embodiments, Y² is -CH₂-CH₂-NHC(O)NH-CH₂-CH₂-.

In some embodiments, Y² is C₀-C₄-aliphatic-NHC(S)NH-C₀-C₄ aliphatic. In some embodiments, Y² is C₁-C₄-aliphatic-NHC(S)NH-C₁-C₄ aliphatic. In some embodiments, Y² is -CH₂-CH₂-NHC(S)NH-CH₂-CH₂-.

In some embodiments, Y² is C₀-C₄-aliphatic-C(O)NH-C₀-C₄ aliphatic. In some embodiments, Y² is C₁-C₄-aliphatic-C(O)NH-C₁-C₄ aliphatic. In some embodiments, Y² is -CH₂-CH₂-C(O)NH-CH₂-CH₂-.

In some embodiments, Y² is C₀-C₄-aliphatic-NHC(O)-C₀-C₄ aliphatic. In some embodiments, Y² is C₁-C₄-aliphatic-NHC(O)-C₁-C₄ aliphatic. In some embodiments, Y² is -CH₂-CH₂-NHC(O)-CH₂-CH₂-.

In some embodiments, Y² is C₀-C₄-aliphatic-NHSO₂-C₀-C₄ aliphatic. In some embodiments, Y² is C₁-C₄-aliphatic-NHSO₂-C₁-C₄ aliphatic. In some embodiments, Y² is -CH₂-CH₂-NHSO₂-CH₂-CH₂-.

As described general herein, each Cy is independently an optionally substituted C₃-C₁₄ cycloaliphatic, 5- to 14-membered heterocyclyl ring having 1-3 heteroatoms selected from N, O, S, 5- to 14-membered heteroaryl ring having 1-3 heteroatoms selected from N, O, S.

As described generally herein, Z¹ and Z² are each independently a cationic or ionizable group selected from optionally substituted 5- to 14-membered heterocyclyl ring having 1-3 heteroatoms selected from N, O, and S, 5- to 14-membered heteroaryl ring having 1-3 heteroatoms selected from N, O, and S, -N+(M)₃,

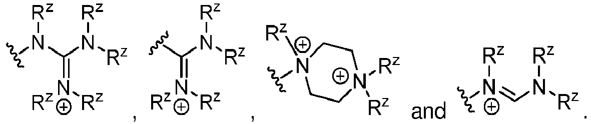

In some embodiments, Z¹ and Z² are each an optionally substituted 5- to 14-membered heterocyclyl ring having 1-3 heteroatoms selected from N, O, and S. In some embodiments, Z¹ and Z² are each an optionally substituted 5- to 6-membered heterocyclyl ring having 1-3 heteroatoms selected from N, O, and S. In some embodiments, Z¹ and Z² are each an optionally substituted 6-membered heterocyclyl ring having 1-3 heteroatoms selected from N, O, and S. In some embodiments, Z¹ and Z² are each selected from optionally substituted piperdinyl, piperazinyl, and morpholinyl.

In some embodiments, Z¹ and Z² are each -N⁺(M)₃. In some embodiments, Z¹ and Z² are each -N⁺(C₀-C₆ aliphatic-R^{z})₃. In some embodiments, Z¹ and Z² are each -N⁺(R^{z})₃. In some embodiments, Z¹ and Z² are each -N⁺H₃. In some embodiments, Z¹ and Z² are each -N⁺H(R^{z})₂. In some embodiments, Z¹ and Z² are each -N⁺H(C₁-C₆ aliphatic)₂. In some embodiments, Z¹ and Z² are each -N⁺(H)₂CH₃. In some embodiments, Z¹ and Z² are each -N⁺H(CH₃)₂. In some embodiments, Z¹ and Z² are each -N⁺(CH₃)₃.

In some embodiments, Z¹ and Z² are each -N⁺(R^{z})₂-C₁-C₆ aliphatic-N⁺(R^{z})₃. In some embodiments, Z¹ and Z² are each -N⁺(R^{z})(C₁-C₆ aliphatic-N⁺(R^{z})₃)₂. In some embodiments, Z¹ and Z² are each:

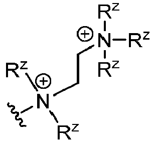

In some embodiments, Z¹ and Z² are each:

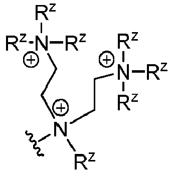

In some embodiments, Z¹ and Z² are each

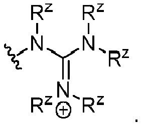

In some embodiments, Z¹ and Z² are each

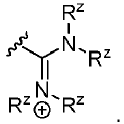

In some embodiments, Z¹ and Z² are each

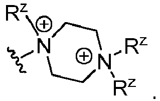

In some embodiments, Z¹ and Z² are each

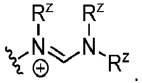

In some embodiments, Z¹ is optionally substituted 5- to 14-membered heterocyclyl ring having 1-3 heteroatoms selected from N, O, and S. In some embodiments, Z¹ is each an optionally substituted 5- to 6-membered heterocyclyl ring having 1-3 heteroatoms selected from N, O, and S. In some embodiments, Z¹ is an optionally substituted 6-membered heterocyclyl ring having 1-3 heteroatoms selected from N, O, and S. In some embodiments, Z¹ is selected from optionally substituted piperdinyl, piperazinyl, and morpholinyl.

In some embodiments, Z¹ is -N⁺(M)₃. In some embodiments, Z¹ is-N⁺(C₀-C₆ aliphatic-R^{z})₃. In some embodiments, Z¹ is-N⁺(R^{z})₃. In some embodiments, Z¹ is -N⁺H₃. In some embodiments, Z¹ is -N⁺H(R^{z})₂. In some embodiments, Z¹ is -N⁺H(C₁-C₆ aliphatic)₂. In some embodiments, Z¹ is -N⁺(H)₂CH₃. In some embodiments, Z¹ is -N⁺H(CH₃)₂. In some embodiments, Z¹ is -N⁺(CH₃)₃.

In some embodiments, Z¹ is -N⁺(R^{z})₂-C₁-C₆ aliphatic-N⁺(R^{z})₃. In some embodiments, Z¹ is -N⁺(R^{z})(C₁-C₆ aliphatic-N⁺(R^{z})₃)₂. In some embodiments, Z¹ is:

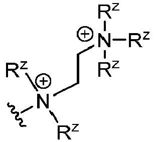

In some embodiments, Z¹ is:

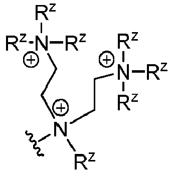

In some embodiments, Z¹ is:

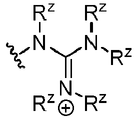

In some embodiments, Z¹ is:

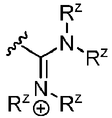

In some embodiments, Z¹ is

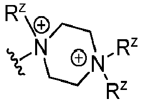

In some embodiments, Z¹ is

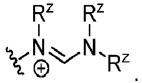

In some embodiments, Z² is optionally substituted 5- to 14-membered heterocyclyl ring having 1-3 heteroatoms selected from N, O, and S. In some embodiments, Z² is each an optionally substituted 5- to 6-membered heterocyclyl ring having 1-3 heteroatoms selected from N, O, and S. In some embodiments, Z² is an optionally substituted 6-membered heterocyclyl ring having 1-3 heteroatoms selected from N, O, and S. In some embodiments, Z² is selected from optionally substituted piperdinyl, piperazinyl, and morpholinyl.

In some embodiments, Z² is -N⁺(M)₃. In some embodiments, Z² is-N⁺(C₀-C₆ aliphatic-R^{z})₃. In some embodiments, Z² is-N⁺(R^{z})₃. In some embodiments, Z² is -N⁺H₃. In some embodiments, Z² is -N⁺H(R^{z})₂. In some embodiments, Z² is -N⁺H(C₁-C₆ aliphatic)₂. In some embodiments, Z² is -N⁺(H)₂CH₃. In some embodiments, Z² is -N⁺H(CH₃)₂. In some embodiments, Z² is -N⁺(CH₃)₃.

In some embodiments, Z² is -N⁺(R^{z})₂-C₁-C₆ aliphatic-N⁺(R^{z})₃. In some embodiments, Z² is -N⁺(R^{z})(C₁-C₆ aliphatic-N⁺(R^{z})₃)₂. In some embodiments, Z² is:

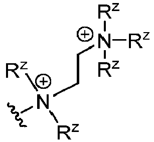

In some embodiments, Z² is:

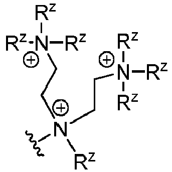

In some embodiments, Z² is:

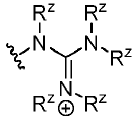

In some embodiments, Z² is:

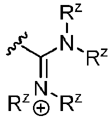

In some embodiments, Z² is

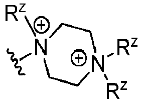

In some embodiments, Z² is

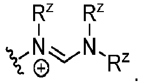

As described generally herein, each M is independently -C₀-C₆ aliphatic-R^{z} or -C₀-C₆ aliphatic-N⁺(R^{z})₃. In some embodiments, each M is C₀-C₆ aliphatic-R^{z}. In some embodiments, each M is -C₀-C₆ aliphatic-N⁺(R^{z})₃.

As described generally herein, each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S.

In some embodiments, each R^{z} is H. In some embodiments, each R^{z} is optionally substituted C₁-C₆ aliphatic. In some embodiments, each R^{z} is optionally substituted C₃-C₂₀ cycloaliphatic. In some embodiments, each R^{z} is optionally substituted C₅-C₆ aryl. In some embodiments, each R^{z} is optionally substituted 3- to 12-membered heterocyclyl comprising 1 to 3 heteroatoms selected from N, O, and S. In some embodiments, each R^{z} is optionally substituted 4- to 12-membered heteroaryl comprising 1 to 3 heteroatoms selected from N, O, and S.

In some embodiments, two or more R^{z} can come together with the atoms to which they are attached to form an optionally substituted 3- to 12-membered heterocyclyl comprising 1 to 3 heteroatoms selected from N, O, and S. In some embodiments, two or more R^{z} can come together with the atoms to which they are attached to form an optionally substituted 4- to 12-membered heteroaryl comprising 1 to 3 heteroatoms selected from N, O, and S.

In some embodiments, an R^{z} on a Z¹ moiety and an R^{z} on a Z² moiety can come together to form a 4- to 6-membered heterocyclic ring having 1 to 3 heteroatoms selected from N, O, and S.

As used herein, a "cationic" moiety is a group having a net positive charge. As used herein, an "ionizable" moiety is a group that may have a neutral charge at a certain pH, but may become charged (e.g., cationic) at a different pH. For example, in some embodiments, an ionizable moiety becomes cationic (i.e., positively charged) at physiological pH (e.g., a pH of about 7.4). Moreover, in some embodiments, a Z¹ and Z² moiety, which is a cationic or ionizable moiety described herein, further comprises one or more suitable counterions (e.g., an anion for each cationic or ionizable moiety). For example, in some embodiments, a Z¹ and Z² moiety comprises a counterion that is a halide, hydroxide, carboxylate, sulfate, phosphate, nitrate, alkyl having from 1 to 6 carbon atoms, sulfonate and aryl sulfonate. In some embodiments, a Z¹ and Z² moiety comprises a counterion that is acetate (CH₃COO⁻), chloride (Cl⁻), iodide (I⁻), bromide (Br⁻), or fluoride (F⁻). Reference to a particular counterion, e.g., a counterion as indicated in Table 1, are intended to refer to encompass the charged moiety in isolation, as well as all chemically feasible counterions.

In some embodiments, Z¹ and Z² are each independently selected from:

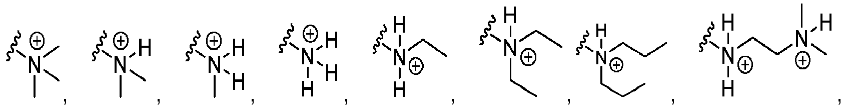

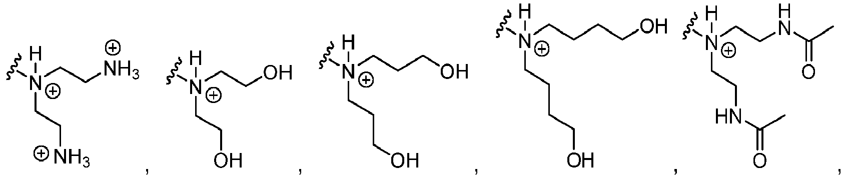

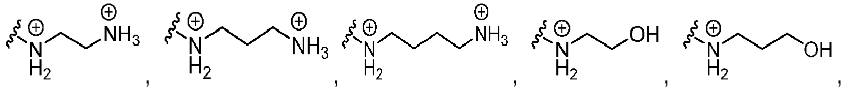

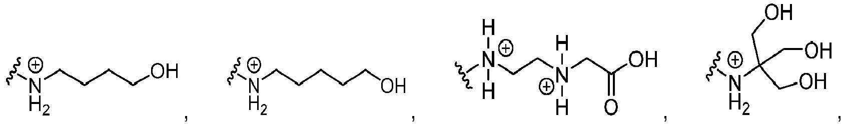

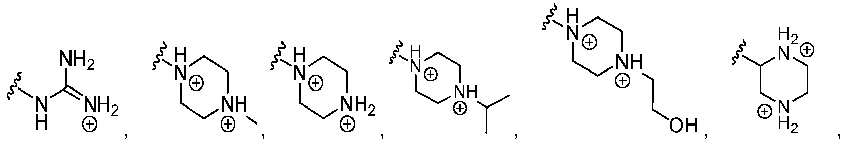

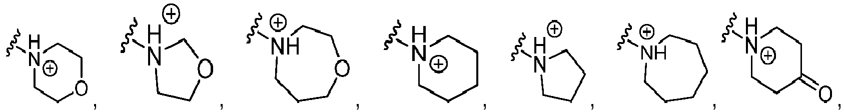

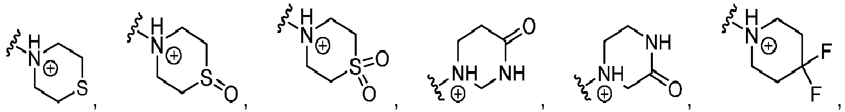

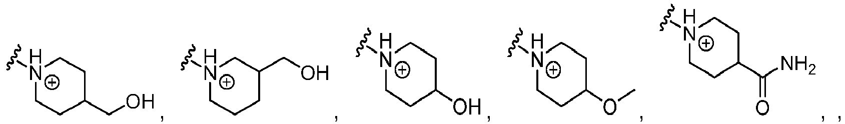

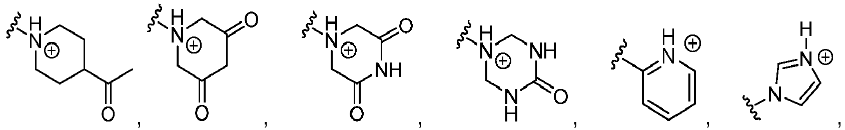

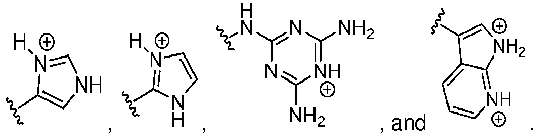

In some embodiments, a compound of formula I is a compound of formula Id: 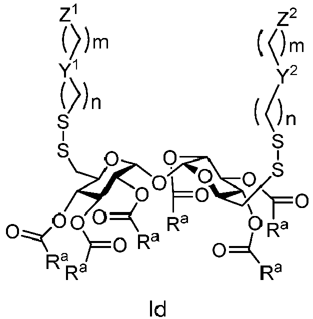 or a pharmaceutically acceptable salt thereof, wherein n and m are each independently selected from 0, 1, 2, 3, 4, 5, or 6, and R^{a}, Y¹, Y², Z¹, and Z² are as described in classes and subclasses herein, both singly and in combination.

In some embodiments, a compound of formula Id is a compound of formula Id-i: 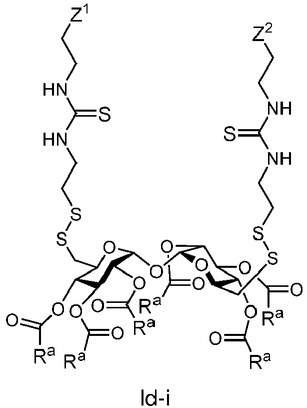 or a pharmaceutically acceptable salt thereof, wherein R^{a}, Z¹, and Z² are as described in classes and subclasses herein, both singly and in combination.

In some embodiments, the present disclosure provides a compound selected from Table 1:

**Table 1**

| **Name** | **Structure** |
|---|---|
| ssJRL9 | 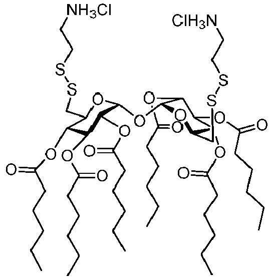 |
| ssJRL13 | 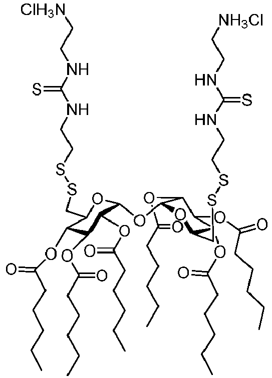 |
| ssJLF102 | 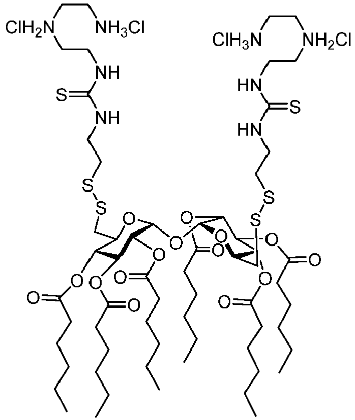 |
| ssJLF97 | 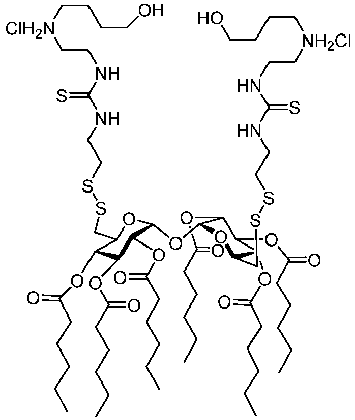 |
| ssJLF94 | 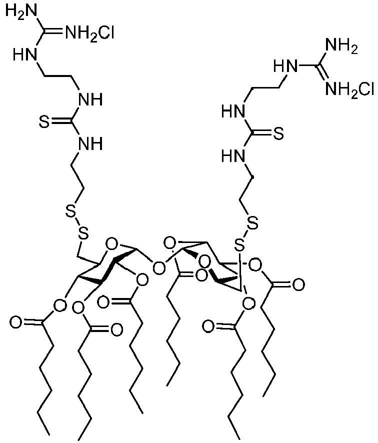 |
| ssJLF93 | 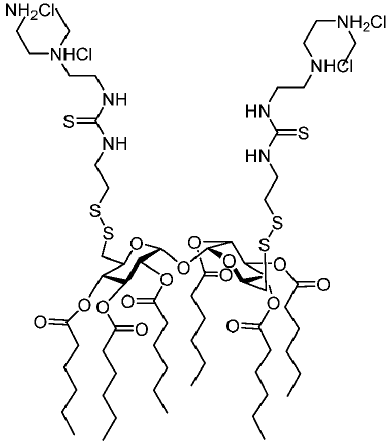 |
| ssJLF62 | 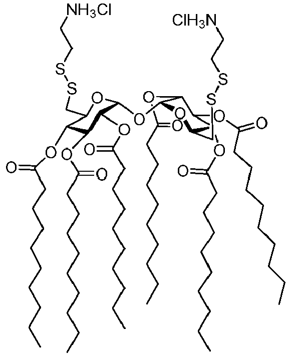 |
| PRX022 | 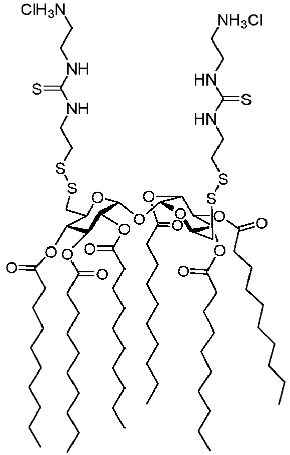 |
| ssJLF99 | 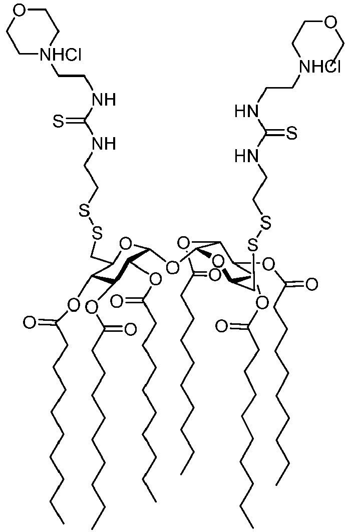 |

or a pharmaceutically acceptable salt thereof.

In some embodiments, provided compounds are provided and/or utilized in a salt form (e.g., a pharmaceutically acceptable salt form). Reference to a compound provided herein is understood to include reference to salts thereof, unless otherwise indicated. Moreover, reference to particular salts herein, e.g., in Table 1, is also intended to include reference to a free base form of said compound, to the extent such a compound is chemically feasible.

It is understood that while the exemplary compounds in Table 1 are represented as particular salts (e.g, chloride salts, iodide salts, and the like) that comprise a counterion (e.g., Cl⁻, I⁻, and the like). A person of skill in the art will appreciate that any suitable counterion can be used in conjunction with the cationic nitrogen groups in Table 1. Accordingly, the specific counterions provided above are provided by way of example and are not intended to be limiting.

### Complexes and Compositions

The compositions described herein exhibit unexpectedly improved characteristics relative to previously described complexes. Moreover, the present disclosure encompasses an insight that cationic and/or ionizable oligosaccharides are surprisingly useful for the delivery and targeted expression of particular therapeutic agents, e.g., nucleic acids, RNA.

Previous work related to cationic oligosaccharides focused on their use with DNA. In particular, Carbajo-Gordillo, *et al.,* reported use of certain oligosaccharides for DNA nanocomplexing and delivery. See Carbajo-Gordillo, et al., Chem. Comm., 55:8227-8230 (2019). Notably, however, the oligosaccharide-DNA complexes reported by Carbajo-Gordillo, *et al.,* exhibit surprisingly distinct properties from those reported in the present application, in particular when complexed with RNA. For example, the compositions reported herein, in some embodiments, exhibit different behavior in vivo, such as targeting (i.e., causing increased expression of RNA relative to other parts of the body) different systems in the body, and can be prepared with significantly reduced ratios of oligosaccharide to nucleic acid. For at least these reasons, the presently reported complexes exhibit unexpected benefits over those complexes previously reported.

Further, the presently reported compositions exhibit improved ratios of cationic moieties, or groups that are ionizable to cationic groups (on an oligosaccharide) to anionic moieties (on a nucleic acid). For example, as described herein, a complex comprises a ratio of cationic groups to anionic groups, which is referred to as an "N/P ratio." As described herein, an N/P ratio refers to a molar ratio of cationic groups (or ionizable groups that can become cationic, referred to as "N" of N/P) in a composition comprising cationic oligosaccharide compounds relative to anionic groups in a composition comprising mRNA (referred to as "P" of N/P). The presently reported compositions exhibit favorable ratios, thereby providing concentrated complexes for RNA delivery, relative to previously reported complexes with DNA. For example, the complexes of Carbajo-Gordillo requires an N/P of about 20:1 for effective targeting and delivery of pDNA. The present complexes, in contrast, comprise an N/P ratio of less than 20:1 for comparable targeting and delivery of RNA. An N/P ratio can also be expressed as a single digit, e.g., 5, where it is understood to refer to the indicated number as a ratio of X:1. For example, an N/P of 5, is intended to refer to an N/P of 5:1. Similarly, an N/P of 10, is intended to refer to an N/P of 10:1, etc.

For example, in some embodiments, an N/P ratio of a complex reported herein is less than 20:1. In some embodiments, an N/P ratio of a complex reported herein is less than or equal to 15:1. In some embodiments, an N/P ratio of a complex reported herein is less than or equal to 12:1. In some embodiments, an N/P ratio of a complex reported herein is less than or equal to 10:1. In some embodiments, an N/P ratio of a complex reported herein is less than or equal to 5:1. In some embodiments, an N/P ratio of a complex reported herein is less than or equal to 2:1. In some embodiments, an N/P ratio of a complex reported herein is less than or equal to 1:1. In some embodiments, an N/P ratio of a complex reported herein is from about 1:1 to about 15:1. In some embodiments, an N/P ratio of a complex reported herein is from about 1:1 to about 12:1. In some embodiments, an N/P ratio of a complex reported herein is from about 1:1 to about 10:1. In some embodiments, an N/P ratio of a complex reported herein is about 1:1, about 2:1, about 3:1, about 4:1, about 5:1, about 6:1, about 7:1, about 8:1, about 9:1, about 10:1, about 11:1, about 12:1, about 13:1, about 14:1, about 15:1, about 16:1, about 17:1, about 18:1, or about 19:1.

In some embodiments, a complex described herein has a diameter of about 30 nm to about 300 nm. In some embodiments a complex described herein has a diameter of about 50 nm to about 200 nm. In some embodiments, a complex described herein has a diameter that is about 50, 60, 70, 80, 90, 100, 110, 120, 130, 140, 150, 160, 170, 180, 190, or 200 nm. In some embodiments, a complex described herein has a diameter of about 30 nm to about 150 nm. In some embodiments, a complex described herein has a diameter of about 30 nm to about 100 nm. In some embodiments, a complex described herein has a diameter of less than 100 nm. In some embodiments, a complex described herein has a diameter of about 30, 35, 40, 45, 50, 55, 60, 65, 70, 75, 80, 85, 90, 95, or 100 nm.

In some embodiments, a composition or complex described herein further comprises a pharmaceutically acceptable surfactant. In some embodiments, a pharmaceutically acceptable surfactant is selected from a polysorbate (e.g., polysorbate 20 (Tween20), polysorbate 40 (Tween40), polysorbate 60 (Tween60), and polysorbate 80 (Tween80)), poloxamers, and an amphiphilic group comprising a moiety selected from polyalkylene glycols (e.g., polyethylene glycol), poly(2-oxazoline), poly(2-methyl-2-oxazoline), polysarcosine, polyvinylpyrrolidone, and poly[N-(2-hydroxypropyl)methacrylamide, wherein the moiety is bound to one or more C₁₂-C₂₀ aliphatic groups.

In some embodiments, a molar ratio of oligosaccharide to surfactant is about 1:0.0075 to about 1:3. In some embodiments, a molar ratio of oligosaccharide to surfactant is about 1:0.0075 to about 1:1.5. In some embodiments, a molar ratio of oligosaccharide to surfactant is about 1:1. In some embodiments, a molar ratio of oligosaccharide to surfactant is In some embodiments, a molar ratio of oligosaccharide to surfactant is about 1:0.0075, about 1:0.01, about 1:0.1, about 1:0.5, about 1:1, about 1:1.5, about 1:2, about 1:2.5. or about 1:3.

In some embodiments, a surfactant is a polysorbate. In some embodiments, a molar ratio of oligosaccharide to polysorbate is 1:0.0075 to 1:3. In some embodiments, a surfactant is a polysorbate. In some embodiments, a molar ratio of oligosaccharide to polysorbate is 1:0.0075 to 1:1.5. In some embodiments, a surfactant is a polysorbate. In some embodiments, a molar ratio of oligosaccharide to polysorbate is 1:0.0075 to 1:1. In some embodiments, a ratio of oligosaccharide to polysorbate is about 1:0.0075, about 1:0.01, about 1:0.1, about 1:0.5, about 1:1, about 1:1.5, about 1:2, about 1:2.5. or about 1:3..

In some embodiments, a complex comprising an oligosaccharide and a pharmaceutically acceptable surfactant has a diameter of about 30 nm to about 150 nm. In some embodiments, a complex described herein has a diameter of about 30 nm to about 100 nm. In some embodiments, a complex comprising an oligosaccharide and a pharmaceutically acceptable surfactant has a diameter of less than 100 nm. In some embodiments, a complex comprising an oligosaccharide and a pharmaceutically acceptable surfactant has a diameter of about 30, 35, 40, 45, 50, 55, 60, 65, 70, 75, 80, 85, 90, 95, or 100 nm.

### RNA

In some embodiments, a complex described herein comprises one or more oligosaccharide compositions and a nucleic acid. In some embodiments, a nucleic acid is RNA.

In some embodiments, an RNA amenable to technologies described herein is a single-stranded RNA. In some embodiments, an RNA as disclosed herein is a linear RNA. In some embodiments, a single-stranded RNA is a non-coding RNA in that its nucleotide sequence does not include an open reading frame (or complement thereof). In some embodiments, a single-stranded RNA has a nucleotide sequence that encodes (or is the complement of a sequence that encodes) a polypeptide or a plurality of polypeptides (e.g., epitopes) of the present disclosure.

In some embodiments, an RNA is or comprises an siRNA, an miRNA, or other non-coding RNA.

In many embodiments, a relevant RNA includes at least one open reading frame (ORF) (*e.g.,* is an mRNA); in some embodiments, a relevant RNA includes a single ORF; in some embodiments, a relevant RNA includes more than one ORF.

In some embodiments, an RNA comprises an ORF, e.g., encoding a polypeptide of interest or encoding a plurality of polypeptides of interest. In some embodiments, an RNA produced in accordance with technologies provided herein comprises a plurality of ORFs (e.g., encoding a plurality of polypeptides). In some embodiments, an RNA produced in accordance with technologies herein comprises a single ORF that encodes a plurality of polypeptides. In some such embodiments, polypeptides are or comprise antigens or epitopes thereof (e.g., relevant antigens).

In some embodiments, an ORF for use in accordance with the present disclosure encodes a polypeptide that includes a signal sequence, e.g., that is functional in mammalian cells, such as an intrinsic signal sequence or a heterologous signal sequence. In some embodiments, a signal sequence directs secretion of an encoded polypeptide, in some embodiments, a signal sequence directs transport of an encoded polypeptide into a defined cellular compartment, preferably the cell surface, the endoplasmic reticulum (ER) or the endosomal-lysosomal compartment.

In some embodiments, an ORF encodes a polypeptide that includes a multimerization element (*e*.*g*., an instrinsic or heterologous multimerization element). In some embodiments, an ORF that encodes a surface polypeptide (e.g., that includes a signal sequence directing surface localization) includes a multimerization element.

In some embodiments, an ORF encodes a polypeptide that includes a transmembrane element or domain.

In some embodiments, an ORF is codon-optimized for expression in a cells of a particular host, *e.g.,* a mammalian host, *e.g.,* a human.

In some embodiments, an RNA includes unmodified uridine residues; an RNA that includes only unmodified uridine residues may be referred to as a "uRNA". In some embodiments, an RNA includes one or more modified uridine residues; in some embodiments, such an RNA (e.g., an RNA including entirely modified uridine residues) is referred to as a "modRNA". In some embodiments, an RNA may be a self-amplifying RNA (saRNA). In some embodiments, an RNA may be a trans-amplifying RNA (taRNA) (*see,* for example, WO2017/162461).

In some embodiments, a relevant RNA includes a polypeptide-encoding portion or a plurality of polypeptide-encoding portions. In some particular embodiments, such a portion or portions may encode a polypeptide or polypeptides that is or comprises a biologically active polypeptide or portion thereof (e.g., an enzyme or cytokine or therapeutic protein such as a replacement protein or antibody or portion thereof). In some particular embodiments, such a portion or portions may encode a polypeptide or polypeptides that is or comprises an antigen (or an epitope thereof), a cytokine, an enzyme, etc. In some embodiments, an encoded polypeptide or polypeptides may be or include one or more neoantigens or neoepitopes associated with a tumor. In some embodiments, an encoded polypeptide or polypeptides may be or include one or more antigens (or epitopes thereof) of an infectious agent (e.g., a bacterium, fungus, virus, etc.). In certain embodiments, an encoded polypeptide may be a variant of a wild type polypeptide.

In some embodiments, a single-stranded RNA (e.g., mRNA) may comprise a secretion signal-encoding region (e.g., a secretion signal-encoding region that allows an encoded target entity or entities to be secreted upon translation by cells). In some embodiments, such a secretion signal-encoding region may be or comprise a non-human secretion signal. In some embodiments, such a secretion signal-encoding region may be or comprise a human secretion signal.

In some embodiments, a single-stranded RNA (e.g., mRNA) may comprise at least one non-coding element (e.g., to enhance RNA stability and/or translation efficiency). Examples of non-coding elements include but are not limited to a 3' untranslated region (UTR), a 5' UTR, a cap structure (e.g., in some embodiments, an enzymatically-added cap; in some embodiments, a co-transcriptional cap), a poly adenine (polyA) tail (e.g., that, in some embodiments, may be or comprise 100 A residues or more, and/or in some embodiments may include one or more "interrupting" [*i.e.,* non-A] sequence elements), and any combinations thereof. Exemplary embodiments of such non-coding elements may be found, for example, in WO2011015347, WO2017053297, US 10519189, US 10494399, WO2007024708, WO2007036366, WO2017060314, WO2016005324, WO2005038030, WO2017036889, WO2017162266, and WO2017162461, each of which is incorporated herein by referenced in its entirety.

### Formats

At least four formats useful for RNA pharmaceutical compositions (e.g., immunogenic compositions or vaccines) have been developed, namely non-modified uridine containing mRNA (uRNA), nucleosidemodified mRNA (modRNA), self-amplifying mRNA (saRNA), and trans-amplifying RNAs (taRNAs).

Features of a non-modified uridine platform may include, for example, one or more of intrinsic adjuvant effect, good tolerability and safety, and strong antibody and T cell responses.

Features of modified uridine (e.g., pseudouridine) platform may include reduced adjuvant effect, blunted immune innate immune sensor activating capacity and thus augmented antigen expression, good tolerability and safety, and strong antibody and CD4-T cell responses. As noted herein, the present disclosure provides an insight that such strong antibody and CD4 T cell responses may be particularly useful for vaccination. Features of self-amplifying platform may include, for example, long duration of polypeptide (e.g., protein) expression, good tolerability and safety, higher likelihood for efficacy with very low vaccine dose.

In some embodiments, a self-amplifying platform (e.g., RNA) comprises two nucleic acid molecules, wherein one nucleic acid molecule encodes a replicase (e.g., a viral replicase) and the other nucleic acid molecule is capable of being replicated (e.g., a replicon) by said replicase *in trans* (trans-replication system). In some embodiments, a self-amplifying platform (e.g., RNA) comprises a plurality of nucleic acid molecules, wherein said nucleic acids encode a plurality of replicases and/or replicons.

In some embodiments, a *trans-replication* system comprises the presence of both nucleic acid molecules in a single host cell.

In some such embodiments, a nucleic acid encoding a replicase (e.g., a viral replicase) is not capable of self-replication in a target cell and/or target organism. In some such embodiments, a nucleic acid encoding a replicase (e.g., a viral replicase) lacks at least one conserved sequence element important for (-) strand synthesis based on a (+) strand template and/or for (+) strand synthesis based on a (-) strand template.

In some embodiments, a self-amplifying RNA comprises a 5'-cap; in some *trans-*replication systems, at least an RNA encoding a replicase is capped. Without wishing to be bound by any one theory, it has been found that a 5'-cap can be important for high level expression of a gene of interest *in trans.*

In some embodiments, a self-amplifying platform does not require propagation of virus particles (e.g., is not associated with undesired virus-particle formation). In some embodiments, a self-amplifying platform is not capable of forming virus particles.

In some embodiments, an RNA may comprise an Internal Ribosomal Entry Site (IRES) element. In some embodiments, an RNA does not comprise an IRES site; in particular, in some embodiments, an saRNA does not comprise an IRES site. In some such embodiments, translation of a gene of interest and/or replicase is not driven by an IRES element. In some embodiments, an IRES element is substituted by a 5'-cap. In some such embodiments, substitution by a 5'-cap does not affect the sequence of a polypeptide encoded by an RNA.

In some embodiments, a complex described herein comprises modRNA, saRNA, taRNA, or uRNA. In some embodiments, a complex comprises modRNA. In some embodiments, a complex comprises saRNA. In some embodiments, a complex comprises taRNA. In some embodiments, a complex comprises uRNA

### Methods of Use

Complexes described herein are useful in the treatment and prophylaxis in a subject of diseases, disorders, and conditions described herein. In some embodiments, the present disclosure provides a method of treating a disease, disorder or condition comprising administering to a patient a complex described herein. In some embodiments, the present disclosure provides use of a complex described herein for the treatment of a disease, disorder, or condition. In some embodiments, a disease, disorder, or condition is an infectious disease, cancer, an autoimmune disease, or a rare disease.

In some embodiments, an infectious disease is caused by or associated with a viral pathogen. In some embodiments, a viral pathogen is of a family selected from poxviridae, rhabdoviridae, filoviridae, paramyxoviridae, hepadnaviridae, coronaviridae, caliciviridae, picornaviridae, reoviridae, retroviridae, and orthomyxoviridae. In some embodiments, an infectious disease is caused by or associated with a virus selected from SARS-CoV-2, influenza, Crimean-Congo Hemorhhagic Fever (CCHF), Ebola virus, Lassa virus, Marburg virus, HIV, Nipah virus, and MERS-CoV.

In some embodiments, an infectious disease is caused by or associated with a bacterial pathogen. In some embodiments, a bacterial pathogen is of a species selected from Actinomyces israelii, bacillus antracis, Bacteroides fragilis, Bordetella pertussis, Borrelia burgdorferi, Borrelia garinii, Borrelia afzelii, Borrelia recurrentis, Brucella abortus, Brucella canis, Brucella melitensis, Brucella suis, Campolobacter jejuni, Chlamydia pneumoniae, Chlamydia trachomatis, Chlamydophila psittaci, Clostridium botulinum, Clostridium difficile, Clostridium perfringens, Clostridium tetani, Corynebacterium idphteriae, Ehrlichia canis, Ehrlichia chaffeensis, Enterococcus faecalis, Enterococcus faecium, Escherichia coli, Francisella tularensis, Haemophilus influenzae, Helicobacter pylori, Klebsiella pneumoniae, Legionella pneumophila, Leptospira, Listeria monocytogenes, Mycobacterium leprae, Mycobacterium tuberculosis, Mycoplasma pneumoniae, Neisseria gonorrhoeae, Neisseria meningitidis, Pseudomonas aeruginosa, Nocardia asteroids, Rickettsia ricektssii, Salmonella typhi, Salmonella typhimurium, Shigella sonnei, Shigella dysenteriae, Staphylococcus aureus, Staphylococcus epidermidis, Staphylococcus saprophyticus, Streptococcus agalactiae, Streptococcus pneumoniae, Streptococcus pyogenes, Streptococcus viridans, Treponema pallidum, Vibrio cholerae, and Yersinia pestis.

In some embodiments, an infectious disease is caused by or associated with a parasite. In some embodiments, a parasite is of a family selected from Plasmodium, Leishmania, Cryptosporidium, Entamoeba, Trypanosomas, Schistosomes, Ascaris, Echinococcus and Taeniidae.

In some embodiments, a disease, disorder, or condition is a cancer. In some embodiments, a cancer is selected from bladder cancer, breast cancer, colorectal cancer, kidney cancer, lung cancer, lymphoma, melanoma, oral/oropharyngeal cancer, pancreatic cancer, prostate cancer, thyroid cancer, and uterine cancer.

In some embodiments, a disease, disorder, or condition is a genetic disorder. In some embodiments, a genetic disorder is associated with a gain-of-function mutation or a loss-of-function mutation.

In some embodiments, a disease, disorder, or condition is an autoimmune disease. In some embodiments, an autoimmune disease is selected from addison disease, celiac disease, rheumatoid arthritis, lupus, inflammatory bowel disease, dermatomyositis, multiple sclerosis, diabetes, guillain-barre syndrome, chronic inflammatory demyelinating polyneuropathy, psoriasis, pernicious anemia, graves' disease, hashimoto's thyroiditis, myasthenia gravis, and vasculitis sjörgen syndrome.

In some embodiments, a disease, disorder, or condition is a rare disease. As described herein, a rare disease refers to a life-threatening or chronically debilitating diseases which are of such low prevalence (e.g., fewer than 1/2000 people) that special combined efforts are needed to address them.

In some embodiments, the present disclosure provides complexes that can selectively target particular systems within a body. For example, in some embodiments, complexes described herein can selectively target the lungs, liver, spleen, heart, brain, lymph nodes, bladder, kidneys, and pancreas. As described herein, a complex "selectively targets" an organ when a single target expresses mRNA in an amount that is 65% or greater than expression in other organs post administration (e.g., 65% or more of mRNA throughout the body is expressed from a single organ, with the remaining 35% distributed between one or more different organs). In some embodiments, a complex described herein selectively targets the lungs. In some embodiments, a complex described herein selectively targets the liver. In some embodiments, a complex described herein selectively targets the spleen. In some embodiments, a complex described herein selectively targets the heart.

### Methods of Delivery

The present disclosure provides, among other things, a complex (e.g., a pharmaceutical composition or a pharmaceutical formulation, as referred to herein) to be administered to a subject. For example, in some embodiments, a complex is administered as a monotherapy. In some embodiments, a complex is administered as part of a combination therapy. In some embodiments, a concentration of total RNA (e.g., a total concentration of all of the one or more RNA molecules) in a pharmaceutical composition described herein is of about 0.01 mg/mL to about 0.5 mg/mL, or about 0.05 mg/mL to about 0.1 mg/mL.

Pharmaceutical compositions may additionally comprise a pharmaceutically acceptable excipient, which, as used herein, includes any and all solvents, dispersion media, diluents, or other liquid vehicles, dispersion or suspension aids, surface active agents, isotonic agents, thickening or emulsifying agents, preservatives, solid binders, lubricants and the like, as suited to the particular dosage form desired. Remington's The Science and Practice of Pharmacy, 21st Edition, A. R. Gennaro (Lippincott, Williams & Wilkins, Baltimore, MD, 2006; incorporated herein by reference in its entirety) discloses various excipients used in formulating pharmaceutical compositions and known techniques for the preparation thereof. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this disclosure.

In some embodiments, an excipient is approved for use in humans and for veterinary use. In some embodiments, an excipient is approved by the United States Food and Drug Administration. In some embodiments, an excipient is pharmaceutical grade. In some embodiments, an excipient meets the standards of the United States Pharmacopoeia (USP), the European Pharmacopoeia (EP), the British Pharmacopoeia, and/or the International Pharmacopoeia.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Such excipients may optionally be included in pharmaceutical formulations. Excipients such as cocoa butter and suppository waxes, coloring agents, coating agents, sweetening, flavoring, and/or perfuming agents can be present in the composition, according to the judgment of the formulator.

General considerations in the formulation and/or manufacture of pharmaceutical agents may be found, for example, in Remington: The Science and Practice of Pharmacy 21st ed., Lippincott Williams & Wilkins, 2005 (incorporated herein by reference in its entirety). In some embodiments, pharmaceutical compositions provided herein may be formulated with one or more pharmaceutically acceptable carriers or diluents as well as any other known adjuvants and excipients in accordance with conventional techniques such as those disclosed in Remington: The Science and Practice of Pharmacy 21st ed., Lippincott Williams & Wilkins, 2005 (incorporated herein by reference in its entirety). Pharmaceutical complexes and compositions described herein can be administered by appropriate methods known in the art. As will be appreciated by a skilled artisan, the route and/or mode of administration may depend on a number of factors, including, e.g., but not limited to stability and/or pharmacokinetics and/or pharmacodynamics of pharmaceutical compositions described herein.

In some embodiments, pharmaceutical compositions described herein are formulated for parenteral administration, which includes modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. In some embodiments, pharmaceutical compositions described herein are formulated for intravenous administration. In some embodiments, pharmaceutically acceptable carriers that may be useful for intravenous administration include sterile aqueous solutions or dispersions and sterile powders for preparation of sterile injectable solutions or dispersions.

In some particular embodiments, pharmaceutical compositions described herein are formulated for subcutaneous (s.c) administration. In some particular embodiments, pharmaceutical compositions described herein are formulated for intramuscular (i.m) administration.

Therapeutic compositions typically must be sterile and stable under the conditions of manufacture and storage. The composition can be formulated as a solution, dispersion, powder (e.g., lyophilized powder), microemulsion, lipid nanoparticles, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. In some embodiments, prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration.

In some embodiments, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Examples of suitable aqueous and nonaqueous carriers which may be employed in the pharmaceutical compositions described herein include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the presence of microorganisms may be ensured both by sterilization procedures, and by the inclusion of various antibacterial and antifungal agents, for example, paraben, chlorobutanol, phenol sorbic acid, and the like. It may also be desirable to include isotonic agents, such as sugars, sodium chloride, and the like into pharmaceutical compositions described herein. In addition, prolonged absorption of the injectable pharmaceutical form may be brought about by the inclusion of agents which delay absorption such as aluminum monostearate and gelatin.

Formulations of pharmaceutical compositions described herein may be prepared by any method known or hereafter developed in the art of pharmacology. In general, such preparatory methods include the step of bringing active ingredient(s) into association with a diluent or another excipient and/or one or more other accessory ingredients, and then, if necessary and/or desirable, shaping and/or packaging the product into a desired single- or multi-dose unit.

A pharmaceutical composition in accordance with the present disclosure may be prepared, packaged, and/or sold in bulk, as a single unit dose, and/or as a plurality of single unit doses. As used herein, a "unit dose" is discrete amount of the pharmaceutical composition comprising a predetermined amount of at least one RNA product produced using a system and/or method described herein.

In some embodiments, an active agent that may be included in a pharmaceutical composition described herein is or comprises a therapeutic agent administered in a combination therapy described herein. Pharmaceutical compositions described herein can be administered in combination therapy, i.e., combined with other agents. In some embodiments, such therapeutic agents may include agents leading to depletion or functional inactivation of regulatory T cells. For example, in some embodiments, a combination therapy can include a provided pharmaceutical composition with at least one immune checkpoint inhibitor.

In some embodiments, pharmaceutical composition described herein may be administered in conjunction with radiotherapy and/or autologous peripheral stem cell or bone marrow transplantation.

In some embodiments, a pharmaceutical composition described herein can be frozen to allow long-term storage.

Although the descriptions of pharmaceutical compositions provided herein are principally directed to pharmaceutical compositions that are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with merely ordinary, if any, experimentation.

### Methods of Preparing Compositions Described Herein

As described herein, the present disclosure provides, among other things, a method of preparing an oligosaccharide of formula la: 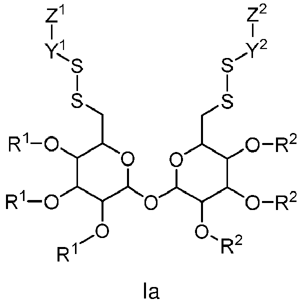 or a pharmaceutically acceptable salt thereof, comprising contacting a compound of formula II 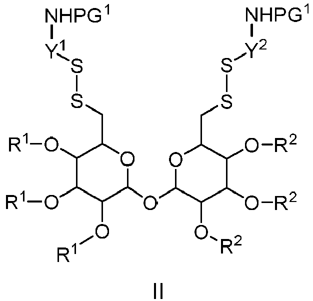 with a first acid to provide the compound of formula la, wherein
Y¹ and Y² are each -CH₂-CH₂-NHC(S)NH-CH₂-CH₂-;
Z¹ and Z² are each -NH₃Cl;
R¹ and R² are each -C(O)-R^{a};
R^{a} is optionally substituted C₁-C₂₀ aliphatic; and
PG¹ is a nitrogen protecting group.

In some embodiments, a method of preparing a compound of formula II comprises contacting a compound of formula III 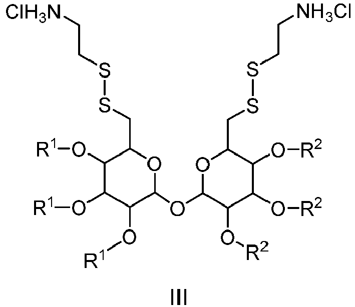 with a compound of formula IV 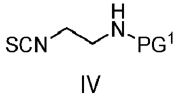 in the presence of a base to provide the compound of formula II.

In some embodiments, a method of preparing a compound of formula II comprises contacting a compound of formula IIIa 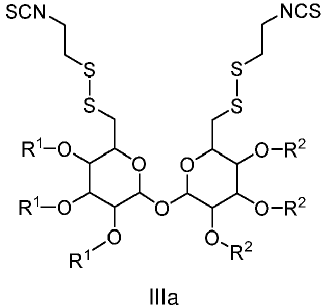 with a compound of formula IVa 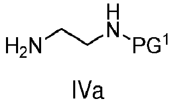 in the presence of a base to provide the compound of formula II.

In some embodiments, a method of preparing a compound of formula IIIa comprises contacting a compound of formula III with CSCl₂ to provide a compound of formula IIIa In some embodiments, a method of preparing a compound of formula III comprises contacting a compound of formula V 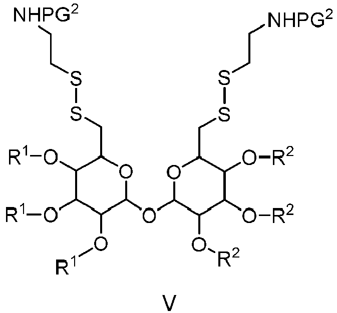 with a second acid to provide the compound of formula III, wherein PG² is a suitable nitrogen protecting group.

In some embodiments, a method of preparing a compound of formula V comprises contacting a compound of formula VI 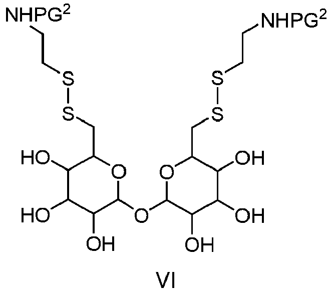 with a compound of formula Via: 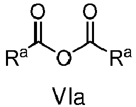 to provide the compound of formula V.

In some embodiments, a method of preparing a compound of formula VI comprises contacting a compound of formula VII 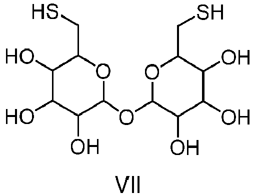 with a compound of formula VIIa 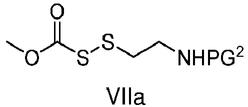 to provide the compound of formula VI.

In some embodiments, a method of preparing a compound of formula VIIa comprises contacting a compound of formula VIII 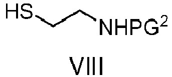 with a compound of formula Villa 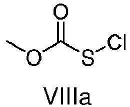 in the presence of dioxane to provide the compound of formula VIIa.

In some embodiments, a method of preparing a compound of formula VII comprises contacting a compound of formula IX 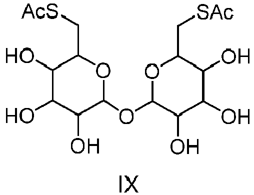 with sodium methoxide to provide a compound of formula VII.

In some embodiments, a method of preparing a compound of formula IX comprises contacting a compound of formula X 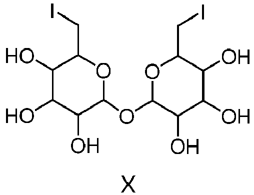 with thioacetic acid in the presence of a third base to provide the compound of formula IX.

In some embodiments, PG¹ and PG² are each independently selected from Boc (tert-butoxycarbonyl), Fmoc (fluorenylmethyloxycarbonyl), Cbz (benzyl chloroformate), Troc (2,2,2-trichloroethoxycarbonyl), Trityl (triphenylmethyl), Phthalimide, Tetrachlorophthalimide, and Trifluoroaceamide.

### Exemplary Embodiments

Embodiment 1. A composition comprising an oligosaccharide of formula I: 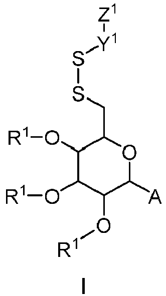 or a pharmaceutically acceptable salt thereof, wherein:
   A is A¹, A², or A³: 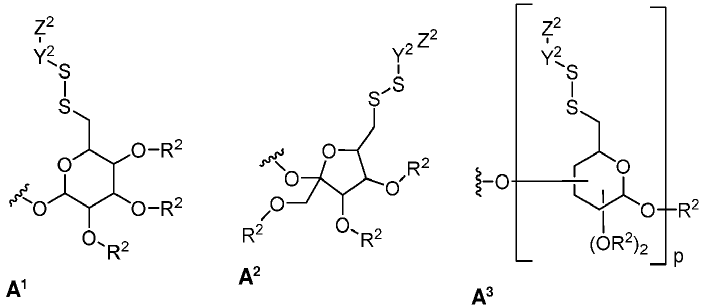
   R¹ and R² are each independently selected from H, R^{a}, and -C(O)-R^{a}, and wherein at least one instance of R¹ or R² is not H;
   each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b};
   each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, and -NHC(O)NHR^{c};
   each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S;
   Y¹ and Y² are each independently an optionally substituted C₁₋₃₀ aliphatic group wherein one or more carbons are optionally and independently replaced by -Cy-, -NH-, -NHC(O)-, -C(O)NH-, -NHC(O)O-, -OC(O)NH-, -NHC(O)NH-, -NHC(S)NH-, -C(S)NH-C(O)NHSO₂-, -SO₂NHC(O)-,-OC(O)O-, -O-, -C(O)-, -OC(O)-, -C(O)O-, -SO-, or -SO₂-; each Cy is independently an optionally substituted C₃-C₁₄ cycloaliphatic, 5- to 14-membered heterocyclyl ring having 1-3 heteroatoms selected from N, O, S, 5- to 14-membered heteroaryl ring having 1-3 heteroatoms selected from N, O, S;
   Z¹ and Z² are each independently a cationic or ionizable group selected from optionally substituted 5- to 14-membered heterocyclyl ring having 1-3 heteroatoms selected from N, O, and S, 5- to 14-membered heteroaryl ring having 1-3 heteroatoms selected from N, O, and S, -N+(M)₃, 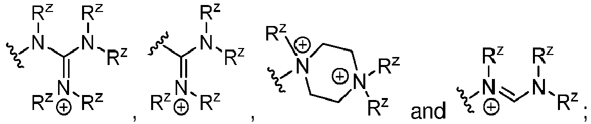
   each M is independently -C₀-C₆ aliphatic-R^{z} or -C₀-C₆ aliphatic-N⁺(R^{z})₃;
   each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
   two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S; and
   p is an integer selected from 1, 2, 3, 4, and 5.
Embodiment 2. The composition of Embodiment 1, wherein the oligosaccharide is of formula la: 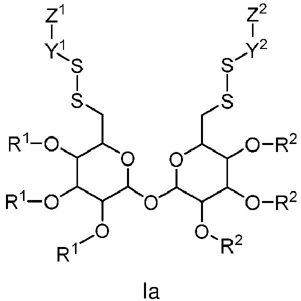 or a pharmaceutically acceptable salt thereof.
Embodiment 3. The composition of Embodiment 1, wherein the oligosaccharide is of formula Ib: 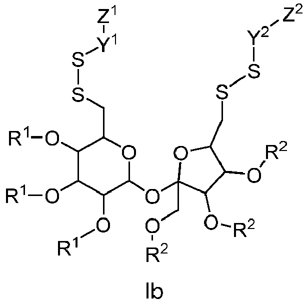 or a pharmaceutically acceptable salt thereof.
Embodiment 4. The composition of Embodiment 1, wherein the oligosaccharide is of formula Ic: 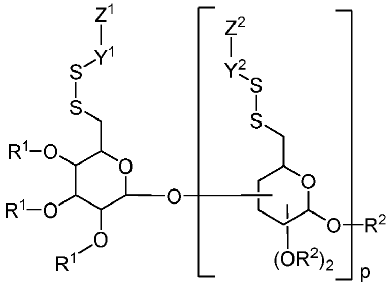 or a pharmaceutically acceptable salt thereof.
Embodiment 5. The composition of any one of Embodiments 1-4, wherein R¹ and R² are each independently selected from R^{a} and -C(O)-R^{a}, and R^{a} is C₁-C₂₀ aliphatic.
Embodiment 6. The composition of any one of Embodiments 1-5, wherein R¹ and R² are each -C(O)-R^{a}, and R^{a} is C₁-C₁₄ aliphatic.
Embodiment 7. The composition of any one of Embodiments 1-6, wherein each R^{a} is C₅-C₁₀ linear alkyl.
Embodiment 8. The composition of Embodiment 1, wherein R¹ and R² are each - C(O)-R^{a}, and R^{a} is 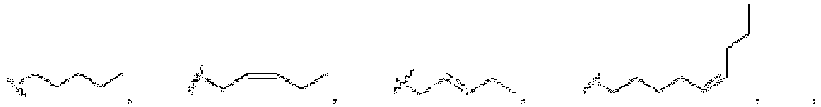 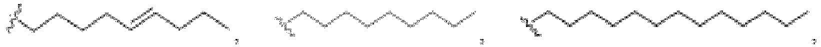 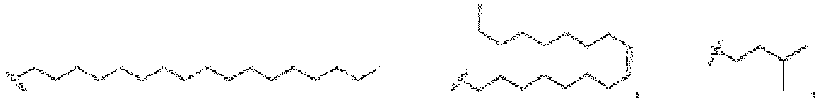 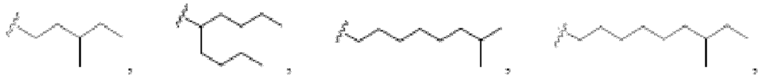 or 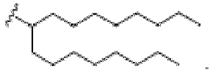
Embodiment 9. The composition of any one of Embodiments 1-8, wherein Y¹ and Y² are each selected from C₁-C₁₀ aliphatic, C₀-C₄-aliphatic-NHC(O)NH-C₀-C₄ aliphatic, C₀-C₄-aliphatic-NHC(S)NH-C₀-C₄ aliphatic, C₀-C₄-aliphatic-C(O)NH-C₀-C₄ aliphatic, Co-C₄-aliphatic-C(S)NH-C₀-C₄ aliphatic, C₀-C₄-aliphatic-NHC(O)-C₀-C₄ aliphatic, C₀-C₄-aliphatic-NHSO₂-C₀-C₄ aliphatic, and C₀-C₄-aliphatic-C(O)-C₀-C₄ aliphatic.
Embodiment 10. The composition of any one of Embodiments 1-9, wherein Y¹ and Y² are each C₁-C₄ aliphatic-NHC(S)NH-C₁-C₄ aliphatic.
Embodiment 11. The composition of Embodiment 1, wherein Y¹ and Y² are each - CH₂-CH₂-NHC(S)NH-CH₂-CH₂-.
Embodiment 12. The composition of any one of Embodiments 1-11, wherein Z¹ and Z² are each independently selected from: N⁺(M)₃, 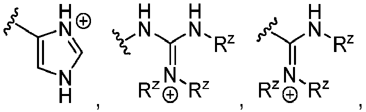 and 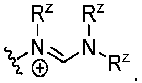
Embodiment 13. The composition of Embodiment 1, wherein Z¹ and Z² are each independently selected from: 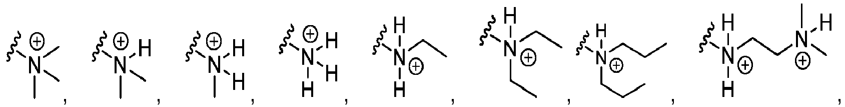 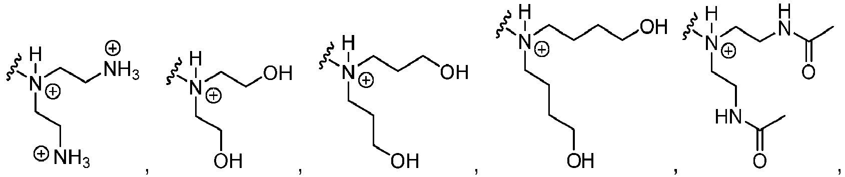 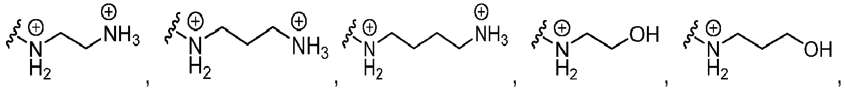 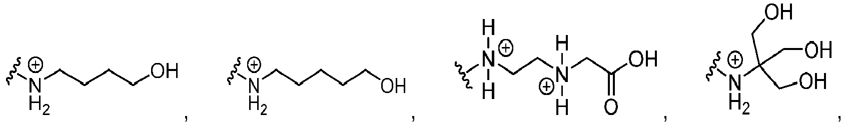 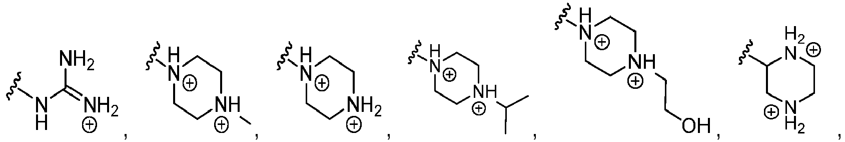 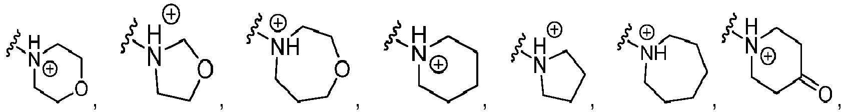 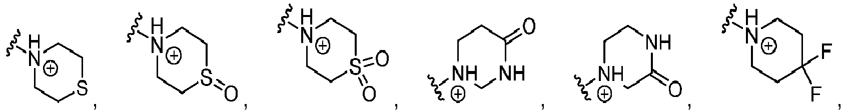 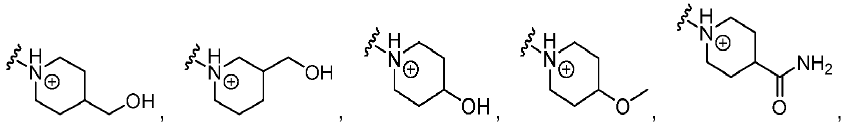 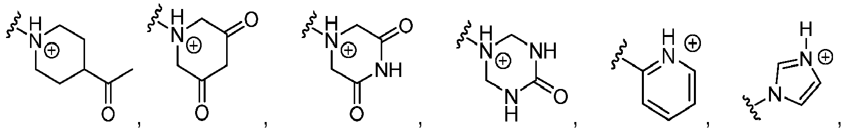 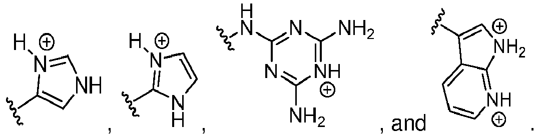
Embodiment 14. The composition of Embodiment 1, wherein the oligosaccharide is a compound of formula Id: 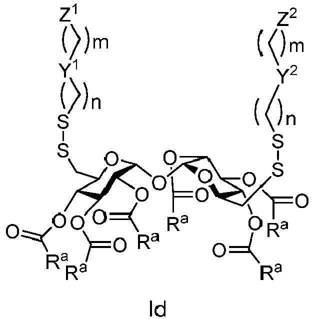 or a pharmaceutically acceptable salt thereof, wherein m and n are each independently selected from 0, 1, 2, 3, 4, 5, or 6.
Embodiment 15. The composition of Embodiment 14, wherein the oligosaccharide is a compound of formula Id-i: 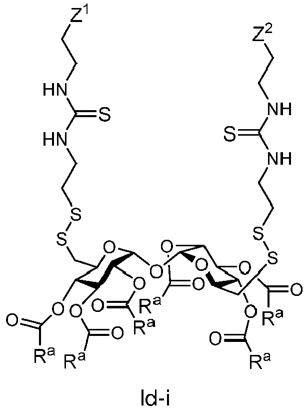 or a pharmaceutically acceptable salt thereof.
Embodiment 16. The composition of any one of Embodiments 1-15, further comprising one or more suitable counterions.
Embodiment 17. The composition of Embodiment 1, wherein the oligosaccharide is selected from Table 1, or a pharmaceutically acceptable salt thereof.
Embodiment 18. A complex comprising the composition oligosaccharide of any one of Embodiments 1-17, and a nucleic acid.
Embodiment 19. The complex of Embodiment 18, wherein the nucleic acid is RNA.
Embodiment 20. The complex of Embodiment 19, wherein the RNA is or comprises modRNA, saRNA, taRNA, or uRNA.
Embodiment 21. The complex of Embodiments 18-20, wherein a ratio of N/P is less than 20:1.
Embodiment 22. The complex of Embodiment 21, wherein the ratio of N/P is less than or equal to 12:1.
Embodiment 23. The complex of any one of Embodiments 18-22, wherein the complex has a diameter of about 30 nm to about 150 nm.
Embodiment 24. A method of delivering a composition to a target in a subject comprising administering to the subject the complex of any one of Embodiments 18-23.
Embodiment 25. The method of Embodiment 24, wherein the target is selected from the liver, the lungs, or the spleen.
Embodiment 26. A method of treating a disease, disorder, or condition in a subject comprising administering to the subject a complex of any one of Embodiments 18-23.
Embodiment 27. The method of Embodiment 26, wherein the disease, disorder, or condition is an infectious disease, cancer, a genetic disorder, an autoimmune disease, or a rare disease.
Embodiment 28. The method of any one of Embodiments 24-27, wherein the complex is administered intramuscularly.
Embodiment 29. The method of any one of Embodiments 24-27, wherein the complex is administered subcutaneously.
Embodiment 30. A method of preparing an oligosaccharide of formula la: 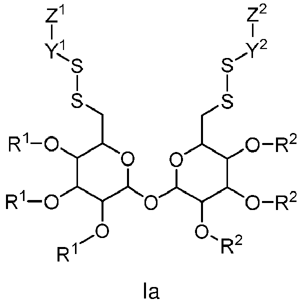 or a pharmaceutically acceptable salt thereof, comprising contacting a compound of formula II 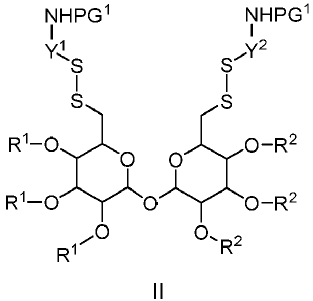 with a first acid to provide the compound of formula la, wherein
   Y¹ and Y² are each -CH₂-CH₂-NHC(S)NH-CH₂-CH₂-;
   Z¹ and Z² are each -NH₃Cl;
   R¹ and R² are each -C(O)-R^{a};
   R^{a} is optionally substituted C₁-C₂₀ aliphatic; and
   PG¹ is a nitrogen protecting group.
Embodiment 31. The method of Embodiment 30, further comprising contacting a compound of formula III 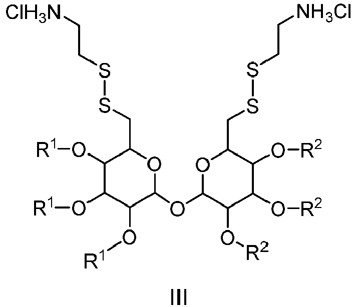 with a compound of formula IV 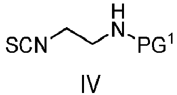 in the presence of a base to provide the compound of formula II.
Embodiment 32. The method of Embodiment 30, further comprising contacting a compound of formula IIIa 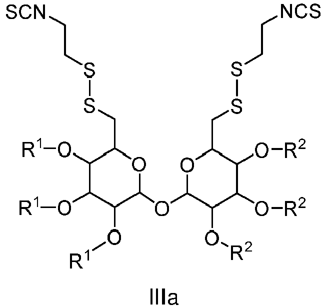 with a compound of formula IVa 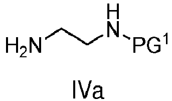 in the presence of a base to provide the compound of formula II.
Embodiment 33. The method of Embodiment 32, further comprising contacting a compound of formula III with CSCl₂ to provide a compound of formula IIIa.
Embodiment 34. The method of Embodiment 31, further comprising contacting a compound of formula V 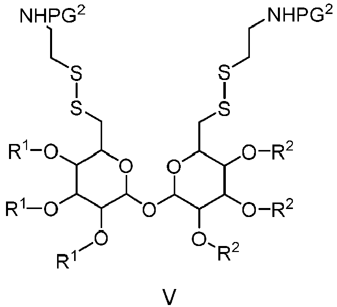 with a second acid to provide the compound of formula III, wherein PG² is a suitable nitrogen protecting group.
Embodiment 35. The method of Embodiment 34, further comprising contacting a compound of formula VI 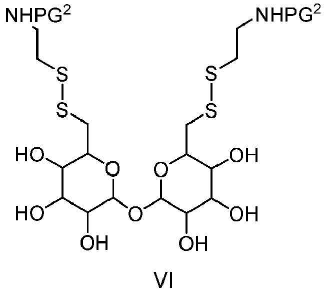 with a compound of formula VIa: 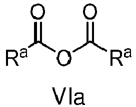 to provide the compound of formula V.
Embodiment 36. The method of Embodiment 35, further comprising contacting a compound of formula VII 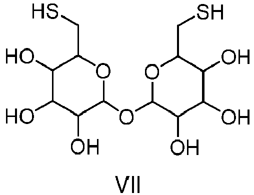 with a compound of formula VIIa 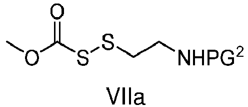 to provide the compound of formula VI.
Embodiment 37. The method of Embodiment 36, further comprising contacting a compound of formula VIII 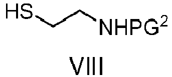 with a compound of formula VIIIa 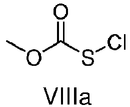 in the presence of dioxane to provide the compound of formula VIIa. Embodiment 38. The method of Embodiments 35 or 36, further comprising contacting a compound of formula IX 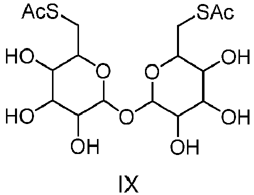 with sodium methoxide to provide a compound of formula VII.
Embodiment 39. The method of Embodiment 38, further comprising contacting a compound of formula X 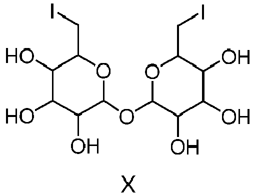 with thioacetic acid in the presence of a third base to provide the compound of formula IX.
Embodiment 40. Use of a complex of any one of Embodiments 18-23, for the treatment of a disease, disorder, or condition.
Embodiment 41. The use of Embodiment 40, wherein the disease, disorder, or condition is an infectious disease, cancer, a genetic disorder, an autoimmune disease, or a rare disease.
Embodiment 42. The complex of any one of Embodiments 18-23 for use in medicine.

The foregoing has been a description of certain non-limiting embodiments of the subject matter described within. Accordingly, it is to be understood that the embodiments described in this specification are merely illustrative of the subject matter reported within. Reference to details of the illustrated embodiments is not intended to limit the scope of the claims, which themselves recite those features regarded as essential.

It is contemplated that systems and methods of the claimed subject matter encompass variations and adaptations developed using information from the embodiments described within. Adaptation, modification, or both, of the systems and methods described within may be performed by those of ordinary skill in the relevant art. Throughout the description, where systems are described as having, including, or comprising specific components, or where methods are described as having, including, or comprising specific steps, it is contemplated that, additionally, there are systems encompassed by the present subject matter that consist essentially of, or consist of, the recited components, and that there are methods encompassed by the present subject matter that consist essentially of, or consist of, the recited processing steps.

It should be understood that the order of steps or order for performing certain action is immaterial so long as any embodiment of the subject matter described within remains operable. Moreover, two or more steps or actions may be conducted simultaneously

### Acknowledgements

The Applicant wishes to thank Consejo Superior de Investigaciones Científicas and FIUS/UNIVERSITY OF SEVILLE/ for their support and contribution to the development of this work.

### Examples

The Examples provided herein document and support certain aspects of the present disclosure but are not intended to limit the scope of any claim. The following non-limiting examples are provided to further illustrate certain teachings provided by the present disclosure. Those of skill in the art, in light of the present application, will appreciate that various changes can be made in the specific embodiments that are illustrated in the present Examples without departing from the spirit and scope of the present teachings. The following abbreviations may be used in the Examples below: aq. (aqueous); ACN (acetonitrile); CSA (camphorsulfonic acid); d (day or days); Da/kDa (Daltons/kiloDaltons); DCM (dichloromethane); ddH₂O (double distilled H₂O); DEA (diethylamine); DHP (dihydropyran); DMF (N,N-dimethylformamide); DIPEA (N,N-diisopropylethylamine); DMAP (4-dimethylaminopyridine); DOTMA (1,2-di-O-octadecenyl-3-trimethylammonium propane); DODMA (1,2-dioleyloxy-3-dimethylaminopropane), DOPE (1,2-dioleoyl-sn-glycero-3-phosphoethanolamine); DMSO (dimethyl sulfoxide); EA (ethyl acetate); ee (enantiomeric excess); equiv. (equivalent); ethanol (EtOH); h (hour or hours); Hex (hexanes); HPLC (high-performance liquid chromatography); IPA (isopropyl alcohol); i.m (intramuscular); i.v (instravenous); KHMDS (potassium bis(trimethylsilyl)amide); LAH (lithium aluminum hydride); LCMS (liquid chromatography-mass spectrometry); LDA (lithium diisopropylamide); LiHMDS (lithium bis(trimethylsilyl)amide); MeOH (methanol); min (minute or minutes); NMR (nuclear magnetic resonance); Pd/C (palladium on carbon); PEI (polyethyleneimine); PPh₃O (triphenylphosphine oxide); Pt/C (platinum on carbon); rb (round-bottomed); Rf (retention factor); rt or RT (room temperature); s.c (subcutaneous); SM (starting material); TEA (triethylamine); THF (tetrahydrofuran); THP (tetrahydropyran); TLC (thin layer chromatography); TsOH (p-toluenesulfonic acid or tosylic acid); and UV (ultraviolet).

### Example 1 - Complexation Protocol

Complexation of exemplary embodiments described herein is produced by asymmetrical volume mixing of an RNA containing solution and a cationic oligosaccharide containing solution. As used herein, reference to "paOS" refers to cationic oligosaccharides described herein. For the RNA solution, the required concentration of RNA is prepared by diluting an appropriate volume of ddH₂O, RNA Stock and buffer (6x). The oligosaccharide solution is prepared in a separate tube by diluting the oligosaccharide in DMSO to desired concentration. RNA and cationic oligosaccharide phases are mixed at a volume ratio of 9:1 (RNA:oligosaccharide). The formulated complexes are incubated 5-10 min at room temperature for stabilization. The buffer used for the formulations in all experiments is MBS (MES buffered Sucrose (10mM MES, pH6.1, 10% Sucrose)) Complexes with either ssJRL13 or JRL13 and saRNA were formulated at different N/P ratios. JRL13 is a compound described in Carbajo-Gordillo, et al., Chem. Comm., 55:8227-8230 (2019) as compound 8, and is of the following structure: 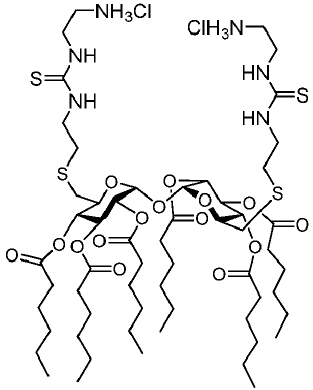 ssJRL13 is a compound of the following structure:

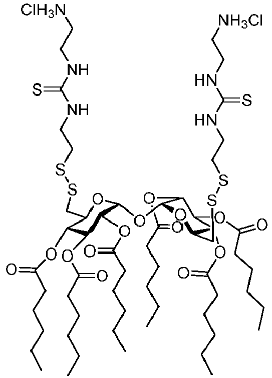

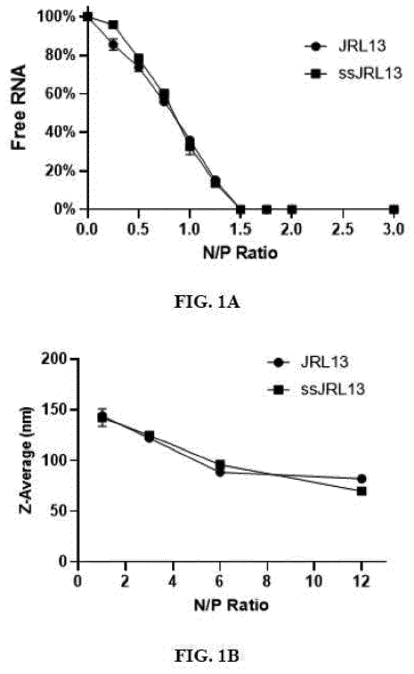

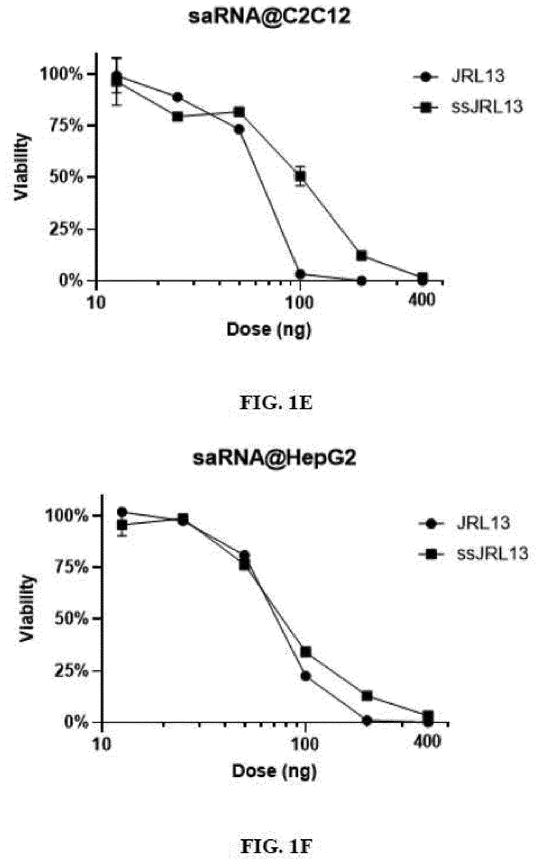

The complexes with either ssJRL13 or JRL13 and a N/P ratio of 12 were tested in C2C12 cell-line and HepG2 cell-line at a dose range between 12.5 and 400ng of formulated RNA. Formulations were complexed in MBS Buffer (final concentration 10% w/v Sucrose, 10mM MES, pH 6.1), at RNA final concentration of 0.05mg/ml and the mixing ratio of the RNA containing solution and the oligosaccharide containing solution was 9:1 (v:v) for all the cases. Formulations were characterized prior to injection to confirm hydrodynamic diameter and zeta potential. **FIG. 1A** indicates percentage of free RNA quantified by gel electrophoresis of formulated saRNA at different N/P ratios between 0-3 for either JRL13 or ssJRL13. **FIG. 1B** indicates hydrodynamic diameter for formulated saRNA at different N/P ratios with either JRL13 or ssJRL13. **FIGs. 1C-1D** indicate expression of luciferase-encoding saRNA after 24 hours of transfection of C2C12 cells (FIG. 1C) or HepG2 cells (FIG. 1D), N/P12 formulated saRNA with either JRL13 or ssJRL13, at 12.5ng to 400ng of RNA/well. Data here represents 3 independent experiments (n=3) with technical duplicates. **FIGs. 1E-1F** indicate expression viability of cells after 24 hours of transfection of C2C12 cells (FIG. 1E) or HepG2 cells (FIG. 1F), N/P12 formulated saRNA with either JRL13 or ssJRL13, at 12.5ng to 400ng of RNA/well. Data here represents 3 independent experiments (n=3) with technical duplicates.

### Example 2 - Intramuscular Vaccination

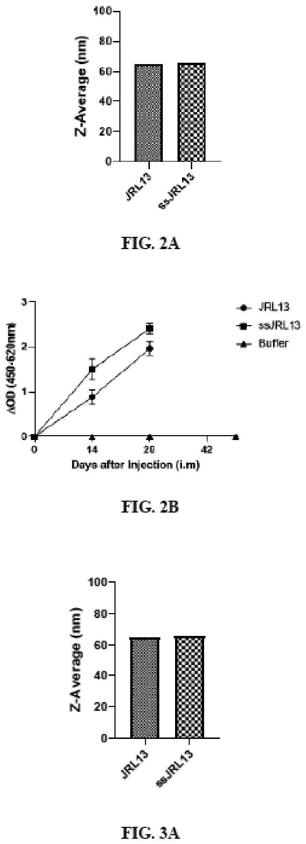

Fifteen female BALB/c mice were divided into three groups. One group received only buffer as a negative control and two groups received 2µg of formulated saRNA coding for Hemagglutinin (HA) protein of the influenza strain California/7/2009. All saRNA formulations were applied i.m in one of the legs. All saRNA groups were formulated at N/P6 but with different oligosaccharide (JRL13 or ssJRL13). Formulations were complexed in MBS Buffer (final concentration 10% w/v Sucrose, 10mM MES, pH 6.1), at RNA final concentration of 0.05mg/ml and the mixing ratio of the RNA containing solution and the oligosaccharide containing solution was 9:1 (v:v) for all the cases. The animals were subjected to non-invasive serological surveillance over 49 days at different time points (day 0, day 14, day 21, day 28, day 49) and spleen was extracted at the end of experiment for quantification of CD4/CD8 T-Cell response to Influenza HA peptides. Anti-Influenza HA antibodies in serum were quantified by enzyme-linked immunosorbent assay. Virus neutralizing titers were quantified by VNT Assay. CD4/CD8 T-Cell response was quantified by via IFN -ELISPOT of Splenocytes. **FIG. 2A** indicates hydrodynamic diameter for formulated saRNA with either JRL13 or ssJRL13. **FIG. 2B** indicates serological levels of specific IgG against HA over the course of the experiment at a serum dilution of 1:300.

### Example 3 - Synthetic Schemes

Scheme 1 depicts synthesis of a compound of formula I wherein A is A¹, R¹ and R² are both -C(O)-R^{a}, X¹ and X² are both -S-, Y¹ and Y² are both -(CH₂)₂- and Z¹ and Z² are NH₃+ (compounds ssJRL13 and PRX022 in Scheme 1). Scheme 1 also shows general routes (routes A1, A2, B1 and B2) implemented for the syntheses of a compound of formula I wherein A is A¹, R¹ and R² are both -C(O)-R^{a}, X¹ and X² are both -S-, Y¹ and Y² are both -(CH₂)₂-NHC(S)NH-(CH₂)₂- and Z¹ and Z² are identical (compounds 8 in Scheme 1):

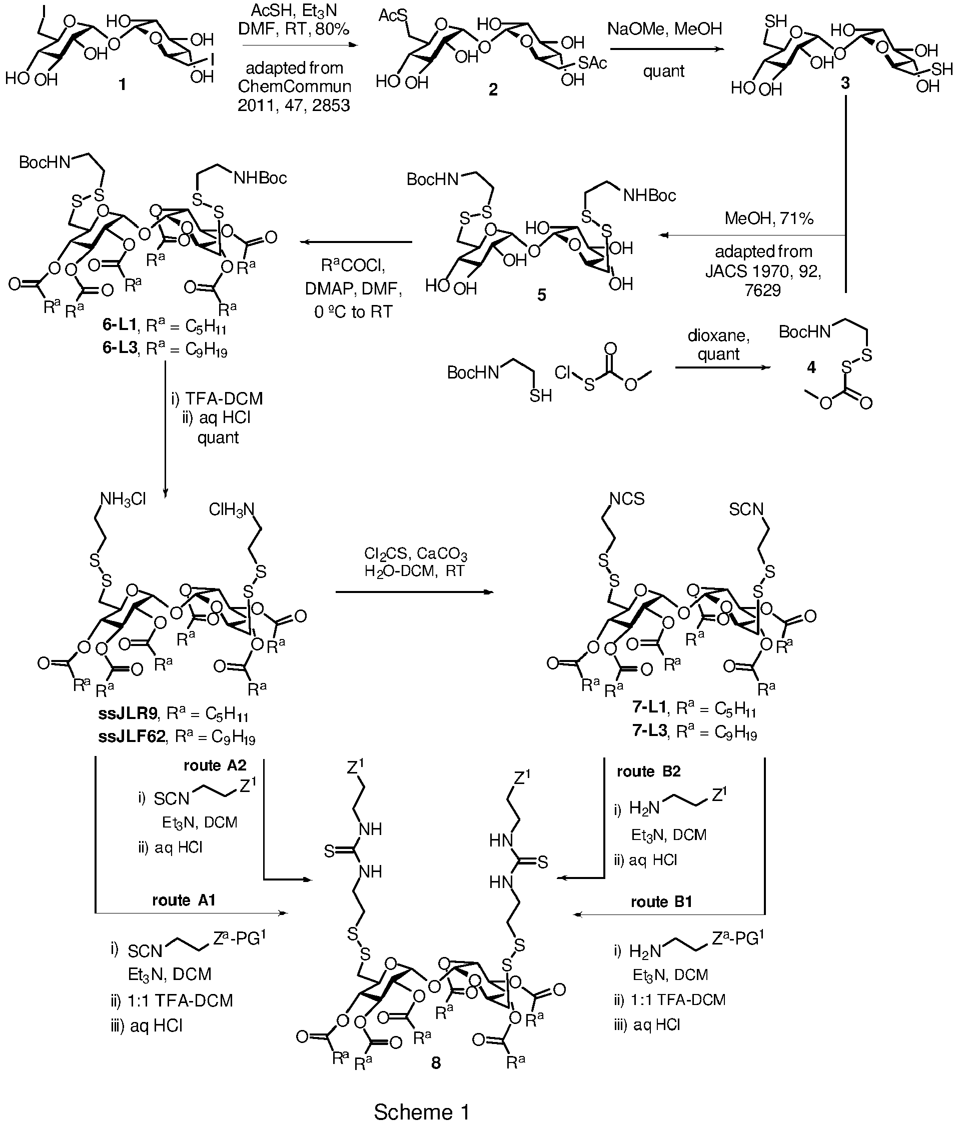

In some embodiments, synthesis of a compound of formula I wherein R¹ and R² are both -C(O)-R^{a} comprises the following lipid reagents:

| **Reagent** | **R^{a}** | **code** |
|---|---|---|
| 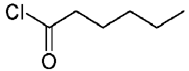 | 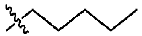 | L1 |
| 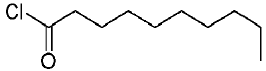 | 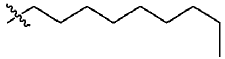 | L3 |

In some embodiments, the synthesis of the compounds of formula I according to Scheme 1 involved the following isothiocyanate or amine reagents in the reaction step leading to the installation of the cationic moieties Z¹ and Z²:

### Exemplary Cationic Moieties

| **reagent** | **Z¹ and Z²** | **code** |
|---|---|---|
| 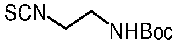 | 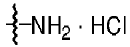 | C1 |
| 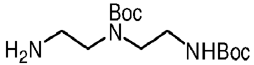 | 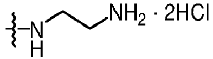 | C18 |
| 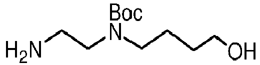 | 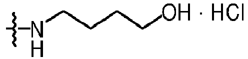 | OH3 |
| 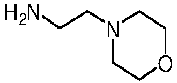 | 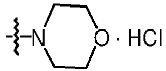 | C9 |
| 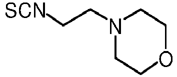 | | |
| 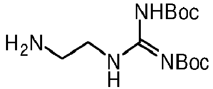 | 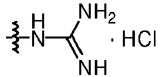 | C11 |

In some embodiments, the following precursors used in the synthesis of specific compounds described herein were prepared according to known methods:
**6,6'-Diiodo-6,6'-dideoxy-α,α'-trehalose (1).** Compound 1 was prepared following the procedure described in literature. See J. M. Garcia Fernández, et al., Carbohydr. Res. 1995, 268, 57-71.
**6,6'-Dithio-6,6'-di-S-acetyl-α,α'-trehalose (2).** Compound 2 was synthesized using a procedure similar to the procedure described by Bong. See D. Bong, et al., Chem. Commun. 2011, 47, 2853-2855. In the present example, potassium thioacetate was replaced by equimolecular amounts of triethylamine and thioacetic acid, that were sequentially added (in this order) to the reaction mixture.
**6,6'- Dithio -α,α'-trehalose (3).** Compound 3 was quantitatively obtained by treatment of compound 2 with sodium methoxide following the procedure described by Bong. D. Bong, et al., Chem. Commun. 2011, 47, 2853-2855.

As used herein, an arrow followed by a code between parentheses, e.g., "(→**code**)" indicates the lipophilic tails (R¹ and R² substituents in a compound of formula I) or cationic moieties (Z¹ and Z² substituents in a compound of formula I) that the preceding reagent provided during the synthesis. E.g., "hexanoyl chloride(→**L1**)" is intended to refer to moiety L1, as described in the tables above, was bonded to a compound through use of hexanoyl chloride.

Hexanoyl (CAS# 2051-49-2, →**L1**) and decanoyl (CAS# 112-13-0, →L3) chlorides were purchased from commercial sources.

2-(*N*-*tert*-Butoxycarbonylamino)ethanethiol (CAS# 67385-09-5), 2-morpholinoethanamine (CAS# 2038-03-1, → **C9**), 2-morpholinoethyl isothiocyanate (CAS# 2038-03-1, → **C9**) and 2-(2-aminoethyl)-1,3-di-(tert-butoxycarbonyl)guanidine (CAS# 203258-44-0, → **C11**) were purchased from commercial sources.

2-(*N*-*tert*-Butoxycarbonylamino)ethyl isothiocyanate (→ **C1**) was prepared according to the described procedure (D. M. Kneeland, K. Ariga, V. M. Lynch, C. Y. Huang, E. V. Anslyn, J. Am. Chem. Soc. 1993, 115, 10042-10055).

*N*-(4-hydroxybutyl)-*N*-(*tert*-butoxycarbonyl)ethylenediamine (→ **OH3**) was synthesized from commercially available *N*-(4-hydroxybupyl)ethylenediamine by sequential primary amine trifluoroacetylation with ethyl trifluoroacetate, carbamoylation of the remaining amino groups with Boc₂O and base-promoted trifluoroacetamide hydrolysis.

The synthetic intermediates **4, 5, 6-L1, 6-L3,** diamines ssJRL9, ssJLF62 and diisothiocyanates **7-L1** and **7-L3** in the reaction sequences depicted in Scheme 1 were prepared as described below:
**2-(tert-Butoxycarbonylamino)ethyl methoxycarbonyl disulfide (4).** Asymmetric disulphide 4 was synthesized by adapting the procedure described by Pilkington for cysteine. See L. I. Pilkington, R, et al., Chem. Commun. 2019, 55, 8868-8871. Briefly, a solution of commercially available 2-(tert-butoxycarbonylamino)ethanethiol (or Boc-protected cysteamine, Boc-Cyst, 1.09 g, 6.16 mmol) in freshly deoxygenated dioxane (20 mL) under N₂ atmosphere at 0 ºC was treated with methoxycarbonylsulfanyl chloride (0.78 g, 0.55 mL, 6.16 mmol). The reaction mixture was allowed to warm up to RT and stirred for 16 h. Then, the mixture was diluted with DCM (50 mL) and washed with water (50 mL). The organic layer was decanted, dried (MgSO₄), filtrated and concentrated to dryness to afford 4 (Yield 1.07 g, 65%) as a colourless oil whose purity was confirmed by TLC, ¹H and ¹³C NMR and MS. Compound **4** was used in the next step without further purification.

TLC (1:5 EtOAc-petroleum ether) 0.50

¹H NMR (400 MHz, CDCl₃) δ 5.39 (bs, 1 H, NH), 3.92 (s, 3 H, OMe), 3.40 (q, 2 H, ³J_{H,H} 5.8 Hz, CH₂NH), 2.89 (t, 2 H, CH₂S), 1.47 (s, 9 H, CMe₃).

¹³C NMR (100.6 MHz, CDCl₃) δ 178.5 (CO thiocarbonate), 155.8 (CO carbamate), 79.5 (CMe₃), 67.1 (OMe), 39.5 (CH₂S), 38.3 (CH₂N), 28.4 (CMe₃)

ESI-MS (m/z) 290.18 ([M + Na]⁺).

**6,6'-Bis-(2-(tert-butoxycarbonylamino)ethyldithio)-α,α'-trehalose (5).** To a solution of disulphide **5** (0.48 g, 1.82 mmol) in freshly degassed MeOH (10 mL) at 0 ºC, a solution of dithiol **4** (0.28 g, 0.76 mmol) in MeOH (5 mL) was dropwise added over a period of 1 h. The reaction was further stirred at 0 ºC for an additional hour and then the solvent was evaporated under reduced pressure. The resulting syrupy residue was purified by column chromatography (20:1 EtOAc-EtOH → 45:5:3 EtOAc-EtOH-water). Yield 0.39 g (71%).

TLC (45:5:3 EtOAc-EtOH-water) 0.46

¹H NMR (400 MHz, MeOD) δ 5.17 (d, 2 H, J_{1,2} 3.8 Hz, H-1), 3.97 (ddd, 2 H, J_{4,5} 3.8 Hz, J_{5,6a} 2.1 Hz, J_{5,6b} 8.6 Hz, H-5), 3.79 (t, 2 H, J_{2,3} = J_{3,4} = 9.3 Hz, H-3), 3.52 (dd, 2 H, H-2), 3.38 (m, 4 H, CH₂N), 3.29 (dd, 2 H, J_{6a,6b} 13.6 Hz, H-6a), 3.23 (dd, 2 H, H-4), 2.94 (dd, 2 H, H-6b), 2.83 (m, 4 H, CH₂S), 1.46 (s, 18 H, CMe₃).

¹³C NMR (100.6 MHz, MeOD) δ156.9 (CO), 93.0 (C-1), 78.9 (CMe₃), 73.4 (C-4), 73.1 (C-3), 71.8 (C-2), 71.2 (C-5), 43.2 (C-6), 39.4 (CH₂N), 37.6 (CH₂S), 37.4 (CMe₃).

ESI-MS (m/z) 1471.71 ([2M + Na]⁺), 747.49 ([M + Na]⁺).

**General procedure for exhaustive hydroxyl acylation of 6,6'-bis[2-(*tert-*butoxycarbonylamino)ethyldithio]-α,α'-trehalose (5) to give compounds ssJRL13 and PRX022.** In a typical experiment, a solution of derivative 5 (0.18 g, 0.25 mmol) and DMAP (0.55 g, 4.51 mmol) in DMF (10 mL) under N₂ atmosphere was dropwise added over a period of 30 min to a solution of the corresponding acyl chloride (2.26 mmol) in DMF (mL) under N₂ at 0 ºC. The reaction mixture was allowed to warm up to RT over 2 h and further stirred overnight. Then, the reaction was diluted with a diethyl ether-toluene mixture (1:1, 100 mL) and successively washed with water (2 × 50 mL) and aq 1N HCI (50 mL). The organic layer was dried over MgSO₄, filtrated and concentrated under reduced pressure and the syrupy residue was purified by column chromatography using the eluent indicated in each case to give the corresponding **6-L1** or **6-L3** reaction intermediate with R^{a} substituents as defined by the codes L1 or L3 in the above lipid reagent list.

**6,6'-Bis[2-(tert-butoxycarbonylamino)ethyldithio]-2,2',3,3',4,4'-hexa-O-hexanoyl-α,α'-trehalose** (**6-L1**). Eluent: petroleum ether → 1:5 EtOAc- petroleum ether. Yield: 79%.

TLC (1:3 EtOAc-petroleum ether) 0.47

¹H NMR (400 MHz, CDCl₃) δ 5.51 (t, 3 H, J_{2,3} = J_{3,4} 9.5 Hz, H-3), 5.33 (d, 2 H, J_{1,2} 3.7 Hz, H-1), 5.13 (dd, 2 H, H-2), 5.00 (bs, 2 H, NH), 4.98 (t, 2 H, J_{4,5} 9.5 Hz, H-4), 3.96 (ddd, 2 H, J_{5,6a} 3.7 Hz, J_{5,6b} 7.0 Hz, H-5), 3.40 (m, 4 H, CH₂N), 2.85 (m, 4 H, H-6), 2.76 (t, 4 H, ³J_{H,H} 6.4 Hz, CH₂S), 2.37, 2.28, 2.22 (3 t, 12 H, CH₂CO), 1.59 (m, 12 H, CH₂CH₂CO), 1.46 (s, 18 H, CMe₃), 1.30 (m, 24 H, CH₂CH₂CH₃), 0.91 (m, 18 H, CH₂CH₃).

¹³C NMR (100.6 MHz, CDCl₃) δ 172.4, 172.3 (CO ester), 155.7 (CO carbamate), 90.9 (C-1), 79.5 (CMe₃), 71.2 (C-4), 69.7 (C-3), 69.5 (C-5), 69.4 (C-2), 42.0 (C-6), 39.0 (CH₂N), 38.1 (CH₂S), 34.0 (CH₂CO), 31.2, 22.3 (CH₂CH₂CH₃), 28.3 (CMe₃), 24.4 (CH₂CH₂CO), 13.8 (CH₂CH₃).

ESI-MS (m/z) 1336.09 ([M + Na]⁺).

**6,6'-Bis[2-(*tert*-butoxycarbonylamino)ethyldithio]-2,2',3,3',4,4'-hexa-*O*-decanoyl-α,α'-trehalose** (6-L3). Eluent petroleum ether → 1:5 EtOAc- petroleum ether. Yield: 70%.

*R*_{f} 0.40 (1:3 EtOAc-petroleum ether).

¹H NMR (400 MHz, CDCl₃) δ 5.50 (t, 3 H, J_{2,3} = J_{3,4} 9.7 Hz, H-3), 5.33 (d, 2 H, J_{1,2} 4.0 Hz, H-1), 5.12 (dd, 2 H, H-2), 4.99 (bs, 2 H, NH), 4.98 (t, 2 H, J_{4,5} 9.7 Hz, H-4), 3.96 (ddd, 2 H, *J*_{5,6a} 4.1 Hz, *J*_{5,6b} 6.9 Hz, H-5), 3.42 (bq, 4 H, ³*J*_{H,H} 6.2 Hz, CH₂N), 2.88, 2.84 (2 dd, 4 H, *J*_{6a,6b} 13.8 Hz, H-6), 2.76 (t, 4 H, CH₂S), 2.36, 2.28, 2.24 (3 m, 12 H, CH₂CO), 1.62, 1.58 (2 m, 12 H, CH₂CH₂CO), 1.46 (s, 18 H, CMe₃), 1.28 (m, 72 H, (C*H*₂)₆CH₃), 0.90 (m, 18 H, CH₂CH₃).

¹³C NMR (100.6 MHz, CDCl₃) δ 172.4 (CO ester), 155.8 (CO carbamate), 91.0 (C-1), 79.5 (CMe₃), 71.3 (C-4), 69.7 (C-3), 69.5 (C-5), 69.4 (C-2), 42.0 (C-6), 39.1 (CH₂N), 38.2 (CH₂S), 34.2-34.1 (CH₂CO), 31.9-31.8, 22.7 ((*C*H₂)₂CH₃), 29.5-29.1 ((*C*H₂)₄CH₂CH₂CH₃), 28.4 (C*Me*₃), 24.9-24.7 (CH₂CH₂CO), 14.2, 14.1 (CH₂CH₃).

ESI-MS *(m*/*z)* 1550.02 ([M - Boc + H]⁺), 1695.01 ([M + HCO₂]⁻) .

**General procedure for *tert*-butoxycarbonyl group hydrolysis** in **reaction intermediates 6-L1** (→ **ssJRL9) and ssJLF62).** To a solution of **6-L1** or **6-L3** in DCM (5 mL per 0.1 mmol of substrate), at RT, trifluoroacetic acid (5 mL per 0.1 mmol of substrate) was added. The reaction mixture was stirred at RT for 1 h, then the solvents were evaporated under reduced pressure and the traces of acid were eliminated by repeated coevaporation with water. The resulting residue was lyophilized from 10-mM HCI to furnish the corresponding diamines ssJRL9 or ssJLF62, as hydrochloride salts, in quantitative yield.

### Example 3a - Compound ssJRL9.

TLC (45:5:3 EtOAc-EtOH-water) 0.50

¹H NMR (600 MHz, MeOD) δ 5.50 (t, 3 H, J_{2,3} = J_{3,4} 9.5 Hz, H-3), 5.38 (d, 2 H, J_{1,2} 3.7 Hz, H-1), 5.15 (dd, 2 H, H-2), 5.08 (t, 2 H, J_{4,5} 9.5 Hz, H-4), 4.06 (m, 2 H, H-5), 3.30 (t, 4 H, ³J_{H,H} 6.6 Hz, CH₂N), 3.00 (d, 4 H, J_{5,6} 5.7 Hz, H-6), 2.98, 2.92 (2m, 4 H, CH₂S), 2.44, 2.34, 2.28 (3 m, 12 H, CH₂CO), 1.67, 1.59 (2 m, 12 H, CH₂CH₂CO), 1.35 (m, 24 H, CH₂CH₂CH₃), 0.94 (m, 18 H, CH₃).

¹³C NMR (100.6 MHz, MeOD) δ 172.7, 172.4 (CO), 90.5 (C-1), 70.9 (C-4), 69.9 (C-3), 69.6 (C-2), 69.3 (C-5), 40.9 (C-6), 37.6 (CH₂N), 35.6 (CH₂S), 33.8-33.6 (CH₂CO), 31.1-31.0, 22.0 (CH₂CH₂CH₃), 24.2-24.1 (CH₂CH₂CO), 13.0-12.9 (CH₂CH₃).

ESI-MS (m/z) 1113.98 ([M - HCI - Cl]⁺), 557.44 ([M - 2Cl]²⁺).

### Example 3b - Compound ssJLF62.

¹H NMR (400 MHz, CDCl₃) δ 5.56 (t, 2 H, *J*_{2,3} = *J*_{3,4} 9.6 Hz, H-3), 5.40 (d, 2 H, *J*_{1,2} 4.0 Hz, H-1), 5.15 (dd, 2 H, H-2), 5.07 (t, 2 H, *J*_{4,5} 9.6 Hz, H-4), 4.04 (ddd, 2 H, *J*_{5,6a} 4.2, *J*_{5,6b} 7.0 Hz, H-5), 3.31 (t, 4 H, ³*J*_{H,H} 6.6 Hz, CH₂NH₂), 3.02, 2.98 (2 dd, 4 H, *J*_{6a,6b} 13.2 Hz, H-6), 2.94 (m, 4 H, CH₂S),2.43, 2.34, 2.23 (3 m, 6 H, CH₂CO), 2.23 (3 m, 12 H, CH₂CO), 1.67, 1.60 (2 m, 12 H, CH₂CH₂CO), 1.33 (m, 72 H, (C*H₂*)₆CH₃), 0.93 (m, 18 H, CH₃).

¹³C NMR (100.6 MHz, CDCl₃) δ 172.5, 172.3 (CO), 90.4 (C-1), 71.0 (C-4), 69.9 (C-3), 69.7 (C-2), 69.3 (C-5), 40.9 (C-6), 37.6 (CH₂N), 34.0 (CH₂S), 33.7-33.6 (CH₂CO), 31.8-31.7, 22.4 ((*C*H₂)₂CH₃), 29.4-28.9 ((*C*H₂)₄CH₂CH₂CH₃), 24.7-24.5 (CH₂CH₂CO), 13.2-13.1 (*C*H₃).

ESI-MS *(m*/*z)* 1449.95 ([M - HCI - Cl]⁺), 725.55 ([M - 2Cl]²⁺).

**General procedure for the synthesis of diisothiocyanate reaction intermediates 7-L1 and 7-L3.** To a solution of the corresponding diamine ssJRL9 or ssJLF62 (1 mmol) in a heterogeneous mixture of water and DCM (1:1, mL), CaCO₃ (0.48 g, 4.8 mmol) and Cl₂CS (0.22 ml, 2.4 mmol) were sequentially added. The mixture was vigorously stirred at RT for 1 h and then transferred to a decantation funnel and diluted with water and DCM (1:1, 100 mL). The organic layer was decanted and washed with water (50 mL) and saturated aq NaHCO₃ (50 mL). The organic phase was dried (MgSO₄), filtered and concentrated affording the corresponding diisothiocyanates **8-L1** or **8-L3** as pale syrups in virtually quantitative yield, which were used in the preparations of compounds **8** (Scheme 1, routes B1 and B2) without further purification.

**6,6'-Bis(2-isothiocyanatoethyldithio)-2,2',3,3',4,4'-hexa-O-hexanoyl-α,α'-trehalose (8-L1).** Yield 93% . *R*_{f} 0.33 (1:4 EtOAc-petroleum ether).

¹H NMR (600 MHz, CDCl₃) δ 5.52 (t, 2 H, *J*_{2,3} = *J*_{3,4} 9.7 Hz, H-3), 5.32 (d, 2 H, *J*_{1,2} 3.9 Hz, H-1), 5.09 (dd, 2 H, H-2), 4.98 (t, 2 H, *J*_{4,5} 9.7 Hz, H-4), 3.95 (ddd, 2 H, *J*_{5,6a} 4.3 Hz, *J*_{5,6b} 6.8 Hz, H-5), 3.81 (t, 4 H, ³*J*_{H,H} 6.6 Hz, CH₂N), 2.91 (m, 8 H, H-6, CH₂S), 2.35, 2.28, 2.23 (3 m, 12 H, CH₂CO), 1.58 (m, 12 H, CH₂CH₂CO), 1.29 (m, 24 H, CH₂CH₂CH₃), 0.90 (m, 18 H, C*H*₃).

¹³C NMR (150.9 MHz, CDCl₃) δ 172.4 (CO), 132.9 (NCS), 91.1 (C-1), 71.0 (C-4), 69.6, 69.5 (C-2,3), 69.4 (C-5), 43.9 (CH₂NCS), 42.7 (CH₂S), 38.0 (C-6), 34.1-34.0 (CH₂CO), 31.3-31.1, 22.3-22.2 (CH₂CH₂CH₃), 24.5-24.4 (CH₂CH₂CO), 13.8-13.8 (CH₂CH₃). ESI-MS 1197.55 ([M + H]+), 1241.72 ([M + HCO₂]⁻) .

**6,6'-Bis(2-isothiocyanatoethyldithio)-2,2',3,3',4,4'-hexa-O-decanoyl-α,α'-trehalose (8-L3).** Yield 49%. *R*_{f} 0.51 (1:4 EtOAc-petroleum ether).

¹H NMR (400 MHz, CDCl₃) δ 5.49 (t, 2 H, *J*_{2,3} = *J*_{3,4} 9.6 Hz, H-3), 5.32 (d, 2 H, *J*_{1,2} 4.0 Hz, H-1), 5.08 (dd, 2 H, H-2), 4.98 (t, 2 H, *J*_{4,5} 9.6 Hz, H-4), 3.94 (ddd, 2 H, *J*_{5,6a} 4.5 Hz, *J*_{5,6b} 6.7 Hz, H-5), 3.80 (t, 4 H, ³*J*_{H,H} 6.5 Hz, CH₂N), 2.90 (m, 8 H, H-6, CH₂S), 2.35, 2.28, 2.22 (3 m, 12 H, CH₂CO), 1.58 (m, 12 H, CH₂CH₂CO), 1.26 (m, 72 H, C*H₂*(C*H₂*)₆CH₃), 0.88 (m, 18 H, C*H*₃).

¹³C NMR (100.6 MHz, CDCl₃) δ 172.4 (CO), 133.0 (NCS), 91.1 (C-1), 71.1 (C-4), 69.5 (C-2,3), 69.4 (C-5), 43.9 (CH₂NCS), 42.7 (CH₂S), 38.0 (C-6), 34.1 (CH₂CO), 31.9-31.8, 29.5-29.1 ((*C*H₂)₆CH₃), 24.8 (CH₂CH₂CO), 22.6 (*C*H₂CH₂CH₂CO), 14.1 (CH₂CH₃). ESI-MS 1577.82 ([M + HCO₂]⁻).

**General procedure for the synthesis of a compound of formula I according to Schemes 1 and 2, routes A1, A2, B1 and B2.**

**Route A1 (schemes 1):** To a solution of diamine dihydrochloride (ssJRL9 or ssJLF62, 0.1 mmol) in DCM (10 mL) triethylamine (Et₃N, 0.3 mmol, 41 µL) and a solution of the corresponding isothiocyanate reagent SCN-(CH₂)₂-Z^{a}-PG¹ (0.3 mmol) in DCM (5 mL) were sequentially added. The reaction mixture was stirred at RT for 16 h while monitoring pH to ensure it remains basic (in case pH is found below neutrality, it should be readjusted to slightly basic (pH ca. 8) by addition of aliquots of Et₃N). Upon reaction completion (TLC revealed the complete disappearance of the starting material ssJRL9 or ssJLF62 and the formation of a novel spot at a higher *R*_{f}), the reaction mixture was concentrated to dryness and the crude material purified by column chromatography using the indicated eluent. The resulting intermediate with Boc-protected amino groups was subsequently treated with a 1:1 DCM-TFA mixture (10 mL) at RT for 1 h followed by evaporation of the solvents under reduced pressure. Traces of TFA were eliminated by coevaporation with toluene (3 × 10 mL). Lyophilization from 10 mM HCI furnished the target products **8** in the yield indicated in each case.

In some embodiments, the following compounds of formula I featuring structure **8** were synthesized according to Scheme 1, route A1: ssJRL13, PRX022.

**Route A2 (scheme 1):** Similarly, to route A1, route A2 implies the coupling reaction of compound ssJRL9 or ssJLF62 with an isothiocyanate reagent SCN-(CH₂)₂-Z¹, using an analogous protocol. Upon reaction completion, the reaction mixture was transferred to a decantation funnel and partitioned between DCM and 0.1 N aq HCI (1:1, 20 mL). The organic layer was decanted, dried over MgSO₄ or Na₂SO₄, filtrated and evaporated under reduced pressure. Column chromatography purification was conducted in these cases. The resulting product was lyophilized from 10 mM HCI to furnish the target product **8** in the yield indicated in each case.

In some embodiments, the following compound of formula I featuring structure **8** was synthesized according to Scheme 1, route A2: ssJLF99

**Route B1 (Scheme** 1): To a solution of the corresponding diisothiocyanate reaction intermediate **7-L1** or **7-L3** (0.1 mmol) in DCM (10 mL) a solution the corresponding amine reagent H₂N-(CH₂)₂-Z^{a}-PG¹ (0.3 mmol) in DCM (5 mL) was added. The reaction mixture was stirred at RT for 16 h while monitoring pH to ensure it remains basic. Upon reaction completion (TLC revealed the complete disappearance of the starting material **7-L1** or **7-L3** and the formation of a novel spot at a lower *R*_{f}), the reaction mixture was concentrated to dryness and the crude material purified by column chromatography using the indicated eluent. The resulting intermediate was further treated with a 1:1 DCM-TFA mixture (10 mL) at RT for 1 h followed by evaporation of the solvents under reduced pressure, coevaporation of the acidic traces with toluene (3 × 10 mL) and lyophilization from 10 mM HCI to furnish the target products **8** in the yield indicated in each case.

In some embodiments, the following compounds of formula I featuring structure **8** were synthesized according to Scheme 1, route B1: ssJLF102, ssJLF97, ssJLF94.

**Route B2 (Scheme 1):** Similarly, to route B1, route B2 implies the coupling reaction of a compound of structure **7-L1** or **7-L3** with an amine reagent H₂N-(CH₂)₂-Z¹, using an analogous protocol. Upon reaction completion, the reaction mixture was transferred to a decantation funnel and partitioned between DCM and 0.1 N aq HCI (1:1, 20 mL). The organic layer was decanted, dried over MgSO₄ or Na₂SO₄, filtrated and evaporated under reduced pressure. Column chromatography purification is unnecessary in these cases. The resulting product was lyophilized from 10 mM HCI to furnish the target products **8** in the indicated yields.

In some embodiments, the following compound of formula I featuring structure **8** was synthesized according to Scheme 1, route B2: ssJLF99.

### Example 3c - Compound ssJRL13

Synthesized from ssJRL9 and the corresponding isothiocyanate (code C1) according to Scheme 1, Route A1 as detailed: To a solution of diamine ssJRL9 (0.20 g, 0.18 mmol) in DCM (5 mL) Et₃N (75 µL, 0.54 mmol, 1.5 eq) and 2-(*tert*-butoxycarbonylamino)ethyl isothiocyanate (87 mg, 0.43 mmol, 1.2 eq) were sequentially added. The reaction mixture was stirred at RT for 16 h and concentrated to dryness. The residue was purified by column chromatography using 1:2 EtOAc-toluene as eluent to furnish the di-Boc-protected intermediate 6,6'-bis[3-(2-(tert-butoxycarbonylamino)ethyl)thioureidoethyldithio)]-2,2',3,3',4,4'-hexa-O-hexanoyl-α,α'-trehalose in 93% yield (0.26 g).

TLC (1:2 EtOAc-toluene) 0.21

¹H NMR (600 MHz, CDCl₃) δ 7.09-6.90 (bs, 4 H, NHCS), 5.51 (t, 3 H, J_{2,3} = J_{3,4} 9.8 Hz, H-3), 5.42 (d, 2 H, J_{1,2} 3.7 Hz, H-1), 5.27 (bs, 2 H, NHCO), 5.10 (dd, 2 H, H-2), 5.00 (t, 2 H, J_{4,5} 9.7 Hz, H-4), 3.79 (m, 2 H, H-5), 3.87, 3.62 (2 bs, 8 H, CH₂NHCS), 3.34 (bt, 4 H, ³J_{H,H} 5.2 Hz, CH₂NHBoc), 2.94 (bt, 4 H, CH₂S), 2.91 (m, 4 H, H-6), 2.37, 2.26, 2.24 (3 t, 12 H, CH₂CO), 1.63 (m, 12 H, CH₂CH₂CO), 1.56 (s, 18 H, CMe₃), 1.45 (s, 18 H, CMe₃), 1.31 (m, 24 H, CH₂CH₂CH₃), 0.90 (m, 18 H, CH₂CH₃).

¹³C NMR (150.9 MHz, CDCl₃) δ 181.2 (CS), 172.7, 172.4 (CO ester), 157.2 (CO carbamate), 90.4 (C-1), 80.0 (CMe₃), 71.1 (C-4), 69.7 (C-2,5), 69.5 (C-3), 42.7 (CH₂NHCS), 42.4 (C-6), 39.9 (CH₂NHBoc), 37.3 (CH₂S), 34.1, 34.0 (CH₂CO), 31.3, 31.2, 22.2 (CH₂CH₂CH₃), 28.4 (CMe₃), 24.5-24.4 (CH₂CH₂CO), 13.9, 13.8 (CH₂CH₃).

ESI-MS (m/z) 1541.02 ([M + Na]⁺).

Subsequent treatment of the di-Boc intermediate (0.21 g, 0.14 mmol) with a 1:1 DCM-TFA mixture (8 mL) at RT for 1 h furnished the deprotected diamine ssJR13 in virtually quantitative yield after solvent evaporation under vacuum and lyophilization from diluted HCl.

¹H NMR (600 MHz, MeOD) δ 5.54 (t, 3 H, J_{2,3} = J_{3,4} 9.7 Hz, H-3), 5.37 (d, 2 H, J_{1,2} 3.9 Hz, H-1), 5.19 (dd, 2 H, H-2), 5.05 (t, 2 H, J_{4,5} 9.7 Hz, H-4), 4.07 (m, 2 H, H-5), 3.87, 3.78 (2 m, 8 H, CH₂NHCS), 3.17 (t, 4 H, ³J_{H,H} 6.1 Hz, CH₂NH₂), 2.99 (dd, 2 H, J_{5,6a} 3.2 Hz, J_{6a,6b} 14.4 Hz, H-6a), 2.93 (m, 6 H, H-6b, CH₂S), 2.44, 2.35, 2.27 (3 m, 12 H, CH₂CO), 1.63, 1.60 (2 m, 12 H, CH₂CH₂CO), 1.35 (m, 24 H, CH₂CH₂CH₃), 0.94 (m, 18 H, CH₃). ¹³C NMR (150.9 MHz, MeOD) δ 181.0 (CS), 172.7, 172.4, 172.3 (CO ester), 90.5 (C-1), 71.1 (C-4), 70.1 (C-3), 69.5 (C-2), 69.0 (C-5), 42.8, 40.9 (CH₂NHCS), 40.9 (C-6), 39.5 (CH₂NH₂), 36.4 (CH₂S), 33.9, 33.6, 33.5 (CH₂CO), 31.2, 31.0, 22.0 (CH₂CH₂CH₃), 24.3, 24.1 (CH₂CH₂CO), 13.0, 12.9, 12.8 (CH₂CH₃).

ESI-MS (m/z) 1318.00 ([M - HCI - Cl]⁺), 1284.00 ([M - 2HCl - SH]+), 659.47 ([M - 2Cl]²⁺).

### Example 3d - Compound ssJLF102

Synthesized from isothiocyanate **7-L1** and the corresponding amine reagent (code C18) according to Scheme 1, Route B1. Overall yield (2 steps) 79%. Chemical Formula: C₆₂H₁₁₈Cl₄N₈O₁₅S₆. Molecular Weight: 1549.83. ESI-MS *(m*/*z)* 1403.6 ([M - HCI - Cl]⁺).

### Example 3e - Compound ssJLF97

Synthesized from isothiocyanate **7-L1** and the corresponding amine reagent (code OH3) according to Scheme 1, Route B1. Overall yield (2 steps) 78%. Chemical Formula: C₆₆H₁₂₂Cl₂N₆O₁₇S₆. Molecular Weight: 1534.99. ESI-MS *(m*/*z)* 731.87 ([M - 2Cl]²⁺).

### Example 3f - Compound ssJLF94

Synthesized from isothiocyanate **7-L1** and the corresponding amine reagent (code C11) according to Scheme 1, Route B1. Overall yield (2 steps) 87%. Chemical Formula: C₆₀H₁₁₀Cl₂N₁₀O₁₅S₆. Molecular Weight: 1474.86. ESI-MS *(m*/*z)* 701.84 ([M - 2Cl]²⁺).

### Example 3g - Compound ssJLF99

Synthesized from isothiocyanate **7-L3** and the corresponding amine reagent (code **C9**) according to Scheme 1, Route B2 or from compound ssJLF92 and the corresponding isothiocyanate reagent (code **C9**) according to Scheme 1, Route A2. Yield 93% (route B2) or 89% (route A2). Molecular Weight: 1867.60. ESI-MS *(m*/*z)* 1794.02 ([M - HCI - Cl]⁺), 898.07 ([M - 2Cl]²⁺).

### Example 3h - Compound PRX022

Synthesized from compound ssJLF62 and the corresponding isothiocyanate reagent (code C1) according to Scheme 1, Route A1. Yield 94%. Chemical Formula: C₈₂H₁₅₄Cl₂N₆O₁₅S₆. Molecular Weight: 1727.42. ESI-MS *(m*/*z)* 1653.9 ([M - HCI - Cl]⁺), 827.5 ([M - 2Cl]²⁺).

### Example 4 - Intramuscular Vaccination

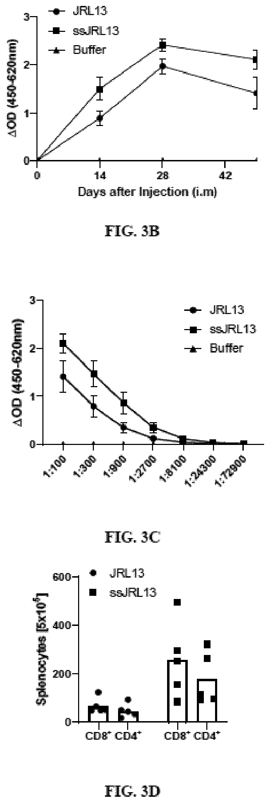

Fifteen female BALB/c mice were divided into three groups. One group received only buffer as a negative control and the other two groups received 2µg of formulated saRNA codig for Hemagglutinin (HA) protein of the influenza strain California/7/2009. All saRNA formulations were applied i.m in one of the legs. All saRNA groups were formulated at N/P 6 but with different oligosaccharides (JRL13 or ssJRL13). Formulations were complexed in MBS Buffer (final concentration 10% w/v Sucrose, 10mM MES, pH 6.1), at RNA final concentration of 0.05mg/ml and the mixing ratio of the RNA solution and the oligosaccharide solution was 9:1 (v:v) for all the cases. The animals were subjected to non-invasive serological surveillance over 49 days at different time points (day 0, day 14, day 21, day 28, day 49) and spleen was extracted at the end of the experiment for quantification of CD4/CD8 T-Cell response to Influenza HA peptides. Anti-Influenza HA antibodies in serum were quantified by enzyme-linked immunosorbent assay. CD4/CD8 T-Cell response was quantified by IFN -ELISPOT of Splenocytes. **FIG. 3A** is a bar graph indicating the hydrodynamic diameter for formulated saRNA with either JRL13 or ssJRL13. **FIG. 3B** is a plot illustrating serological levels of specific IgG against HA over the course of the experiment at a serum dilution of 1:300. **FIG. 3C** is a plot indicating end-point titration of the serological levels of specific IgG against HA. **FIG. 3D** is a bar graph indicating CD8/CD4 response against HA-peptide pools. The present example shows that introduction of disulfide bonds into the cationic element of JRL13 leads to a significant improvement in the overall immunogenicity.

### Example 5 - Intramuscular Vaccination

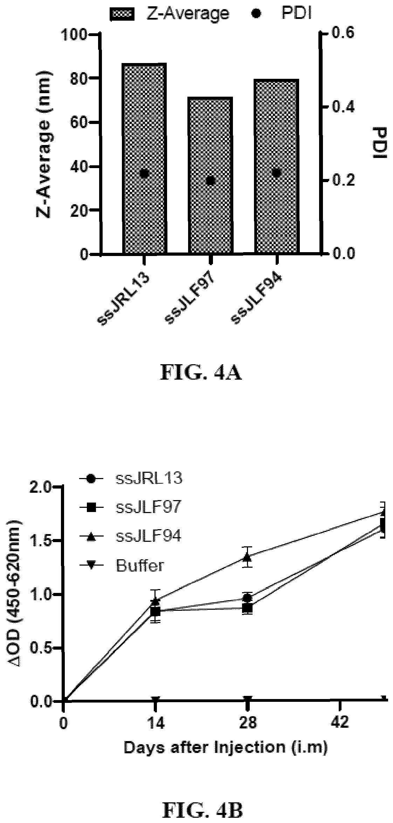

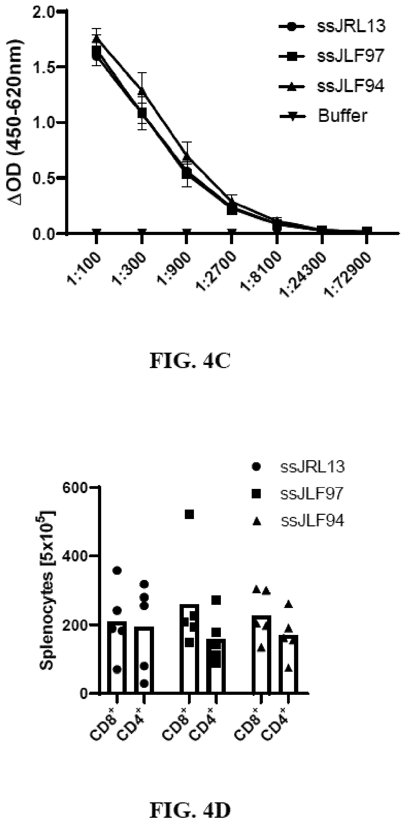

Twenty female BALB/c mice were divided into four groups. One group received only buffer as a negative control and the other three groups received 2µg of formulated saRNA coding for Hemagglutinin (HA) protein of the influenza strain California/7/2009. All saRNA formulations were applied i.m in one of the legs. All saRNA groups were formulated at N/P6 with different oligosaccharides (ssJRL13, ssJLF97 or ssJLF94). Formulations were complexed in MBS Buffer (final concentration 10% w/v Sucrose, 10mM MES, pH 6.1), at RNA final concentration of 0.01mg/ml and the mixing ratio of the RNA solution and the oligosaccharide solution was 9:1 (v:v) for all the cases. The animals were subjected to non-invasive serological surveillance over 49 days at different time points (day 0, day 14, day 21, day 28, day 49) and spleen was extracted at the end of the experiment for quantification of CD4/CD8 T-Cell response to Influenza HA peptides. Anti-Influenza HA antibodies in serum were quantified by enzyme-linked immunosorbent assay. CD4/CD8 T-Cell response was quantified by IFN -ELISPOT of Splenocytes. **FIG. 4A** is a bar graph the hydrodynamic diameter for formulated saRNA with ssJRL13, ssJRL13 or ssJLF94. **FIG. 4B** is a plot illustrating serological levels of specific IgG against HA over the course of the experiment at a serum dilution of 1:300 . **FIG. 4C** is a plot illustrating end-point titration of the serological levels of specific IgG against HA. **FIG. 4D** is a bar graph illustrating CD8/CD4 response against HA-peptide pools.

## Claims

1. A composition comprising an oligosaccharide of formula I: 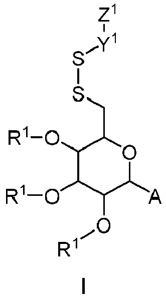 or a pharmaceutically acceptable salt thereof, wherein:
A is A¹, A², or A³: 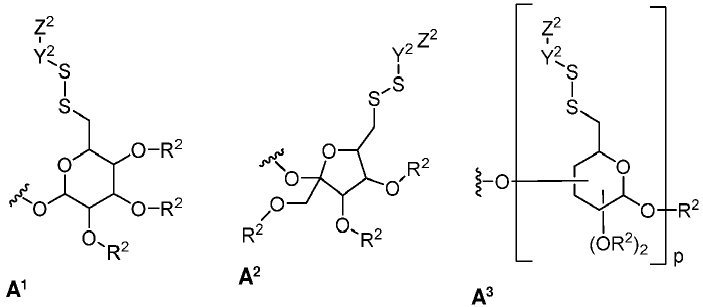
R¹ and R² are each independently selected from H, R^{a}, and -C(O)-R^{a}, and wherein at least one instance of R¹ or R² is not H;
each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b};
each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, and -NHC(O)NHR^{c};
each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S;
Y¹ and Y² are each independently an optionally substituted C₁₋₃₀ aliphatic group wherein one or more carbons are optionally and independently replaced by -Cy-, -NH-, -NHC(O)-, -C(O)NH-, -NHC(O)O-, -OC(O)NH-, -NHC(O)NH-, -NHC(S)NH-, -C(S)NH-C(O)NHSO₂-, -SO₂NHC(O)-,-OC(O)O-, -O-, -C(O)-, -OC(O)-, -C(O)O-, -SO-, or -SO₂-;
each Cy is independently an optionally substituted C₃-C₁₄ cycloaliphatic, 5- to 14-membered heterocyclyl ring having 1-3 heteroatoms selected from N, O, S, 5- to 14-membered heteroaryl ring having 1-3 heteroatoms selected from N, O, S;
Z¹ and Z² are each independently a cationic or ionizable group selected from optionally substituted 5- to 14-membered heterocyclyl ring having 1-3 heteroatoms selected from N, O, and S, 5- to 14-membered heteroaryl ring having 1-3 heteroatoms selected from N, O, and S, -N⁺(M)₃, 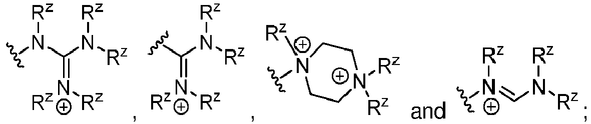
each M is independently -C₀-C₆ aliphatic-R^{z} or -C₀-C₆ aliphatic-N⁺(R^{z})₃;
each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S; and
p is an integer selected from 1, 2, 3, 4, and 5.

2. The composition of claim 1, wherein the oligosaccharide is of formula la: 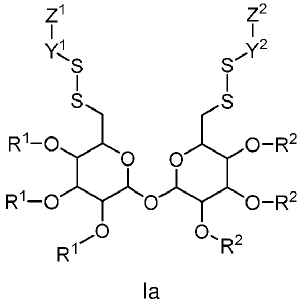 or a pharmaceutically acceptable salt thereof.

3. The composition of claim 1, wherein the oligosaccharide is of formula lb: 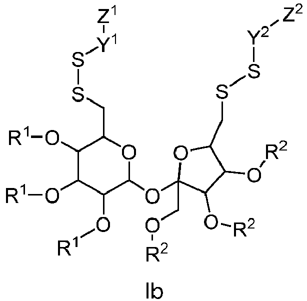 or a pharmaceutically acceptable salt thereof.

4. The composition of claim 1, wherein the oligosaccharide is of formula Ic: 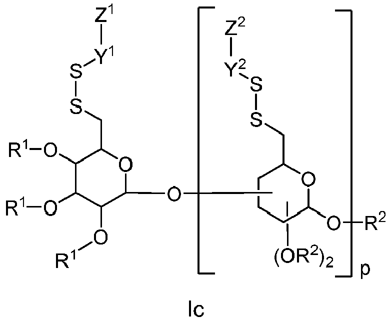 or a pharmaceutically acceptable salt thereof.

5. The composition of any one of claims 1-4, wherein R¹ and R² are each independently selected from R^{a} and -C(O)-R^{a}, and R^{a} is C₁-C₂₀ aliphatic.

6. The composition of any one of claims 1-5, wherein R¹ and R² are each -C(O)-R^{a}, and R^{a} is C₁-C₁₄ aliphatic.

7. The composition of any one of claims 1-6, wherein each R^{a} is C₅-C₁₀ linear alkyl.

8. The composition of claim 1, wherein R¹ and R² are each -C(O)-R^{a}, and R^{a} is 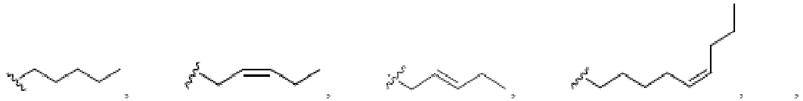 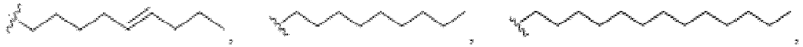 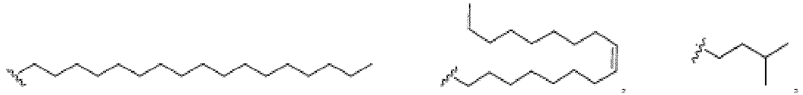 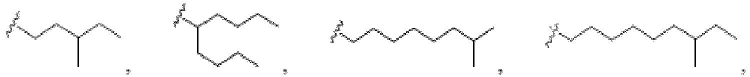 or

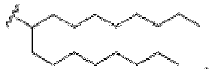

9. The composition of any one of claims 1-8, wherein Y¹ and Y² are each selected from C₁-C₁₀ aliphatic, C₀-C₄-aliphatic-NHC(O)NH-C₀-C₄ aliphatic, C₀-C₄-aliphatic-NHC(S)NH-C₀-C₄ aliphatic, C₀-C₄-aliphatic-C(O)NH-C₀-C₄ aliphatic, C₀-C₄-aliphatic-C(S)NH-C₀-C₄ aliphatic, C₀-C₄-aliphatic-NHC(O)-C₀-C₄ aliphatic, C₀-C₄-aliphatic-NHSO₂-C₀-C₄ aliphatic, and C₀-C₄-aliphatic-C(O)-C₀-C₄ aliphatic.

10. The composition of any one of claims 1-9, wherein Y¹ and Y² are each C₁-C₄ aliphatic-NHC(S)NH-C₁-C₄ aliphatic.

11. The composition of claim 1, wherein Y¹ and Y² are each -CH₂-CH₂-NHC(S)NH-CH₂-CH₂-.

12. The composition of any one of claims 1-11, wherein Z¹ and Z² are each independently selected from: N⁺(M)₃, 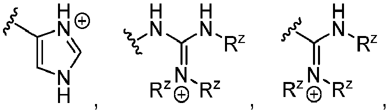 and

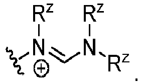

13. The composition of any one of claims 1-11, wherein Z¹ and Z² are each independently selected from:

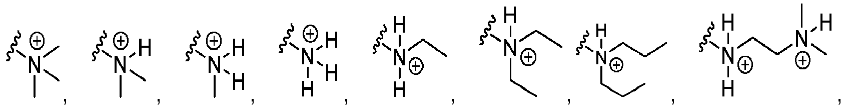

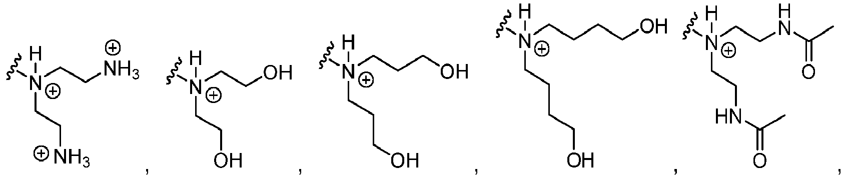

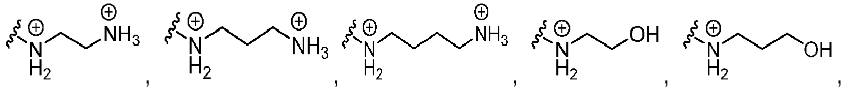

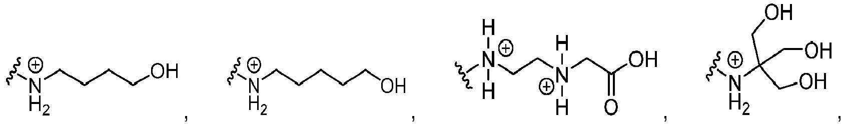

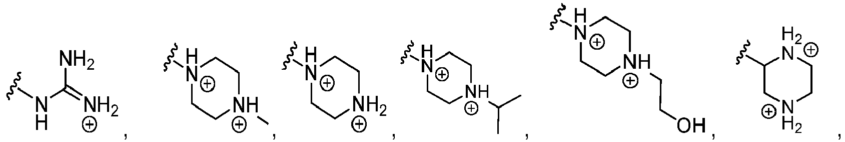

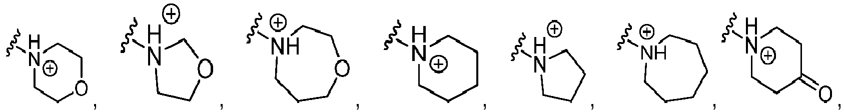

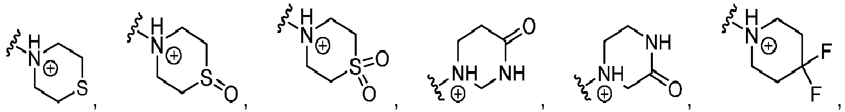

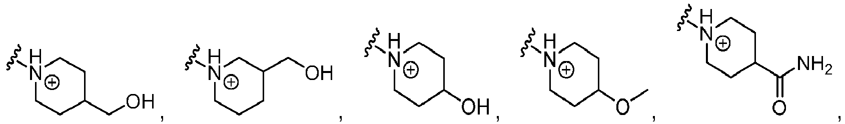

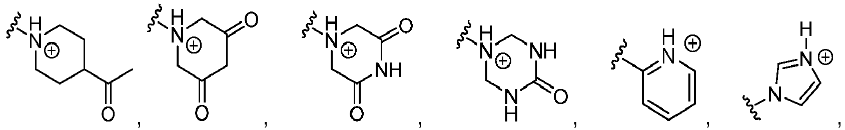

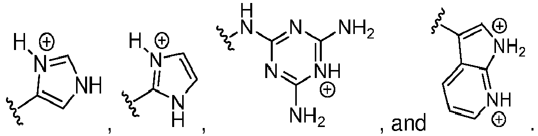

14. The composition of claim 1, wherein the oligosaccharide is a compound of formula Id: 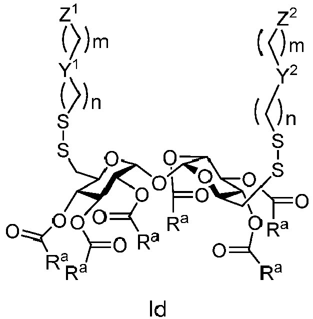 or a pharmaceutically acceptable salt thereof, wherein m and n are each independently selected from 0, 1, 2, 3, 4, 5, or 6.

15. The composition of claim 14, wherein the oligosaccharide is a compound of formula Id-i: 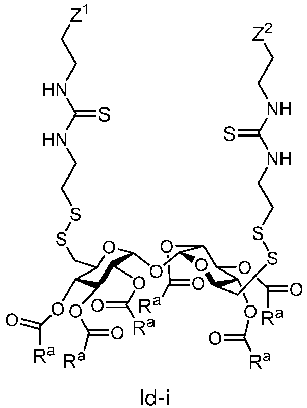 or a pharmaceutically acceptable salt thereof.

16. The composition of any one of claims 1-15, further comprising one or more suitable counterions.

17. The composition of claim 1, wherein the oligosaccharide is selected from Table 1.

18. A complex comprising the composition of any one of claims 1-17, and a nucleic acid.

19. The complex of claim 18, wherein the nucleic acid is RNA.

20. The complex of claim 19, wherein the RNA is a modRNA or a saRNA.

21. The complex of claims 18-20, wherein a ratio of N/P is less than 20:1.

22. The complex of claim 21, wherein the ratio of N/P is less than or equal to 12:1.

23. The complex of any one of claims 18-22, wherein the complex has a diameter of about 30 nm to about 150 nm.

24. A method of delivering a composition to a target in a subject comprising administering to the subject the complex of any one of claims 18-23.

25. The method of claim 24, wherein the target is selected from the liver, the lungs, or the spleen.

26. A method of treating a disease, disorder, or condition in a subject comprising administering to the subject a complex of any one of claims 18-23.

27. The method of claim 26, wherein the disease, disorder, or condition is an infectious disease, cancer, a genetic disorder, an autoimmune disease, or a rare disease.

28. The method of any one of claims 24-27, wherein the complex is administered intramuscularly.

29. The method of any one of claims 24-27, wherein the complex is administered subcutaneously.

30. A method of preparing an oligosaccharide of formula la: 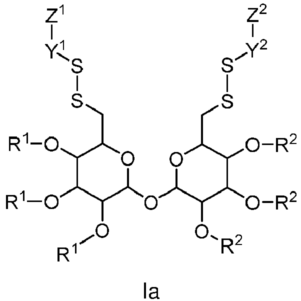 or a pharmaceutically acceptable salt thereof, comprising contacting a compound of formula II 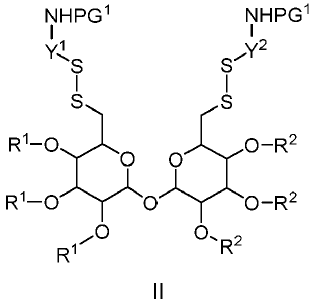 with a first acid to provide the compound of formula la, wherein
Y¹ and Y² are each -CH₂-CH₂-NHC(S)NH-CH₂-CH₂-;
Z¹ and Z² are each -NH₃Cl;
R¹ and R² are each -C(O)-R^{a};
R^{a} is optionally substituted C₁-C₂₀ aliphatic; and
PG¹ is a nitrogen protecting group.

31. The method of claim 30, further comprising contacting a compound of formula III 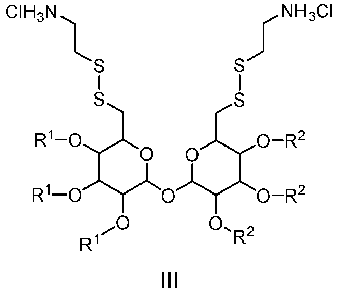 with a compound of formula IV 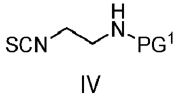 in the presence of a base to provide the compound of formula II.

32. The method of claim 30, further comprising contacting a compound of formula IIIa 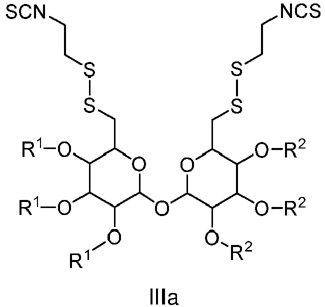 with a compound of formula IVa 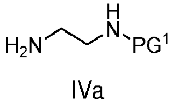 in the presence of a base to provide the compound of formula II.

33. The method of claim 32, further comprising contacting a compound of formula III with CSCl₂ to provide a compound of formula IIIa.

34. The method of claim 31, further comprising contacting a compound of formula V 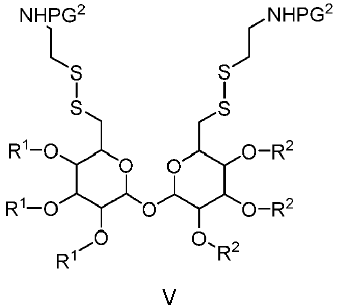 with a second acid to provide the compound of formula III, wherein PG² is a suitable nitrogen protecting group.

35. The method of claim 34, further comprising contacting a compound of formula VI 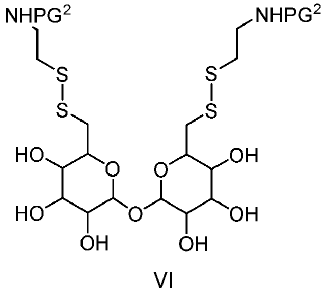 with a compound of formula VIa: 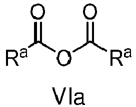 to provide the compound of formula V.

36. The method of claim 35, further comprising contacting a compound of formula VII 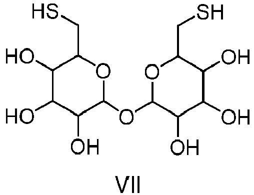 with a compound of formula VIIa 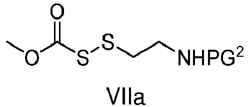 to provide the compound of formula VI.

37. The method of claim 36, further comprising contacting a compound of formula VIII 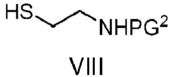 with a compound of formula VIIIa 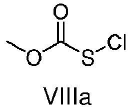 in the presence of dioxane to provide the compound of formula VIIa.

38. The method of claims 36 or 37, further comprising contacting a compound of formula IX 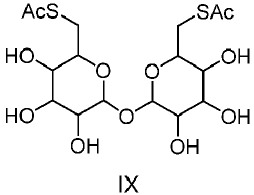 with sodium methoxide to provide a compound of formula VII.

39. The method of claim 38, further comprising contacting a compound of formula X 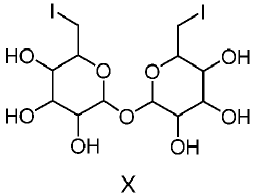 with thioacetic acid in the presence of a third base to provide the compound of formula IX.
